# EUROPEAN PATENT SPECIFICATION

(11) **EP 1 841 874 B1**
(45) Date of publication and mention of the grant of the patent: **06.01.2010**
(21) Application number: 06719694.9
(22) Date of filing: 27.01.2006
(51) Int. Cl.: C12N 15/62

(54) **LEADER SEQUENCES FOR DIRECTING SECRETION OF POLYPEPTIDES AND METHODS FOR PRODUCTION THEREOF**
LEITSEQUENZEN ZUR ANLEITUNG VON POLYPEPTIDSEKRETION UND VERFAHREN ZUR HERSTELLUNG
SEQUENCES DE TETE DE CONDUITE DE LA SECRETION DE POLYPEPTIDES ET METHODES DE PRODUCTION ASSOCIEES

(30) Priority: 27.01.2005 US 647013 P
(43) Date of publication of application: 10.10.2007
(73) Proprietor: Five Prime Therapeutics, Inc., San Francisco CA 94158-2261 (US)
(72) Inventor: HALENBECK, Robert, Forgan, San Rafael, CA 94903 (US); BOSCH, Elizabeth, Cupertino, CA 95014 (US); LINNEMANN, Thomas, San Francisco, CA 94115 (US); LEE, Ernestine, Kensington, CA 94707 (US)
(74) Representative: Brasnett, Adrian Hugh
(86) International application number: PCT/US2006/002951
(87) International publication number: WO 2006/081430

(56) References cited:
- WO-A-2005/105840
- ORITANI K ET AL: "IDENTIFICATION OF STROMAL CELL PRODUCTS THAT INTERACT WITH PRE-B CELLS" THE JOURNAL OF CELL BIOLOGY, ROCKEFELLER UNIVERSITY PRESS, US, vol. 134, no. 3, 1 August 1996 (1996-08-01), pages 771-782, XP009001265 ISSN: 0021-9525
- LO K-M ET AL: "High level expression and secretion of Fc-X fusion proteins in mammalian cells" PROTEIN ENGINEERING, OXFORD UNIVERSITY PRESS, SURREY, GB, vol. 11, no. 6, June 1998 (1998-06), pages 495-500, XP002125745 ISSN: 0269-2139
- CHOI J H ET AL: "Secretory and extracellular production of recombinant proteins using Escherichia coli" APPLIED MICROBIOLOGY AND BIOTECHNOLOGY, vol. 64, no. 5, June 2004 (2004-06), pages 625-635, XP002402621 ISSN: 0175-7598

## Description

### CROSS-REFERENCE TO RELATED APPLICATION

This application claims the benefit (pursuant to 35 U.S.C. § 119(e)) of provisional application 60/647,013, filed in the United States Patent and Trademark Office on January 27, 2005.

### FIELD OF THE INVENTION

The present invention relates to leader sequences that are useful for production of heterologous secretable polypeptides; heterologous secreted polypeptides; nucleic acid constructs encoding such leader sequences; nucleic acid constructs encoding such heterologous secretable polypeptides; vectors that contain such nucleic acid constructs; recombinant host cells that contain such nucleic acid constructs, vectors and polypeptides; and methods of making such secretable polypeptides with such heterologous leader sequences; and methods of using such secretable polypeptides.

### BACKGROUND OF THE INVENTION

Proteins are the most prominent biomolecules in living organisms. In addition to being structural components and catalysts, they play crucial roles in regulatory processes. The cooperation of numerous cellular and extracellular proteins controls and affects the regulation of cell proliferation and metabolism. For example, many signal transduction pathways that affect physiological responses operate through proteins *via* intermolecular interactions.

Extracellular proteins, sometimes referred to as "secreted proteins," or "secretable proteins" herein, often function as intercellular signal communicators. In this role, they act as ligands. Their counterpart, the membrane-associated receptors that have extracellular, intracellular, or cytoplasmic domains, transmit extracellular signals into the cells when ligand/receptor binding events take place on the cell surfaces.

While receptors often make potentially important therapeutic targets, secretable proteins are of particular interest as therapeutic agents. Because of their frequent involvement in signaling or hormonal pathways, secretable proteins tend to exhibit high and specific biological activities (*Schoen,* 1994). For example, secretable proteins have been reported to control or regulate physiological processes such as differentiation and proliferation, blood clotting and thrombolysis, somatic growth and cell death, as well as various immune responses *(Id.).* Significant resources and research efforts have been expended to discovering new secretable proteins and investigating their regulatory functions. Some of these secretable proteins, including cytokines and peptide hormones, have been manufactured and used as therapeutic agents (*Zavyalov* et al. 1997), but they constitute a minority amongst the thousands of proteins that are expected to be secreted and potentially efficacious therapeutically.

Typically, a secretable protein is expressed as a full-length polypeptide, sometimes referred to as a "protein precursor," which is then processed in the Endoplasmic Reticulum (ER) and the Golgi in the post-translational phase. During this phase, a signal peptidase cleaves off a characteristic hydrophobic amino acid sequence at the N-terminus, a sequence that is generally referred to as a "signal peptide" (SP) or a "secretory leader sequence." A typical SP is about 16 to 30 amino acid residues in length. The resulting polypeptide sans the SP is then exported to outside the cell. The resulting polypeptide is called a "mature protein" or a "secreted polypeptide." And compared to the original secretable protein, this mature protein lacks the signal peptide sequence. Some proteins do not have an SP at the N-terminus, such as some of the members in the fibroblast growth factor family.

Naturally-occurring secretable proteins are expressed in varying amounts depending on their physiological roles *in vivo.* Many of them, under the regulation of their natural or endogenous SP, are expressed in quantities that are too low to be used commercially. It would therefore be advantageous if nucleic acid constructs and methods are devised to enable the production of secretory proteins *in vivo* or *in vitro* to meet the manufacturing needs for therapeutic applications.
Reference is made to the following documents: WO 2005/105840 published 10 November 2005, and Oritani et al "Identification Of Stromal Cell Products That Interact With Pre-B Cells" J. Cell Biol. 134; 771-782, 1996.

### SUMMARY OF THE INVENTION

The present invention relates to nucleic acid and polypeptide constructs for producing proteins in higher yields than when such proteins are produced from sequences that comprise their endogenous signal peptide. The present invention also relates to vectors, host cells and methods for producing proteins in higher yields than when such proteins are produced from DNA sequences that encode the protein with its endogenous signal peptide or without an endogenous signal peptide; the higher yield being achieved either by replacing the endogenous secretory leader sequence with an heterologous secretory leader sequence of the invention, or by adding a heterologous secretory leader sequence of the invention to a protein that would otherwise not contain a leader sequence. Accordingly, the present invention relates to polypeptide and polynucleotide constructs where the polypeptides and polynucleotides are modified, such as to form a fusion molecule with a fusion partner. The fusion molecules of the invention may be prepared by any conventional technique.

The present invention thus provides a heterologous polypeptide comprising a secretory leader and a second polypeptide, wherein the secretory leader is operably linked to the N-terminus of the second polypeptide, wherein the secretory leader is not so linked to the second polypeptide in nature, and wherein the secretory leader comprises a leader sequence of a secretable protein, and the secretable protein is collagen type IX alpha 1 chain.

The secretory leader may comprises an amino acid sequence of SEQ ID NO: 27.

The second polypeptide may be selected from a second secretable polypeptide, an extracellular portion of a transmembrane protein, and a soluble receptor; for example the second secretable polypeptide may be selected from a growth factor, a cytokine, a lymphokine, an interferon, a hormone, a stimulatory factor, an inhibitory factor, and splice variants thereof.

The heterologous polypeptide of the invention may further comprise a fusion partner, such as a polymer. This polymer may be a third molecule, and wherein the third molecule is selected from polyethylene glycol and all or part of human serum albumin, fetuin A, fetuin B and Fc.

The invention also provides an isolated nucleic acid molecule comprising a polynucleotide sequence, wherein the polynucleotide sequence encodes an amino acid sequence of a heterologous polypeptide of the invention, wherein the leader sequence is encoded by a first polynucleotide and the second polypeptide is encoded by a second polynucleotide, the first polynucleotide and the second polynucleotide are operably linked to facilitate secretion of the heterologous polypeptide from a cell, and wherein the first and second polynucleotide are not so linked in nature.

The invention further provides a nucleic acid molecule encoding a heterologous polypeptide, comprising a first polynucleotide that encodes the leader sequence of collagen type IX alpha 1 chain and a second polynucleotide that encodes a second polypeptide, wherein the first polynucleotide and the second polynucleotide are operably linked to facilitate secretion of the heterologous polypeptide from a cell, and wherein the first and second polynucleotide are not so linked in nature. The leader sequence of collagen type IX alpha 1 chain may comprise an amino acid sequence of SEQ ID NO: 27.

In such a nucleic acid of the invention, the second polypeptide may be selected from a secretable polypeptide, an extracellular portion of a transmembrane protein, and a soluble receptor.

The nucleic acid may further comprise a third polynucleotide, wherein the third polynucleotide is a Kozak sequence or a fragment thereof that is situated at its 5' end.

The nucleic acid may further comprise a fourth polynucleotide, wherein the fourth polynucleotide comprises a restriction enzyme-cleavable sequence at its 3' end.

The nucleic acid may further comprise a fifth polynucleotide, that encodes a tag, e.g. a purification tag or a tag selected from V5, HisX6V(SEQ ID NO: 257), HisX8 (SEQ ID NO: 258, an avidin molecule, and a biotin molecule.

The nucleic acid comprising a fourth polynucleotide may further comprise a sixth polynucleotide, that encodes a second enzyme-cleavable sequence that can be cleaved by a second enzyme, wherein the second cleavable sequence is situated upstream of the tag if the tag is situated at the C-terminus of the heterologous polypeptide, or downstream of the tag if the tag is situated at the N-terminus of the heterologous polypeptide.

The second enzyme may be thrombin or TEV from a tobacco virus.

The invention further provides a vector comprising the nucleic acid molecule of the invention described above and further comprising an origin of replication and a selectable marker. In such a vector the origin of replication may be selected from SV40 ori, Pol ori, EBNA ori, and pMB1 ori. In such a vector the selectable marker may be an antibiotic resistance gene such as a puromycin resistance, kanamycin resistance, and ampicillin resistance gene.

The invention further provides a recombinant host cell comprising a cell and the heterologous polypeptide of the invention as set out above, the nucleic acid molecule of the invention as set out above, or the vector of the invention as set out above. The recombinant host cell may be a eukaryotic cell such as a mammalian cell.

The invention further provides a method of producing a secreted polypeptide, comprising:
(a) providing the nucleic acid molecule of the invention as set out above; and
(b) expressing the nucleic acid molecule in an expression system.

The expression system may be a cellular expression system and the cell a mammalian cell. Mammalian cells include a 293 cell line (e.g. a 293-T or 293-6E cell), a PER.C6^{®} cell line and a CHO cell line.

Examples of a second polypeptide which is a secretable protein include alpha-2-antiplasmin precursor (alpha-2-plasmin inhibitor), trinucleotide repeat containing 5,ARMET protein, calumenin, COL9AI protein, NBLI, PACAP protein, alpha-1H-glycoprotein precursor (alpha-1B glycoprotein), similar to brain-specific angiogenesis inhibitor 2 precursor, SPOCK2, protein disulfide-isomerase (EC 5341) ER60 precursor, serine (or cysteine) proteinase inhibitor, clade A (alpha-1), GM2 ganglioside activator precursor, coagulation factor X precursor, secreted phosphoprotein 1 (osteopontin, bone sialoprotein 1), Vitamin D-binding protein precursor, interleukin 6 (interferon, beta 2), orosomucoid 1 precursor, hemopexin, glycoprotein hormones, alpha polypeptide precursor, kininogen 1, proly14-hydroxylase, beta subunit, proopiomelanocortin, prostaglandin D2 synthase 21 kDa, alpha-2-glycoprotein 1, zinc, chromogranin A, cystatin M precursor, clusterin isoform 1, inter-alpha (globulin) inhibitor HI, leukemia inhibitory factor (cholinergic differentiation factor), lumican, secretoglobin, family 2A, member 2, nov precursor, reticulocalbin 1 precursor, reticulocalbin 2, EF-hand calcium binding domain, gastric intrinsic factor (vitamin B synthesis), cerberus 1, lipocalin 2 (oncogene 24p3), interleukin 18 binding protein isoform C precursor, cell growth regulator with EF hand domain 1, leukocyte immunoglobulin-like receptor, subfamily A, spondin 2, extracellular matrix protein, transmembrane protein 4, parc/osteonectin, cwcv and kazal-like domain proteoglycan, Rho GTPase activating protein 25 isoform b, dickkopfhomolog 3, ameloblastin precursor, chorionic gonadotropin, beta polypeptide 8 precursor, multiple coagulation factor deficiency 2, similar to common salivary protein 1, hypothetical protein FLJ321 15, oncoprotein-induced transcript 3, hypothetical protein MGC40499, interleukin 18 binding protein isoform A precursor, interleukin 1 receptor antagonist isoform 1 precursor, WFIKKN2 protein, similar to hypothetical protein 9330140G23, and SEQ ID NOs: 2-3, 9, 19, 22, 26, 31, 37, 41, 47, 54, 57, 62, 68, 75, 79, 82, 86, 88, 94, 97, 102, 104, 107, 111, 116, 120, 127, 131, 137, 140, 145, 147, 153, 159, 167, 175, 177, 181, 185, 189, 191, 196, 200, 207, 209, 215, 218, 222, 227, 232, 235, 239, 241, 245, 248, and 254.

### Description of the Figures

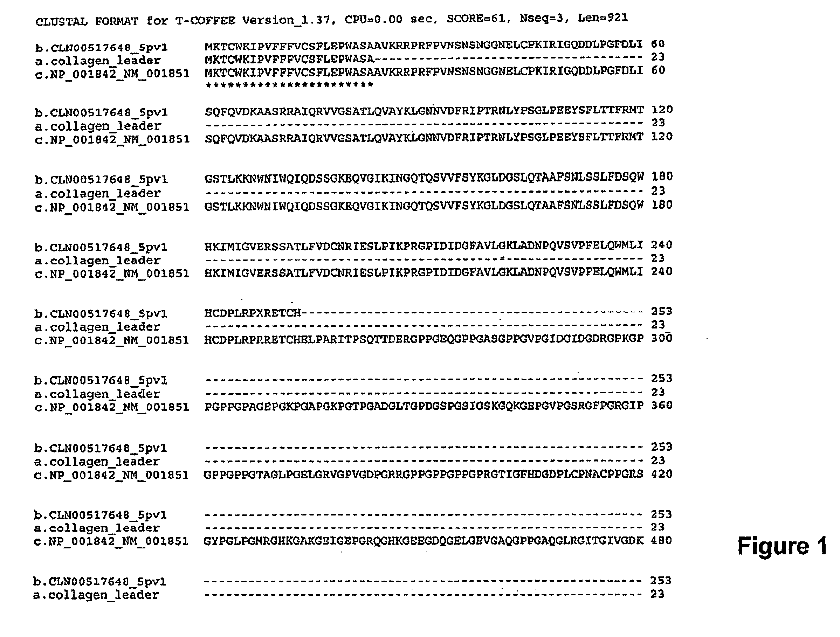

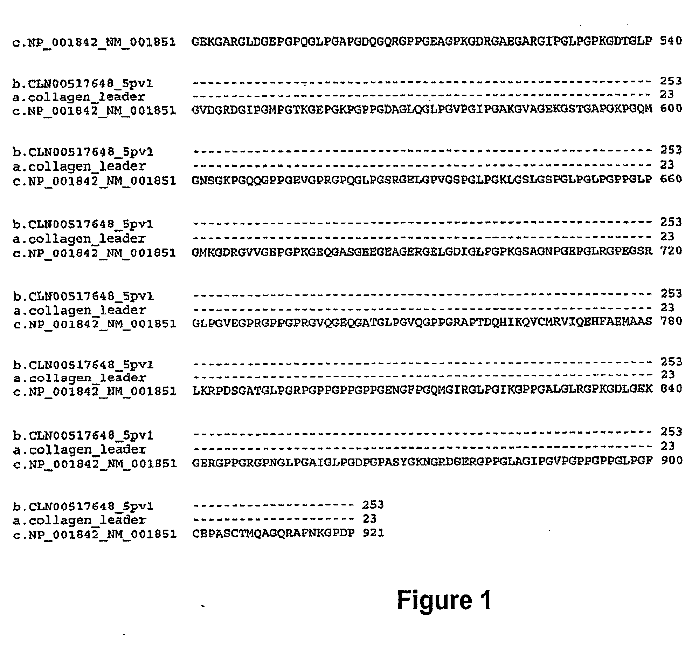

**FIGURE 1****:** is an alignment of the amino acid sequences of: (a) a leader sequence ("collagen_leader"); (b) a cDNA clone previously designated as MGC:21955 having an annotation of an unknown protein, and designated herein as CLN00517648; and (c) a publicly accessible sequence NP_001 842_NM_001851, corresponding to collagen type IX alpha I chain, long form *Hiomo Sapiens*)*.* These sequences all start with a methionine ("M") as amino acid residue 1 at the N terminus. This clone CLN00517648_5pv1 was sequenced and found to contain 253 amino acid residues.

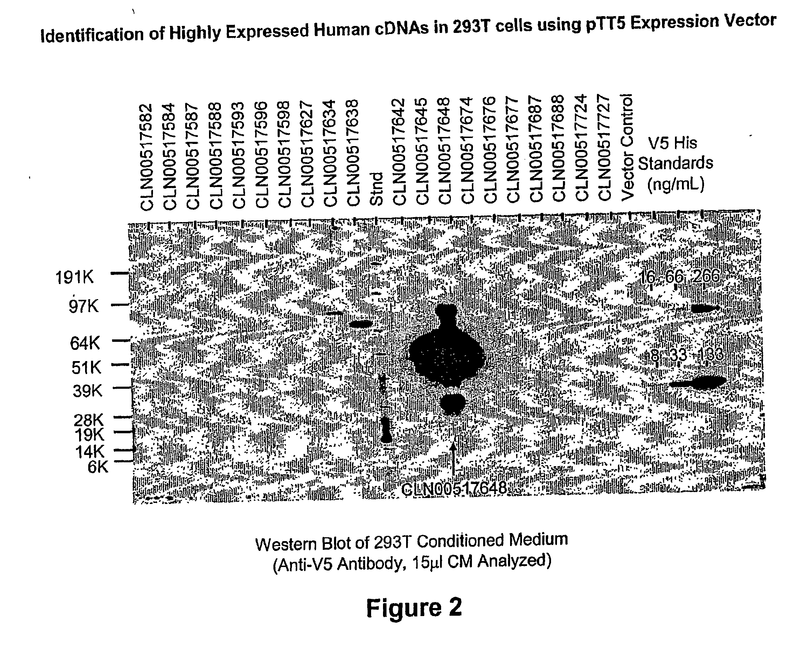

**FIGURE 2**: is a Western blot showing expression of several secretable polypeptides in media conditioned by cultured 293-T cells, which are transfected with cDNAs encoding proteins of the invention, subcloned into a pTT5 vector (as described in greater detail in Examples 2-4). The construct expressing the secretable protein encoded by clone CLN00517648 demonstrated the highest level of protein secretion in the conditioned media. The amount of protein secreted into the conditioned media was compared to two standards: (1) V5-Hisx6 tagged Delta-like protein 1 extracellular protein (15 µl at 16, 66, and 266 ng/ml); and (2) V5-Hisx6 tagged CSF-1 Receptor extracellular domain (15 µl at 8, 33, and 133 ng/ml). These standards were mixed and loaded into the three right hand lanes at the designated concentrations.

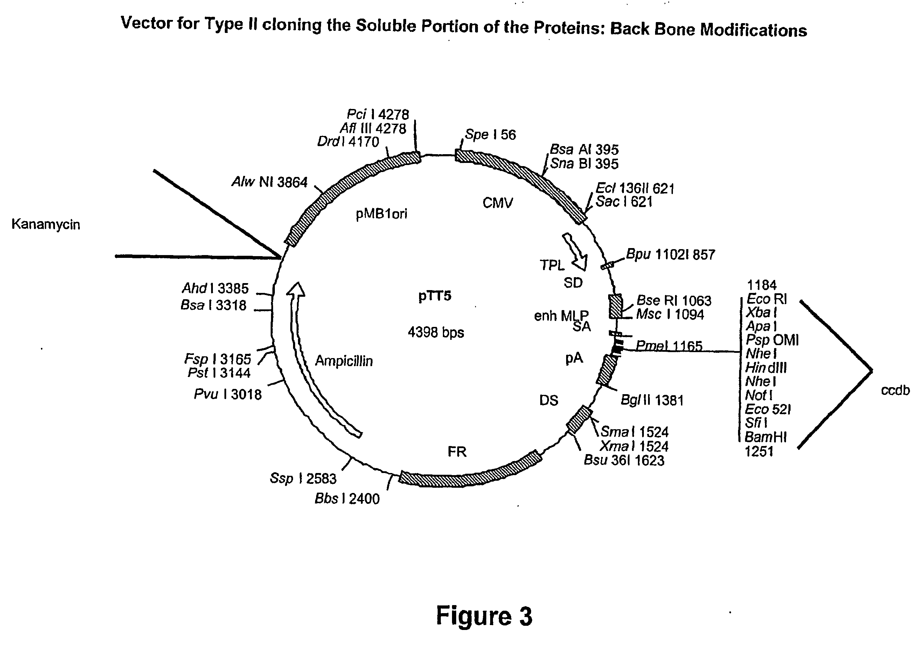

**FIGURE 3****:** is a diagrammatic representation of a starting vector pTT5 (4398 bps) provided by Dr. Yves Durocher (Durocher, 2002).

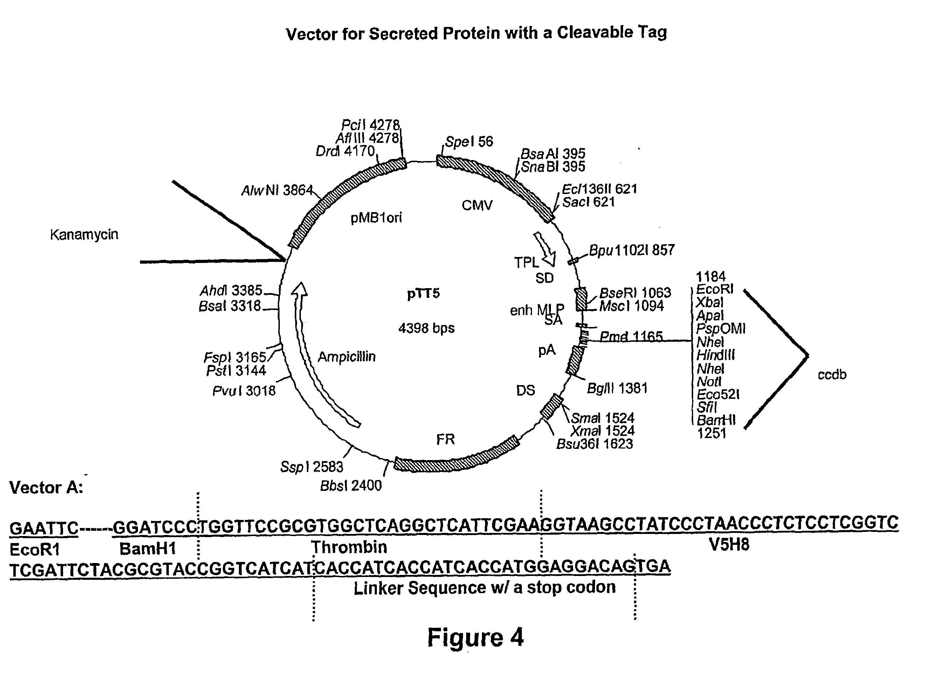

**FIGURE 4****:** shows the sequence of Vector A, which is inserted into the pTT5 vector to replace the "ccdb" region for the purpose of this invention. Vector A includes from left to right: an EcoR I site; the open reading frame (ORF), or the gene of interest encoding the mature polypeptide, which is represented by "-----;" a BamH1 site; a cleavable sequence exemplified by a sequence encoding a thrombin cleavage site; a tag exemplified by V5H8; and a linker sequence followed by a stop codon.

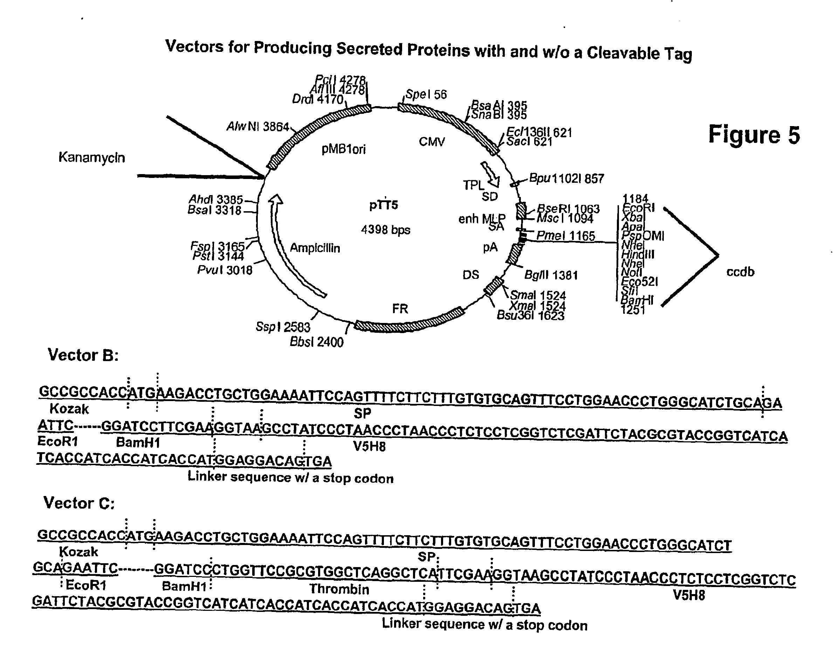

**FIGURE 5****:** shows sequences for Vector B and Vector C. Vector B includes, from left to right: a Kozak sequence, a leader sequence ("SP") such as the collagen leader sequence of the present invention, an EcoR1 site, the ORF or the gene of interest encoding the mature polypeptide as represented by "------," a BamH1 site, a tag such as V5H8, and a linker sequence followed by a stop codon. Vector C includes, from left to right: a Kozak sequence, a leader sequence ("SP") exemplified by the collagen leader sequence of the present invention, an EcoR1 site, the ORF or the gene of interest encoding the mature polypeptide as represented by "------," a BamH1 site, a cleavable sequence exemplified by a sequence encoding thrombin, a tag such as V5H8, and a linker sequence followed by a stop codon.

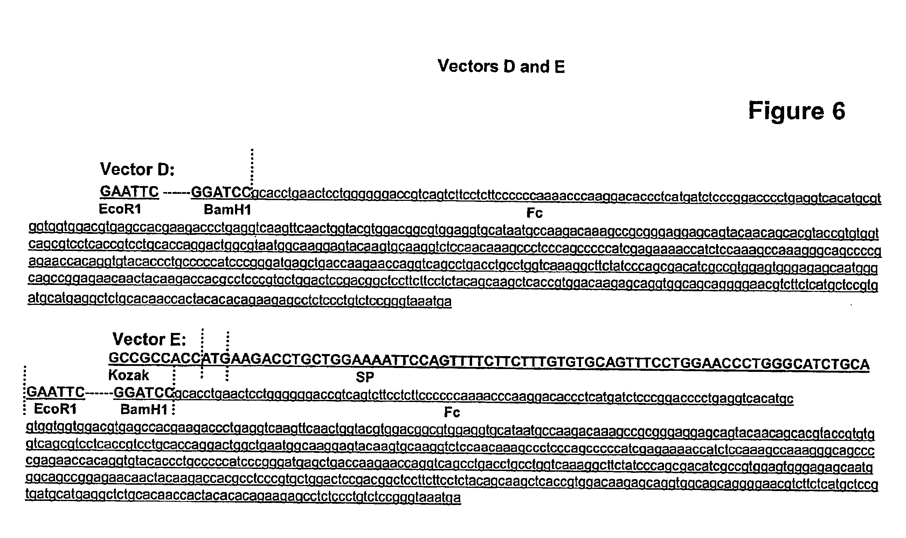

**FIGURE 6****:** shows sequences for Vector D and Vector E. Vector D includes, from left to right: an EcoR1 site, the ORF or the gene of interest encoding the mature polypeptide as represented by "------," a BamH1 site, and an Fc domain sequence followed by a stop codon. Vector E includes, from left to right: a Kozak sequence ("GCCGCCACC"), a signal peptide/leader sequence of the invention, an EcoR1 site, the ORF or the gene of interest encoding the mature polypeptide as represented by "------," a DamH1 site, and an Fc domain sequence followed by a stop codon.

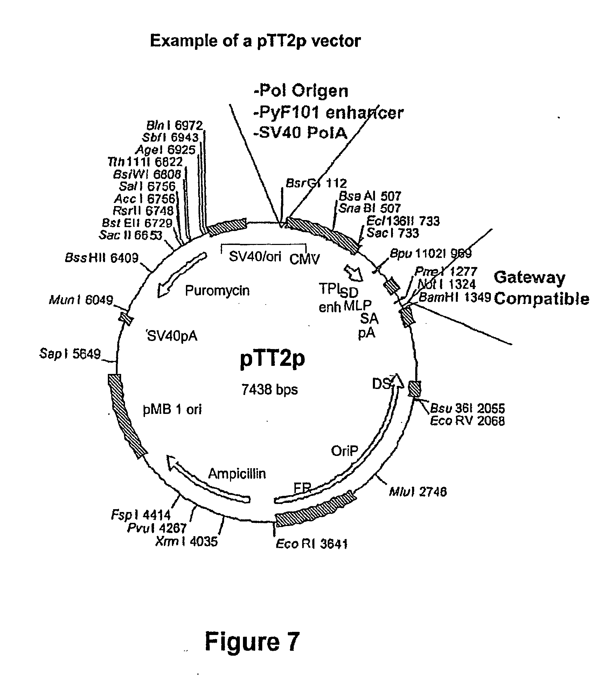

**FIGURE 7****:** is an example of a pTT2p vector for making stable puromycin-resistant cell lines. Specifically, the pTT2p vector includes, *inter alia,* murine polyoma signals to make an episomal pTT2-gateway vector.

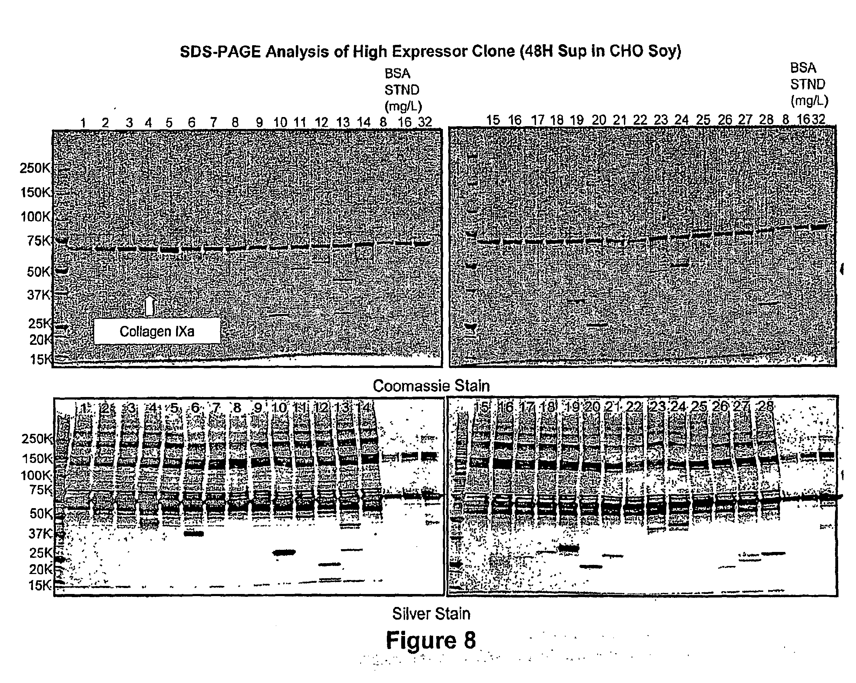

**FIGURE 8****:** shows an SDS-PAGE analysis of protein expression in CHO SOY medium, employing 28 of the secretable proteins described herein. The top two (2) panels show SDS-PAGE developed with Coomassie stain and the bottom two (2) panels show SDS-PAGE developed with silver stain. Table 3, columns 6-11, identifies the specific leader sequence represented in each SDS-PAGE lane. In the three right-hand lanes, a bovine serum albumin (BSA) standard was run at concentrations that reflect corresponding expression levels of 8, 16, and 32 milligrams/liter (mg/L), respectively.

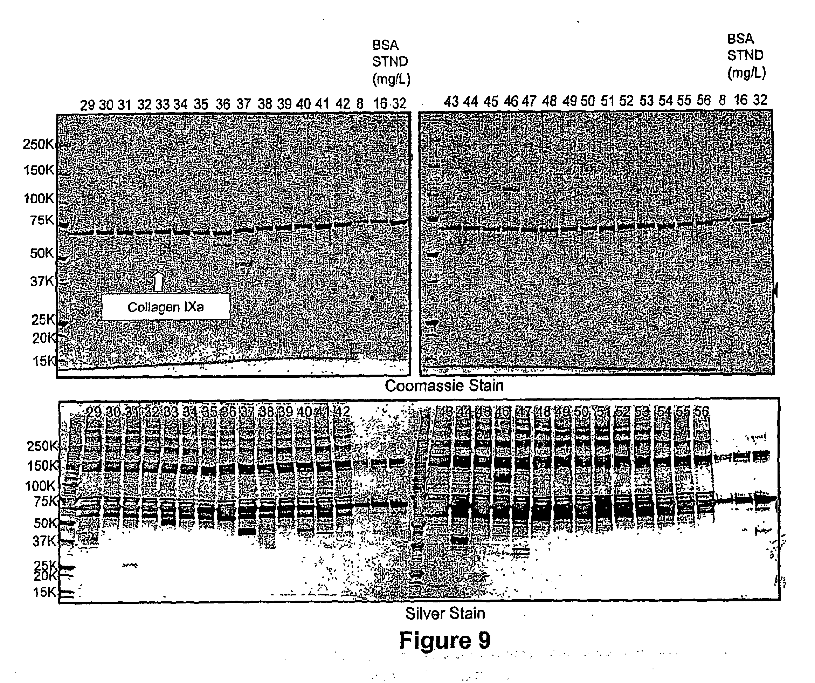

**FIGURE 9****:** shows an SDS-PAGE analysis of protein expression in CHO SOY medium, employing the secretable proteins of 29-56 as described herein. The top two (2) panels show SDS-PAGE developed with Coomassie stain and the bottom two (2) panels show SDS-PAGE developed with silver stain. Table 3, columns 6-11, identifies the specific secretable protein represented in each SDS-PAGE lane. A bovine serum albumin (BSA) standard was run at concentrations that reflect corresponding expression levels of 8, 16, and 32 milligrams/liter (mg/L).

**Table 1:** lists information regarding the secretable proteins from which leader sequences are derived. Column 1 lists the internal designation identification numbers; column 2 lists the reference identification numbers; column 3 lists the identities of the secretable proteins.

**Table 2:** lists information regarding the leader sequences of the invention. Column 1 lists the internal designation identification numbers; column 2 lists the SEQ ID NOs. for the leader sequences (P1); column 3 lists the reference identification numbers; column 4 lists the leader sequence types, *i.e.,* full length versus alternative leader sequences; and column 5 lists the secretable proteins from which the leader sequences are derived.

**Table 3:** summarizes the results obtained with the leader sequences of the current invention. Column 1 lists the clone designation identification numbers; column 2 lists the protein concentrations in micrograms/milliliter (µg/ml) as detected and measured from the Coomassie-stained SDS-PAGE; column 3 ranks the expression levels as measured by Coomassie-stained SDS-PAGE, silver stained SDS-PAGE, or quantitative Western Blot using an Anti-V5 antibody relative to purified V5-tagged protein standards, of each construct on a scale of 1 to 56, from the lowest at 56 to the highest at 1; column 4 lists whether a band was detected using silver-stain developed SDS-PAGE; column 5 lists the molecular weights of the tested secretable proteins in Daltons; column 6 lists the gel numbers and lane numbers corresponding to Figures 8-9; column 7 lists the internal designations for the secretable proteins; column 8 lists protein identification numbers; column 9 lists the internal designation identification numbers; column 10 lists the source identification numbers; column 11 identifies the secretable proteins.

**Appendix A**/Sequence Listing lists the amino acid sequences of the leader sequences (P1) in Table 2.

### Detailed Description of the Invention

To express and secrete the proteins of interest in larger quantities (*e.g.*, about 10% more, 20% more, 30% more, or a higher percentage more) than those obtained when the proteins are expressed and secreted from DNA sequences that encode their full-length amino acid sequence and contain their endogenous signal peptide, the inventors replaced their endogenous secretory leader sequence with that from another, *i.e.*, different or heterologous, secretable protein. The latter secretable protein of interest is typically one that is expressed and/or secreted at high levels ("high expressor protein" or "high secretor protein"), or moderately high levels ("moderate expressor protein" or "moderate secretor protein") under typical conditions for assaying protein expression and secretion, which are not limited to those described in detail in the Examples of the invention. In other words, if one were to express a panel of proteins (including but are not limited to those listed in this specification, in Appendix A, and those listed in Tables 1-3), and all were expressed under the same assay conditions, one would find that some proteins are expressed and/or secreted at higher levels than others. Accordingly, it is an aspect of the invention to recognize the differences in expression and secretion levels among the proteins of the invention, and take advantage of these recognized differences to further identify from the leader sequences those that are useful for improving the secretion and/or expression of otherwise low expressor proteins, or of proteins that are not secreted at the desirable levels.

Employing heterologous secretory leader sequences is further advantageous in that, during the secretion process, the resulting mature amino acid sequence of the secretable polypeptide is not altered as the secretory leader sequence is removed in the endoplasmic reticulum (ER) or the Golgi. A secretory leader sequence of the invention serves to direct certain proteins to the ER. The ER separates the membrane-bounded proteins from all other types of proteins amongst those comprising the leader sequences. Each group is then separately moved to the Golgi apparatus. The Golgi apparatus then distributes the proteins to vesicles such as secretory vesicles, the cell membranes, the lysosomes, or other organelles.

Moreover, the addition of a heterologous secretory leader facilitates the expression and secretion of the extracellular domains of transmembrane proteins. An example of such a transmembrane protein is the Type II single transmembrane proteins (STM), the secretory leader of which is also the transmembrane domain, which must be removed before the protein becomes soluble and secreted.

Thus, to identify robust secretory leader sequence(s), which enhance or improve the secretion and expression of proteins relative to that achieved by the endogenous leader sequence, and which optionally can be used universally for making secretable proteins, many different secretable proteins have been cloned and expressed, as described herein. The expression and secretion levels of the cloned and expressed proteins in the supernatant of the mammalian 293 cells have also been measured, the results of which are shown in, for example, Example 1, Figures 8-9, and Table 3. Several high-expressor and high-secretor proteins were observed. The high-expressor proteins may or may not be the same as the high-secretor proteins for the purposes of this invention.

In one embodiment, a secretory leader sequence that is a part of the secretable protein collagen type IX alpha I chain, long form, has been identified. This particular leader sequence was selected to further examine its ability to promote expression and secretion when used as a heterologous and/or universal secretory leader sequence. The amino acid sequence of the secretory leader, which is part of the secretable protein collagen type IX alpha I chain, long form, is predicted to be MKTCWKIPVFFFVCSFLEPWASA (SEQ ID NO: 1). As further described herein, vectors were constructed to comprise this particular secretory leader. Using these vectors, several proteins were cloned without their own naturally-existing secretory leaders, yielding secretable proteins with a heterologous secretory leader sequence. The expression and secretion levels of these fusion proteins were found to be about 10%, about 20%, about 30%, about 40%, about 50%, about 60%, about 70% or more higher than the expression or secretion levels as observed with their non-fusion counterparts.

The present invention may be more clearly understood in light of the following definitions. Generally, the terms used herein have their ordinary meanings and the meanings given them specifically below.

The terms "polynucleotide," "nucleotide," "nucleic acid," "nucleotide molecule," "nucleic acid molecule," "nucleic acid sequence," "polynucleotide sequence," and "nucleotide sequence" are used interchangeably herein to refer to polymeric forms of nucleotides of any length. The polynucleotides can contain deoxyribonucleotides, ribonucleotides, and/or their analogs or derivatives. For example, nucleic acids can be naturally occurring DNA or RNA, or can be synthetic analogs of the naturally occurring DNA or RNA, as known in the art. The terms also encompass genomic DNA; genes; gene fragments; exons; introns; regulatory sequences or regulatory elements, such as promoters, enhancers, initiation and termination regions, other control regions, expression regulatory factors and expression controls; isolated DNA; and cDNA. In addition, the terms encompass mRNA, tRNA, rRNA, ribozymes, splice variants, antisense RNA, antisense conjugates, RNAi, siRNA and isolated RNAs. The terms also encompass recombinant polynucleotides, heterologous polynucleotides, branched polynucleotides, labeled polynucleotides, DNA/RNA hybrids, polynucleotide constructs, vectors comprising the subject nucleic acids, nucleic acid probes, primers and primer pairs. The terms comprise modified nucleic acid molecules, such as analogs of purines and pyrimidines, with alterations in the backbones; sugars; or heterocyclic bases, such as methylated nucleic acid molecules; peptide nucleic acids; and nucleic acid molecule analogs, which may be suitable as, for example, probes if they demonstrate superior stability and/or binding affinity under assay conditions. Analogs of purines and pyrimidines, including radiolabeled and fluorescent analogs, are known in the art. The polynucleotides can have any three-dimensional structure. The terms also encompass single-stranded, double-stranded and triple-helical molecules that are DNA, RNA, or hybrid DNA/RNA, and that may encode a full-length gene or a biologically active fragment thereof. Biologically active fragments of polynucleotides can encode the polypeptides herein, as well as anti-sense, ribozymes, or RNAi molecules. Thus, for example, the full length polynucleotides herein may be treated with enzymes, such as Dicer, to generate a library of short RNAi fragments, which are also within the scope of the present invention.

The terms "polypeptide," "peptide," and "protein," used interchangeably herein, refer to a polymeric form of amino acids of any length. The amino acids can include naturally-occurring amino acids; coded and non-coded amino acids; chemically or biochemically modified, derivatized, or designer amino acids; amino acid analogs; peptidomimetics and depsipeptides; and polypeptides having modified, cyclic, bicyclic, depsicyclic, or depsibicyclic peptide backbones. The terms may also refer to conjugated proteins; fusion proteins, including, but not limited to, GST fusion proteins, fusion proteins with a heterologous amino acid sequence, fusion proteins with heterologous and homologous leader sequences, fusion proteins with or without N-terminal methionine residues; pegylated proteins; and immunologically tagged proteins. Also included in the terms are variations of naturally occurring proteins, where such variations are homologous or substantially similar to the naturally occurring proteins, as well as their corresponding homologs from different species. Variants of polypeptide sequences include insertions, additions, deletions, or substitutions when compared with the original polypeptides, but nonetheless retaining the same type of biological activity albeit possibly at a different level. The term also includes peptide aptamers.

A "secretory leader," "signal peptide," or a "leader sequence," contains a sequence comprising amino acid residues that directs the intracellular trafficking of the polypeptide to which it is a part. Polypeptides contain secretory leaders, signal peptides or leader sequences, typically at their N-terminus. These polypeptides may also contain cleavage sites where the secretory leaders, signal peptides or leader sequences may be cleaved from the rest of the polypeptides by signal endopeptidases. Such polypeptides, after cleavage at the cleavage sites, generate mature polypeptides. Cleavage typically takes place during secretion or after the intact polypeptide has been directed to the appropriate cellular compartment.

According to the invention, a "high secretor signal peptide/secretory leader sequence" is one that (i) can be operably linked to a protein as an heterologous sequence, thereby replacing its endogenous signal peptide; and (ii) is capable of enhancing the level of secretion of the protein at least about 5 fold, when compared to the level of secretion that the protein exhibits when it carries its endogenous SP.

Also according to the invention, a "moderate secretor signal peptide/secretory leader sequence" is one that (i) can be operably linked to a protein as an heterologous sequence, thereby replacing its endogenous signal peptide; and (ii) is capable of enhancing the level of secretion of the protein about 2 to 5 fold, when compared to the level of secretion that the protein exhibits when it carries its endogenous SP.

Further according to the invention, a "low secretor signal peptide/secretory leader" is one that (i) can be operably linked to a protein as an heterologous sequence, thereby replacing its endogenous signal peptide; and (ii) is capable of enhancing the level of secretion of the protein less than about 2 fold or does not enhance the level of secretion of the protein when compared to the level of secretion that the protein exhibits when it carries its endogenous SP.

Moreover, a secretory leader of the invention can also be added to a protein which is otherwise not predicted to be secreted via the ER-Golgi and does not have an endogenous signal peptide. In this case, the above definitions of "high/moderate/low secretor signal peptide/secretory leader sequence" are not applicable since there is no baseline secretion level for the protein that can be used for comparison purposes. In this case, the effect that the addition of the signal peptide/secretory leader sequence has on the secretion of an otherwise non-secretable protein will be compared among the resulting heterologous proteins.

For the purpose of this invention, the above definitions of "high/moderate/low secretor signal peptide/secretory leader sequence" relate only to the signal peptide (or secretory leader sequences). They do not relate to "high secretor proteins," "moderate secretor proteins" or "low secretor proteins". The proteins themselves were ranked as such on a basis of a relative scale that served to rank all the proteins of the invention (Tables 1-3 and Appendix A) relatively to each other, with regards to their own expression and secretion levels in either wheat germ extracts, or mammalian cells (see Examples 1-3 for detailed explanation).

A "secretable" protein is one capable of being directed to the ER, secretory vesicles, or the extracellular space by a secretory leader, signal peptide, or leader sequence. It may also be one that is released into the extracellular space without necessarily containing a signal sequence. If the secretable protein is one that is released into the extracellular space, it can undergo processing to produce a "mature" polypeptide." Proteins that contain transmembrane domains and typically remain inserted into the plasma membrane are considered, for the purposes of the invention, secretable proteins because they are also synthesized in the ER-Golgi, and some fragments or parts of such proteins can be released into the extracellular compartment, for example, by proteolytic cleavage. Thus, release into the extracellular space can occur in multiple ways, including, for example, exocytosis and proteolytic cleavage.

The terms "mature protein" and "secreted protein" are used interchangeably herein, and refer to the form(s) of a secretable protein after it is secreted to the outside of the cell (for example, into the media conditioned by cells in culture). Typically, the mature protein has the amino acid sequence of the secretable protein sans the signal peptide. However, when a protein is expressed in nature or recombinantly, parts of the signal peptides are often not removed, resulting in a mature-protein mixture that may contain many forms of the mature protein, attached to varying lengths of the signal peptides. Thus, multiple "mature forms" can exist for a secretable protein depending on the specific amino acids cleaved off by the signal endopeptidase. Other proteases can also cleave off amino acids from a secretable protein, further adding to the heterogeneity of its "mature-protein." The exact place where a signal peptide has been removed from a particular protein sample may be determined by N-terminal protein sequencing or otherwise by standard methods known to those skilled in the art.

A "biologically active" entity, or an entity having "biological activity," is one that has the structural, regulatory, or biochemical functions of a naturally occurring molecule, or one that has the functions related to or associated with a metabolic or physiological process. A biologically active polynucleotide fragment or polypeptide fragment according to this invention is one that exhibits activities similar, but not necessary identical, to the activities of the counterpart polynucleotide or polypeptide, to which the fragment is a part. Biological activities may include, but are not limited to, an improved desired activity and a decreased undesirable activity. For example, an entity demonstrates biological activity when it participates in molecular interactions with other molecules. An example of such an interaction is hybridization. Another example of such an interaction may be the exhibition of therapeutic effectiveness in alleviating a disease condition, or prophylactic effectiveness in inducing an immune response to the molecule. Another example of such an interaction may be the demonstration of potential uses as diagnostic tools in determining the presence of the molecule, for example, when the active fragment of a polynucleotide or a polypeptide is unique to the polynucleotide or the polypeptide, allowing the detection of the polynucleotide or the polypeptide by detecting fragment. A biologically active polypeptide or fragment thereof includes one that can participate in a biological reaction, for example, one that can serve as an epitope or immunogen to stimulate an immune response, which includes but is not limited to the production of antibodies; or one that participates in signal transduction pathways by binding to receptors, proteins, or nucleic acids; or one that activates enzymes or substrates. Yet another example of such an interaction may be the suitability of using the polynucleotide molecule as a primer in PCR.

An "isolated" or "substantially isolated" polynucleotide or polypeptide, or a polynucleotide or polypeptide in "substantially pure form," in "substantially purified form," or as an "isolate," is one that is substantially free of the sequences with which it is associated in nature, or of other nucleic acid sequences that do not include a sequence or fragment of the subject polynucleotide or polypeptide. "Substantially free" means that less than about 10%, less than about 20%, less than about 30%, less than about 40%, or less than about 50%, of the composition is composed of the undesired materials.

"Operably linked" refers to an arrangement of elements wherein the components so described are configured so as to perform their desired function. Thus, a given promoter operably linked to a coding sequence is capable of effecting the expression of the coding sequence when the proper transcription factors and conditions are present. The promoter need not be contiguous with the coding sequence, so long as it functions to direct the expression thereof. Thus, for example, intervening untranslated yet transcribed sequences can be present between the promoter sequence and the coding sequence, as can translated introns, and the promoter sequence can still be considered "operably linked" to the coding sequence.

"Recombinant," when used to describe a nucleic acid molecule, means a polynucleotide of genomic, cDNA, viral, or synthetic origin which, by virtue of its origin or manipulation, is not associated with all or a portion of the polynucleotide with which it is associated in nature. The term "recombinant," when used to describe a protein or polypeptide, means a polypeptide produced by expression of a recombinant polynucleotide.

A "control element" refers to a polynucleotide sequence that aids in the expression of a coding sequence to which it is linked. The term may refer to promoters, transcription termination sequences, upstream regulatory domains, polyadenylation signals, and when appropriate, leader sequences and enhancers, which collectively provide for the transcription and translation of a coding sequence in a host cell.

A "promoter" as used herein refers to a DNA regulatory region capable of binding RNA polymerase in a mammalian cell and initiating transcription of a downstream (3' direction) coding sequence operably linked thereto. For purposes of the present invention, a promoter sequence includes the minimum number of bases or elements required to initiate transcription of a gene of interest at a level detectable above background. Within the promoter sequence is a transcription initiation site, as well as protein binding domains (consensus sequences) responsible for the binding of RNA polymerase. Eukaryotic promoters often, but not always, contain "TATA" boxes and "CAT" boxes. Promoters further include those that are naturally contiguous to nucleic acid molecules and those that are not naturally contiguous to nucleic acid molecules. Additionally, promoters may include inducible promoters; conditionally active promoters, such as a cre-lox promoter; constitutive promoters; and tissue specific promoters.

A "selectable marker" refers to a gene that confers one or more phenotypes on a cell expressing the marker, such that the cell can be identified in appropriate conditions under which the phenotypes associated with the markers are manifested and observable. Generally, a selectable marker allows selection of transformed cells based on their ability to thrive in the presence or absence of one or more chemicals and/or other agents that inhibit an essential cell function. Suitable markers, therefore, include genes coding for proteins that confer drug resistance or sensitivity thereto, impart color to, or change the antigenic characteristics of those cells transfected with a molecule encoding the selectable marker, when the transfected cells are grown in an appropriate selective medium. For example, selectable markers include: cytotoxic markers and drug resistance markers, whereby cells are selected by their ability to grow on media containing one or more of the cytotoxins or drugs; auxotrophic markers by which cells are selected by their ability to grow on defined media with or without particular nutrients or supplements, such as thymidine and hypoxanthine; metabolic markers by which cells are selected for phenotypes such as their abilities to grow on defined media containing the appropriate sugar as the sole carbon source; or markers that confer the abilities of forming colored colonies on chromogenic substrates or the abilities to fluoresce.

"Transformation," as used herein, refers to the insertion of a polynucleotide into a host cell, regardless of the method used for insertion, which may be, for example, transformation, transfection, infection, and the like. The introduced polynucleotide may be maintained as a nonintegrated vector, for example, an episome, or alternatively, may be integrated into the host genome.

A "gene" comprises a DNA region encoding a gene product, as well as all DNA sequence regions that regulate the production of the gene product, whether or not such regulatory sequence regions are adjacent to coding sequences that may or may not be transcribed. Accordingly, a gene may be, for example, a promoter sequence, a terminator, a translational regulatory sequence such as a ribosome binding site or an internal ribosome entry site, an enhancer, a silencer, an insulator, a boundary element, a replication origin, a matrix attachment site, or a locus control region.

"Gene expression" refers to the conversion of the information, contained in a gene, into a gene product. A gene product can be the direct transcriptional product of a gene (*e.g.*, mRNA, tRNA, rRNA, antisense RNA, ribozyme, structural RNA or any other type of RNA) or a protein produced by translating an mRNA. A gene product can also be an RNA that is modified, by a process such as capping, polyadenylation, methylation, or editing; or a protein modified by, for example, methylation, acetylation, phosphorylation, ubiquitination, ADP-ribosylation, myristilation, and glycosylation.

A "coding sequence" or a sequence that "encodes" a selected polypeptide, is a nucleic acid molecule that is transcribed (in the case of a DNA) and translated (in the case of an mRNA) into a polypeptide *in vivo,* when the sequence is placed under the control of one or more appropriate regulatory sequences. The coding sequence begins at a start codon at the 5' (amino) terminus and ends at a translation stop codon at the 3' (carboxy) terminus. A coding sequence can be, for example, a cDNA from viral, prokaryotic, or eukaryotic mRNA; a genomic DNA viral sequence (*e.g.* DNA viruses and retroviruses); a prokaryotic DNA; or a synthetic DNA sequence. A transcription termination sequence may be located at a position that is 3' to the coding sequence.

A "fragment" refers to a polypeptide or polynucleotide comprising only a part of the sequence and structure of an intact full-length polypeptide or polynucleotide. The polypeptide fragment can comprise a C-terminal deletion, an N-terminal deletion, and/or an internal deletion from the intact polypeptide. The polynucleotide fragment can comprise a 5' deletion, a 3' deletion, and/or an internal deletion from the intact polynucleotide. A fragment of a protein generally comprises at least about 5-10 contiguous amino acid residues of the full-length molecule, at least about 15-25 contiguous amino acid residues of the full-length molecule, and at least about 20-50 or more contiguous amino acid residues of the full-length molecule. A fragment of a polynucleotide generally comprises at least about 15-30 contiguous nucleotides of the full-length molecule, at least about 45-75 continuous nucleotides of the full-length molecule, and at least about 60-150 or more contiguous nucleotides of the full-length molecule. In a certain embodiment, the number of amino acid residues in the fragment may be any integer between 5 and the total number of amino acid residues in the full-length molecule. In another embodiment, the number of nucleotides in the polynucleotide fragment may be any integer between 15 and the total number of nucleotides in the full-length molecule.

The term "host cell" or "recombinant host cell" refers to an individual cell, cell line, cell culture, or a cell *in vivo,* which can be or has been a recipient of one or more polynucleotides or polypeptides of the invention, which may be, for example, a recombinant vector, an isolated polynucleotide, an antibody, or a fusion protein. Host cells may be progeny of a single host cell, and the progeny may not necessarily be identical in morphology, physiology, in total DNA, RNA, or in polypeptide complement to the original recipient cell, as a result of natural, accidental, or deliberate mutations and/or changes. Host cells can be prokaryotic or eukaryotic, including but are not limited to, mammalian, insect, amphibian, reptile, crustacean, avian, fish, plant and fungal cells. A host cell may be a cell that is transformed, transfected, transduced, or infected *in vivo* or *in vitro* with a polynucleotide of the invention such as a recombinant vector. A host cell that comprises a recombinant vector of the invention may be called a "recombinant host cell."

The term "receptor" refers to a polypeptide that binds to a specific extracellular molecule and this binding may initiate a cellular response.

The term "ligand" refers to a molecule that binds to a specific site on another molecule.

It is to be understood that both the foregoing general description and the following detailed description are exemplary and explanatory only and are not restrictive of the invention as claimed. Moreover, it must be understood that the invention is not limited to the particular embodiments described, as the embodiments may, of course, vary. Further, the terminology used to describe particular embodiments is not intended to be limiting, since the scope of the present invention will be limited only by its claim.

Unless defined otherwise, the meanings of all technical and scientific terms used herein are those commonly understood by one of ordinary skill in the art to which this invention belongs. One of ordinary skill in the art will appreciate that any methods and materials similar or equivalent to those described herein can also be used to practice or test the invention.

It must be noted that, as used herein and in the appended claims, the singular forms "a," "or," and "the" include plural referents unless the context clearly dictates otherwise. Thus, for example, reference to "a subject polypeptide" includes a plurality of such polypeptides and reference to "the agent" includes reference to one or more agents as well as equivalents thereof known to those skilled in the art.

Further, all numbers expressing quantities of ingredients; reaction conditions, % purity, polypeptide and polynucleotide lengths, and so forth, used in the specification and the claims, are modified by the term "about," unless otherwise indicated. Accordingly, the numerical parameters set forth in the specification and claims are approximations that may vary depending upon the desired properties of the present invention. At the very least, and not as an attempt to limit the application of the doctrine of equivalents to the scope of the claims, each numerical parameter should be construed in light of the number of reported significant digits, applying customary rounding techniques.

Nonetheless, the numerical values set forth in the specific examples are reported as precisely as possible. Any numerical value, however, inherently contains certain errors from the standard deviation of its experimental measurement.

### Leader Sequences

As described herein, secretory leader sequences, which are identified from secretable proteins, are demonstrated to be useful for producing proteins at an amount that is about 5% higher, about 10% higher, about 20% higher, about 30% higher, about 40% higher, or about 50% or more higher, than when such proteins are produced under the same conditions from DNA sequences that contain the protein's endogenous secretory leader sequence. Secretory leader sequences identified and described herein include, for example, those from the following secretable proteins: interleukin-9 precursor, T cell growth factor P40, P40 cytokine, triacylglycerol lipase, pancreatic precursor, somatoliberin precursor, vasopressin-neurophysin 2-copeptin precursor, beta-enoendorphin-dynorphin precursor, complement C2 precursor, small inducible cytokine A14 precursor, elastase 2A precursor, plasma serine protease inhibitor precursor, granulocyte-macrophage colony-stimulating factor precursor, interleukin-2 precursor, interleukin-3 precursor, alpha-fetoprotein precursor, alpha-2-HS-glycoprotein precursor, serum albumin precursor, inter-alpha-trypsin inhibitor light chain, serum amyloid P-component precursor, apolipoprotein A-II precursor, apolipoprotein D precursor, colipase precursor, carboxypeptidase A1 precursor, alpha-s1 casein precursor, beta casein precursor, cystatin SA precursor, follitropin beta chain precursor, glucagon precursor, complement factor H precursor, histidine-rich glycoprotein precursor, interleukin-5 precursor, alpha-lactalbumin precursor, Von Ebner's gland protein precursor, matrix Gla-protein precursor, alpha-1-acid glycoprotein 2 precursor, phospholipase A2 precursor, dendritic cell chemokine 1, statherin precursor, transthyretin precursor, apolipoprotein A-1 precursor, apolipoprotein C-III precursor, apolipoprotein E precursor, complement component C8 gamma chain precursor, serotransferrin precursor, beta-2-microglobulin precursor, neutrophils defensins 1 precursor, triacylglycerol lipase gastric precursor, haptoglobin precursor, neutrophils defensins 3 precursor, neuroblastoma suppressor of tumorigenicity 1 precursor, small inducible cytokine A13 precursor, CD5 antigen-like precursor, phospholipids transfer protein precursor, dickkopf related protein-4 precursor, elastase 2B precursor, alpha-1-acid glycoprotein 1 precursor, beta-2-glycoprotein 1 precursor, neutrophils gelatinase-associated lipocalin precursor, C-reactive protein precursor, interferon gamma precursor, kappa casein precursor, plasma retinol-binding protein precursor, interleukin-13 precursor, and any of the secretable proteins listed in Tables 1-3.

The above-identified secretory leader sequences, together with the vectors and methods of the invention, are useful in expressing a wide variety of polypeptides, including, for example, secretable polypeptides, extracellular proteins, transmembrane proteins, and receptors, such as a soluble receptor. Examples of such polypeptides include cytokines and growth factors, such as Interleukins 1 through 18; the interferons; the lymphokines; hormones; RANTS; lymphotoxin-β; Fas ligand; flt-3 ligand; ligand for receptor activator of NF-kappa B (RANKL); TNF-related apoptosis-inducing ligand (TRAIL); CD40 ligand; Ox40 ligand; 4-1BB ligand and other members of the TNF family; thymic stroma-derived lymphopoietin; stimulatory factors such as, for example, granulocyte colony stimulating factor and granulocyte-macrophage colony stimulating factor; inhibitory factors; mast cell growth factor, stem cell growth factor, epidermal growth factor, growth hormone, tumor necrosis factor; leukemia inhibitory factor; oncostatin-M; splice variants; and hematopoietic factors such as erythropoietin and thrombopoietin.

Descriptions of some of the proteins that can be expressed according to the invention may be found, for example, in HUMAN CYTOKINES: HANDBOOK FOR BASIC AND CLINICAL RESEARCH, Vol. II (Aggarwal and Gutterman, eds., Blackwell Sciences, Cambridge, MA 1998); in GROWTH FACTORS: A PRACTICAL APPROACH (McKay and Leigh, eds., Oxford University Press Inc., New York, NY 1993); and in THE CYTOKINE HANDBOOK (A.W. Thompson, ed., Academic Press, San Diego, CA 1991).

Receptors for any of the aforementioned proteins may also be expressed using secretory leader sequences, vectors and methods described herein. The receptors may include, for example, both forms of tumor necrosis factor receptor (referred to as p55 and p75), Interleukin-1 receptors (types 1 and 2), Interleukin-4 receptor, Interleukin-15 receptor, Interleukin-17 receptor, Interleukin-18 receptor, granulocyte-macrophage colony stimulating factor receptor, granulocyte colony stimulating factor receptor, receptors for oncostatin-M and leukemia inhibitory factor, receptor activator of NF-kappa B (RANK), receptors for TRAIL, and receptors that comprise death domains, such as Fas or Apoptosis-Inducing Receptor (AIR).

Other proteins can also be expressed using the secretory leader sequences, vectors and methods described herein. These proteins include, for example, cluster of differentiation antigens (referred to as "CD proteins" or "CD molecules") such as those disclosed in LEUKOCYTE TYPING VI (Proceedings of the VIth International Workshop and Conference; Kishimoto et al. eds.; Kobe, Japan 1996), or in the proceedings of subsequent workshops. Examples of CD molecules include CD27, CD30, CD39, CD40, and ligands thereto, such as the CD27 ligand, the CD30 ligand and the CD40 ligand. Several of these are members of the TNF receptor (TNFR) family, which includes 4-1BB and OX40; the ligands, including the 4-1BB ligand and the OX40 ligand, are often members of the TNF family. Accordingly, members of the TNF and TNFR families can be expressed using the secretory leader sequences, vectors and methods of the present invention.

Proteins that are enzymes may also be expressed employing the herein described secretory leader sequences, vectors and methods. These enzymes may include, for example, members of the metalloproteinase-disintegrin family; various kinases such as streptokinase, tissue plasminogen activator, Death Associated Kinase Containing Ankyrin Repeats, IKR 1, or IKR 2; TNF-alpha Converting Enzyme; and numerous other enzymes. Ligands for enzymes can also be expressed by applying the secretory leader sequences, vectors and methods of the instant invention.

The secretory leader sequences, vectors and methods described herein, are also useful for the expression of other types of recombinant proteins. These recombinant proteins may include, for example, immunoglobulin molecules or portions thereof, as well as chimeric antibodies (*e.g.*, antibodies that have human constant regions coupled to murine antigen-binding regions) or fragments thereof. Numerous techniques are known by which DNAs encoding immunoglobulin molecules can be manipulated to yield DNAs capable of encoding recombinant proteins such as single chain antibodies, antibodies with enhanced affinity, or other antibody-based polypeptides (*see, e.g., Larrick* et al. 1989; *Reichmann* et al. 1988; *Roberts* et al. 1987; *Verhoeyen* et al. 1988; *Chaudhary* et al. 1989).

### Vectors, Host Cells, and Protein Production

The present invention provides recombinant vectors that contain, for example, nucleic acid constructs that encode one or more secretory leader sequences of interest or selected heterologous polypeptides of interest that are not necessarily secretory leader sequences, and host cells that are genetically engineered to incorporate the recombinant vectors.

The vector of the invention may be one that contains a selectable marker for propagation in a host and a secretory leader sequence such as one of those listed in Table 1. Such selectable markers may be, for example, dihydrofolate reductase; G418; neomycin-, or puromycin-resistance for eukaryotic cell cultures; or tetracycline-, kanamycin-, puromycin-, or ampicillin-resistance for *E. coli* and other bacterial cultures.

The vector of the invention may be, for example, a phage, plasmid, viral, or retroviral vector. Generally, a plasmid vector is introduced in a precipitate form, such as a calcium phosphate precipitate, or in a complex comprising a charged lipid. If the vector is a virus, it may be packaged *in vitro* using an appropriate packaging cell line, and then incorporated into host cells by transduction. A retroviral vectors may be replication competent or replication defective. And when it is replication defective, viral propagation generally occurs only in complementing host cells.

Among vectors useful in the present invention are the herein described vectors employing a pTT vector backbone (*see, e.g.,* Figures 3-7) (*Durocher* et al. 2002). Briefly, the pTT vector backbone may be prepared with the following method: (1) obtain the pIRESpuro/EGFP (pEGFP) basic vector and the pSEAP basic vector from CLONETECH® (Palo Alto, CA); (2) obtain the pcDNA3.1, pCDNA3.1/M:yc-(His)₆, and pCEP4 vectors from INVITROGEN®; (3) obtain SUPERGLO™ GFP variant ("sgGFP") from Q-BIOGENE® (Carlsbad, CA); (4) prepare a pCEP5 by the following steps: (a) remove the CMV promoter and the polyadenylation signal of pCEP4 by sequential digestion and self-ligation, using *Sal* I and *Xba*I restriction enzymes, resulting in a pCEP4Δ plasmid; (b) ligating a *Bgl* II fragment from pAdCMV5 (*Massie* et al. 1998), which encodes the CMV5-poly(A) expression cassette, into a *Bgl* II-linearized pCEP4Δ, resulting in a pCEP5 vector; (5) generate the pTT vector by deleting the hygromycin and EBNA1 expression cassettes, the deletion of the former being accomplished by *Bsm* I and *Sal* I excision and subsequent fill-in and ligation, while the deletion of the latter being accomplished by *Cla* I and *Nsi* I excision and subsequent fill-in and ligation; (6) replacing the ColEI origin, which comprises the *Fsp* I*-Sal* I fragment that includes the 3' end of β-lactamase ORF, with a *Fsp I-Sal* I fragment containing the pMBI origin and the same 3' end of β-lactamase ORF from pcDNA3.1; (7) ligating in-frame into the pcDNA3.1/Myc-His digested with *Hind* III and *Eco*R V; and (8) add a Myc-(His)₆ C-terminal fusion tag to SEAP, which is a *Hind* III-*Hpa* I fragment from pSEAP-basic. Plasmids are then amplified in *E. coli* (DH5α) grown in LB medium. They are purified from the medium using MAXI-PREP™ columns from QIAGEN™ (Mississauga, ON, Canada). The quantity of the plasmids thus made is measured by diluting the plasmids in 50 mM Tris-HCl, pH 7.4, and measuring the absorbencies at 260 nm and 280 nm. For the purpose of the invention, plasmid preparations with A₂₆₀/A₂₈₀ ratios between about 1.75 and about 2.00 are used.

The nucleic acid constructs of interest may be a DNA that is operatively linked to an appropriate promoter. The appropriate promoter may be, for example, the phage lambda PL promoter, the *E. coli* lac, trp, phoA and tac promoters, the SV40 early and late promoters, or one of the promoters from retroviral LTRs. The promoters may also be, for example, the metallothionein promoters derived from the genome of mammalian cells. Alternatively, the promoters may be the adnovirus late promoters or the vaccinia virus 7.5K promoters derived from mammalian viruses. Other suitable promoters are known to the person skilled in the art.

The expression constructs further contain sites for transcription initiation, termination and, in the transcribed region, a ribosome binding site. The coding portion of the transcripts expressed by the constructs will preferably include an appropriately-positioned translation initiating codon at the beginning and a termination codon (UAA, UGA or UAG) at the end of the polypeptide to be translated. The heterologous polypeptides the polynucleotides encode may include, for example, extracellular fragments of secretable proteins, type I membrane proteins, type II membrane proteins, multi-membrane proteins, and soluble receptors.

A construct can be introduced into a host cell by calcium phosphate transfection, DEAE-dextran-mediated transfection, cationic lipid-mediated transfection, electroporation, transduction, infection, or any other methods known to the person skilled in the art. Such methods are described in many standard laboratory manuals, such as by Davis et al., in BASIC METHODS IN MOLECULAR BIOLOGY (1986). Representative examples of appropriate hosts include, but are not limited to, bacterial cells, such as *E. coli,* Streptomyces and Salmonella typhimurium cells; fungal cells, such as yeast cells; insect cells such as Drosophila S2 and Spodoptera Sf9 cells; animal cells such as CHO, COS, 293 (including 293-6E and 293-T) and Bowes melanoma cells; and plant cells. Appropriate culture mediums and conditions for growing these representative host cells are known in the art.

A variety of host-expression vector systems may be used to express the polypeptides of the invention. Such host-expression systems are vehicles by which the coding sequences of interest may be produced and subsequently purified. These systems can also be cells that, when transformed or transfected with the appropriate nucleotide coding sequences, express the polypeptides of the invention. These systems may include, for example, microorganisms, such as bacteria like *E. coli* or *B. subtilis,* transformed with recombinant bacteriophage DNA, plasmid DNA, or cosmid DNA expression vectors that contain the polypeptide coding sequences. These systems may also include, for example, yeast such as *Saccharomyces* or *Pichia,* transformed with recombinant yeast expression vectors that contain the polypeptide coding sequences. They may also be insect cells infected with recombinant virus expression vectors such as baculovirus, which contain the polypeptide coding sequences. They may also be plant cells infected with recombinant virus expression vectors such as cauliflower mosaic viruses ("CaMV") or tobacco mosaic viruses ("TMV"), or transformed with recombinant plasmid expression vectors such as Ti plasmids, which contain the polypeptide coding sequences. They may further include mammalian cells such as COS, CHO, BHK, 293, 293-6E, PER.C6®, 293T, or 3T3, which harbor recombinant expression constructs that contain promoters.

After the host cells are transfected with the vectors or DNA constructs encoding the polypeptides of interest, the cells are then grown on proper mediums and under proper conditions to produce the polypeptides of the present invention.

Typically, a heterologous polypeptide may be expressed as a fusion protein. It may further include not only one or more of the secretion signals, but also one or more of the secretory leader sequences as exemplified in Table 1. The expression of such fusion proteins according to the invention is described in detail below.

Additionally, peptide moieties and/or purification tags may be added to the polypeptide to facilitate purification, improve stability, and engender secretion or excretion. The moieties and/or tags may be removed prior to the final steps of purification. The techniques are familiar and routine to one skilled in the art. In certain embodiments, such a tag may be a hexa-histidine peptide, such the one provided in a pQE™ vector (QIAGEN™, Inc., Chatsworth, CA). Another peptide tag, the "HA" tag that is an epitope derived from the influenza hemagglutinin protein may also be fused with the polypeptide of the present invention. (*See Wilson* et al. 1984). Other suitable purification tags may be, for example, V5, HISX8, avidin, or biotin.

In a certain embodiment, the fusion protein comprises a heterologous region from immunoglobulin, the presence of which may facilitate purification and may help to stabilize the purified protein. For example, EP-A-O 464 533 and its Canadian counterpart 2045869 describe fusion proteins comprising various parts of the immunoglobulin constant region (Fc) and a human protein or parts thereof. According to EP-A 0232 262, the Fc regions in a fusion protein is thought to be advantageous for use in therapy and diagnosis because they tend to lead to improved pharmacokinetic properties. But for some other uses, it might be desirable to delete the Fc regions after the fusion protein has been expressed, detected and purified, especially when the Fc regions hinder the use of the polypeptide to which the regions are fused in therapy and diagnosis. For example, the deletion of Fc regions might be necessary when the fusion protein is used as an antigen for immunization.

The purification tags may also be used in drug discovery. For example, a human protein hIL-5 was fused with the Fc regions to facilitate the identification of hIL-5 antagonists using high-throughput screening assays. (*Bennett* et al. 1995; *Johanson* et al. 1995).

A heterologous polypeptide of the invention can be purified from a recombinant cell culture by well-known methods, which include, for example, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. In a particular embodiment, high performance liquid chromatography ("HPLC") is employed for purification. Polypeptides of the present invention may include, for example, products purified from directly-isolated or cultured natural sources such as bodily fluids, tissues and cells; products of chemical synthetic procedures; products produced by recombinant techniques from prokaryotic or eukaryotic hosts such as bacterial cells, yeast, higher plant cells, insect cells, mammalian cells; or products produced by recombinant techniques from cell-free expression systems.

### Modifications

The invention encompasses polypeptides that are differentially modified during or after translation, for example, by glycosylation, acetylation, methylation, phosphorylation, amidation, derivatization by known protecting/blocking groups, proteolytic cleavage, or linkage to an antibody molecule or other cellular ligand. Any of these chemical modifications may be carried out by known techniques, including specific chemical cleavage by cyanogen bromide; digestion by trypsin, chymotrypsin, papain, or V8 protease; treatment by NABH₄; acetylation; formylation; oxidation; reduction; and metabolic synthesis in the presence of tunicamycin.

Depending upon the hosts employed in the recombinant production procedures, the polypeptides of the present invention may be glycosylated or non-glycosylated. A polypeptide of the invention may also include an initial modified methionine residue at the N-terminus, usually as the result of host-mediated processes. It is known in the art that the N-terminal methionine encoded by the translation initiation codon can generally be removed with high efficiency after translation in eukaryotic cells. While the N-terminal methionines can be efficiently removed from most prokaryotic proteins, the removal processes are not always efficient in prokaryotes. The efficiency depends on the nature and identity of the amino acids to which the N-terminal methionines are covalently linked.

Additional post-translational modifications according to by the invention include, for example, N-linked or O-linked carbohydrate chains, processing of N-terminal or C-terminal ends, attaching the chemical moieties to the amino acid backbone, chemical modifications of N-linked or O-linked carbohydrate chains, or addition or deletion of an N-terminal methionine as the result of prokaryotic host cell expression. To facilitate detection and isolation of the protein, the polypeptide may also be modified with one or more detectable labels, which may be, for example, an enzymatic, fluorescent, isotopic, or affinity label.

Additional embodiments of the invention may be chemically modified derivatives of the polypeptides of the invention, which may provide additional advantages such as increased solubility, stability and circulating time for the polypeptides, or decreased immunogenicity in biological systems (U.S. Pat. No. 4,179,337). The chemical moieties used in derivitization may be selected from water soluble polymers such as polyethylene glycol, ethylene glycol/propylene glycol copolymers, carboxymethylcellulose, dextran, polyvinyl alcohol and the like. The polypeptides may be modified at random positions, or at predetermined positions within the molecule, and may include one, two, three, or more attached chemical moieties. Reaction conditions may be selected from any of those known in the art and those subsequently developed, but should be selected so that the protein to be modified is not exposed or will suffer only limited loss of activity due to harsh temperature, solvent, and pH conditions. In general, the larger the ratio of polymer to polypeptide conjugate, the greater the percentage of conjugated product. The optimum ratio, measured by the efficiency of the reaction, may be determined by factors such as the desired degree of derivatization (*e.g.*, mono-, di-, tri- etc.), the molecular weight of the polymer selected, the degree of branching, and the reaction conditions. The ratio of polymer to polypeptide generally ranges from 1:1 to 100:1. One or more purified conjugates may be prepared from each mixture by standard purification techniques, which includes, for example, dialysis, salting-out, ultrafiltration, ion-exchange chromatography, gel filtration chromatography, and electrophoresis.

A polymer may be of any molecular weight, and may be branched or unbranched. In certain embodiments, where the polypeptides of the invention are modified by polyethylene glycol, the molecular weight of the polyethylene glycol is from about 1 kDa to about 100 kDa. The term "about," when used in the description of polyethylene glycol, is intended to suggest that, during the preparations of polyethylene glycol, some molecules will weigh more, some less, than the stated molecular weight. The size of the polyethylene glycol used in the modification may depend on the desired therapeutic profile, such as, for example, the desired duration of sustained release; the effects, if any, on biological activities; the ease of handling; the degree or the lack of antigenicity; as well as other known effects of the polyethylene glycol on a therapeutic protein or an analog.

There are a number of attachment methods available to those skilled in the art. For example, EP 0 401 384 describes the coupling of PEG to G-CSF. *Malik* et al. reported pegylation of GM-CSF using tresyl chloride (*Malik* et al. 1992). Polyethylene glycol may be covalently bound to a reactive group of an amino acid residue, which may be, for example, a free amino group, a carboxyl group, or a sulfhydryl group. In the context of pegylation, which means attaching polyethylene glycol moieties to a molecule, reactive groups are defined as groups to which an activated polyethylene glycol molecule may be bound.

One may specifically desire proteins chemically modified at the N-terminus. This may be accomplished by reductive alkylation, which exploits the different reactivities of primary amino groups such as the internal lysines and the N-terminal amino acid, which are available for derivatization. For example, one may selectively attach a polymer to the N-terminus of a protein by performing the reaction at a pH where only the α-amino group of an N-terminal residue, and not the ε-amino residue, would be susceptible to the reaction, taking advantage of the pKa differences between these types of amino groups. The polymer used in reductive alkylation typically have a single reactive aldehyde. The N-terminally chemically modified protein may be separated from other monoderivatized moieties, if necessary, by purifying the N-terminally modified protein from a population of protein molecules that are modified elsewhere.

### Fusion Molecules of the Invention

In a further embodiment of the invention, the heterologous polypeptides of may be combined with one or more fusion partners to form fusion molecules. Such fusion molecules may advantageously provide improved pharmacokinetic properties when compared to their unmodified non-fused counterparts. These fusion molecules comprising the heterologous polypeptides of the invention may be prepared by a person skilled in the art who is apprised with the disclosures herein. Suitable chemical moieties for derivatization of a heterologous polypeptide in this regard may be, for example, polymers such as water soluble polymers; all or part of human serum albumin; fetuin A; fetuin B; or Fc regions.

Specifically, a modified heterologous polypeptide of the invention may be prepared by attaching one or more polyaminoacids, peptide moieties, or branch-point amino acids to the polypeptide. Polyaminoacids are commercially available and widely used in drug delivery technology and other emerging technologies such as gene therapy. In addition to the advantages one may achieve with a fusion molecule as described above, the polyaminoacid may be a carrier that serves to increase the polypeptide's circulation half-life. For the therapeutic purpose of the present invention, such a polyaminoacid should ideally be one that does not generate neutralizing antigenic or other adverse responses. As described herein, the position at which the polyaminoacid is attached to the polypeptide or fusion polypeptide may be located at the N-terminus, C-terminus, or any other positions in between. The polyaminoacid may also be connected by a chemical "linker" moiety to either end of the selected polypeptide or fusion polypeptide.

A method for preparing a fusion molecule conjugated with one or more polymers, such as water-soluble polymers is described above.

Additionally, heterologous polypeptides of the present invention and the epitope-bearing fragments thereof can be combined with parts of the immunoglobulin constant domain, resulting in chimeric polypeptides. These particular fusion molecules facilitate purification and tend to show an increased half-life *in vivo* when compared to their pre-fusion counterparts. Examples of these chimeric polypeptides include, for example, the chimeric proteins comprising the first two domains of the human CD4-polypeptide and various domains of the mammalian immunoglobulin constant regions (EP A 394,827; *Traunecker* et al. 1988). A fusion molecule having a disulfide-linked dimeric structure tends to be more efficient in binding and neutralizing other molecules than, for example, a monomeric polypeptide or fragment (*Fountoulalkis* et al. 1995).

In another embodiment, a human serum albumin fusion molecule may also be prepared as described herein and as further described in U.S. Patent No. 6,686,179, which is hereby incorporated by reference in its entirety.

Moreover, the polypeptides of the present invention can also be fused to a purification tag, which is a peptide region that would facilitate the purification of the polypeptides to which they are a part. The method of fusing the tag to the polypeptide of interest is described herein.

It will be clear to those skilled in the art that the invention may be practiced in ways other than those particularly described in the foregoing descriptions and the examples herein. Many modifications and variations of the present invention are possible in light of the teachings herein and, therefore, are within the scope of the appended claims.

### Examples

### Example 1: Expression of Biologically Active Mature Secreted Proteins using a Cell-free System.

Recombinant technologies allow for expression of proteins *in vitro* or *in vivo*. Examples of *in vitro* systems for protein expression include cell-free systems such as rabbit reticulocyte lysates and wheat germ extracts, and cell-based systems such as bacteria, insect cells, yeast cells and mammalian cells (for example, CHO cells, 293 cells, and human embryonic retinal cells PER.C6^{®} cells (Crucell, Netherlands)). *In vivo* expression of recombinant proteins is useful, for example, in the generation of transgenic animals in which the transgene(s) encodes protein(s) tagged with markers such as, for example, Green Fluorescent Proteins and its variants or β-galactosidase. Such tags allow for easier visualization, tracking and/or isolation of the cells in which the tagged protein is expressed. Another example of *in vivo* expression of recombinant proteins is the use of transgenic mice, or of cells implanted into mice, that have been genetically modified for the expression of secretable proteins. The latter can be proteins that, for example, are thought to promote tumor development, work as hormones, as growth factors, and/or as survival factors. In that setting, it can be important to obtain various levels of protein secretion (low, moderate, high) in order to obtain a specific result (*e.g.* tumor promotion). Many proteins are not efficiently secreted when expressed in recombinant settings. In that case, it is useful to be able to replace, via recombinant methods, its endogenous leader sequence with a leader sequence that is capable of driving its efficient secretion.

It is often useful to confirm that a given isolated cDNA is capable of supporting the expression of the protein which its nucleotide sequence encodes *in vitro,* before the cDNA is used to express that protein *in vivo.* This process may also serve, for example, to obtain further information regarding the post-translational modifications that the protein undergoes in a specific host cell (*e.g.* CHO cells versus PER.C6^{®} cells), and the activity of the protein. In the case of a secretable protein, the cDNA sequence may either encode its full-length form, its mature form (i.e., the protein without the leader sequence), or any other parts of the protein, such as a particular domain.

### Preparation of Plasmid Templates for Recombinant Protein Expression in Cell-free Systems.

To recombinantly express a cDNA encoding the mature form of any protein of interest, it is often useful that the cDNA be modified in order to include, in addition to the coding sequence, a translational initiation site/translational enhancer (*e.g.* KOZAK sequence, Omega sequence, Non-Omega sequences). In this example, the mature form refers to the most typical product of secretion, which is the protein without the signal peptide. Furthermore, if no antibody exists for the protein of interest, a tag may also be added which facilitates both the detection and the purification processes. Examples of such tags are Glutathione-S-Transferase (GST), and the epitopes V5, HisX6, and HisX8(H8). The addition of these features to a cDNA encoding a protein of interest can be done by a variety of cloning methods. If no appropriate restriction enzyme sites are present in the cDNA of interest. PCR amplification methods such as those described below can also be used during the cloning process. A cloning process that involves three PCR steps and results in a mature ORF tagged with Glutathione-S-Transferase is exemplified below.

To begin, a first plasmid containing the cDNA sequence encoding the mature open reading frame (mature ORF) of interest was provided for the first PCR. To add the translational initiation site/translational enhancer to the 5' region of the coding sequence for the mature ORF, a nucleotide primer (forward primer FP1) was designed and synthesized, which contained 5'GTTCTGTTCCAGGGGCCC 3' followed by the first nineteen nucleotides predicted to encode the amino terminus of the mature secretable protein of interest. A second primer (reverse primer RP1) was designed and synthesized, based on a region of the plasmid approximately 1000 nucleotides downstream from the coding sequence (mature ORF) of the cDNA to be expressed. In fact, the RP1 primer was designed as the reverse complement of the vector sequence in this region such that RP1 could be used with FP1 in a PCR to amplify the mature ORF. The exact sequence of RP1 would vary depending on the starting plasmid, but it was typically 17-23 nucleotides long with a Tm of approximately 55-65°C.

The purified starting plasmid containing the cDNA to be expressed as a mature ORF, or *E coli* cells containing the purified plasmid, was then added as a template to a standard PCR, which included the two primers (FP1 and RP1), as described above, standard PCR reagents, and a DNA polymerase. The reaction mixture was then subjected to 15-30 cycles of PCR amplification. The product of this first PCR is called the "PCR1 coding templates" for the purpose of this application.

A separate PCR was performed to prepare a "GST-Mega primer," whose purpose was to provide the GST portion of the final GST-mature ORF expression template in the second PCR step. To this end, a different starting plasmid template was used, for example, one containing a GST coding sequence downstream from the Non-Omega translation initiation sequence, and which is herein referred to as "template 2." It is often useful that the GST fusion protein is linked to the mature ORF via a cleavable bridge. To this end, the template might have a GST protein modified to include a protease-cleavable sequence, such as one sensitive to thrombin, or to the commercially available PreScission™ Protease (Amersham, NJ). This allows for the two proteins, mature ORF and GST, to be separated at the end of the purification procedure by protease-mediated cleavage. Thus, a PCR was prepared to amplify "template 2" using two primers: FP2, of sequence 5' GGTGACACTATAGAACTCACCTATCTCCCCAACA 3'; and RP2, of sequence 5' GGGCCCCTGGAACAGAACTTC 3'. The amplification took place for 15 to 30 cycles in a standard PCR mixture that included template 2, the two primers described above (FP2 and RP2), standard PCR reagents, and a DNA polymerase. After the PCR was complete, the amplification product was treated with exonuclease I for 30 minutes at 37°C, and then heat-inactivated at 80°C for 30 minutes. The product was then purified by agarose gel electrophoresis and extracted using a gel purification kit (Amersham, NJ), producing the "GST-Mega primer." The "GST-Mega primer" was, in fact, one of the two templates used in the second PCR that yields a GST-fusion expression template. The other template of the final reaction was the "PCR1 coding template," prepared as described above.

The final construct, which was the mature ORF/GST fusion expression template, was prepared as follows. The two templates "GST-Mega Primer" and "PCR1 coding template" were combined via the second PCR involving the mature ORF. This PCR reaction mix included: (i) standard PCR reagents; (ii) a DNA polymerase; (iii) an aliquot of the "PCR1 coding template" (*e.g.,* 0.5 µl); (iv) an aliquot of the "GST-Mega primer" (e.g., 1 µl); (iv) a fifth primer, FP3, of sequence 5' GCGTAGCATTTAGGTGACACT 3', which comprised part of the SP6 promoter sequence, and was annealed to the 5' end of the "GST Mega primer" via its common 3' end (compare underlined sequences); and (v) a sixth primer, RP3, which was designed as the reverse complement of the vector sequence in the same region of the vector as RP1 but starting three nucleotides upstream of RP1 to specifically anneal only on the full-length PCR1 coding template; RP3 is typically 17-23 nucleotides long with a Tm of approximately 55-65°C, and can be used in amplifying the "PCR1 coding template." After 15-30 cycles of PCR amplification, the "Mature ORF/GST-fusion expression template" was thus generated.

### Expression of GST-fusion Expression Templates in Wheat Germ Extracts

In order to express a mature protein of interest in a cell-free system, the mRNA can be both transcribed and translated from the "Mature ORF/GST-fusion expression template" in the same reaction, or in separate reactions. A separate *in vitro* transcription reaction (50 µl) can be prepared with 5 µl of the "GST-fusion expression template" in the following buffer: 80 mM HEPES KOH pH 7.8, 16 mM Mg(OAc)₂, 2 mM spermidine, 10 mM DTT, 1 unit of SP6 (Promega, WI) and 1 unit of RNasin (Promega, WI). The reaction mixture is incubated for 3 hours at 37°C. The resulting mRNA is subjected to ethanol precipitation in a solution containing 200 µl of RNase-free water, 37.5 µl of 5 M ammonium acetate, and 862 µl of 99% ethanol. The ethanol precipitation comprises the steps of mixing by vortexing and pelleting by centrifugation at 15,000 x g for 10 minutes at 4°C. The mRNA pellet is then washed in 70% ethanol and again pelleted by centrifugation at 15,000 x g for 5 minutes at 4°C, after which steps the pelleted mRNA is ready for *in vitro* translation.

Wheat germ extracts can be used for *in vitro* translation of the mRNA, prepared separately as described above. First, a stock solution of 2x Dialysis Buffer was prepared from mixing two separate stocks of amino acids. The first stock contained 20 mM HEPES KOH buffer pH 7.8, 200 mM KOAc, 5.4 mM Mg(OAc)₂, 0.8 mM Spermidine, 100 µM DTT, 2.4 mM ATP, 0.5 mM GTP, 32 mM creatine phosphate, 0.02 % NaN₃, and 0.6 mM of an amino acid mix that did not contain aspartic acid, tryptophan, glutamic acid, isoleucine, leucine, phenyalanine and tyrosine. The second stock contained a 80 mM mix of the amino acids aspartic acid, tryptophan, glutamic acid, isoleucine, phenylalanine and tyrosine in 1 N HCl. After all the amino acids in the second stock were dissolved, the two stocks were mixed, so that the final concentration of the second-stock of amino acids was 0.6 mM. The 2x Dialysis Buffer stock was then adjusted to pH 7.6 using 5 N KOH, filter sterilized, and stored frozen in aliquots at -80°C.

To resuspend the *in vitro* transcribed mRNA (prepared separately as described above), a 50 µl "translation mixture" was prepared that includes Wheat Germ Reagent (Promega, WI) at a final OD₂₆₀ₙₘ of 60 prepared in 1 x Dialysis buffer containing 2 mM dithiothreitol (DTT). After removing the supernatant (ethanol) from the precipitated mRNA, the 50 µl "translation mixture" was added to the precipitate and allowed to sit for 5-10 minutes before the mRNA was resuspended into the translation mixture. The complete translation mixture containing the resuspended mRNA was then layered under 250 µl of 1x Dialysis Buffer that had already been added to one well of 96 well round bottom microtiter plate to setup a Bilayer Reaction. The plate was then sealed manually with a plate seal and the *in vitro* translation reaction allowed to incubate for 20 hours at 26°C.

At the end of the *in vitro* translation reaction period, and to recover the recombinant mature ORF protein expressed as a GST fusion, the translation mixture was transferred to a tube and diluted five-fold with phosphate buffer-saline containing 0.25 M sucrose and 2 mM DTT. Ten microliters of glutathione(GSH)-sepharose beads (Amersham-Pharmacia Biotech, NJ), to which the Glutathione-S-Transferase (GST) protein binds, were then added to the mixture, which was then incubated at 4°C for 3 hours, with constant agitation to ensure mixing. The GSH-sepharose beads, containing the bound GST-fusion protein, were then washed three times in PBS containing 0.25 M sucrose and 2 mM DTT. If the mature ORF and the GST were recombinantly engineered to be fused via a protease cleavable bridge, a fourth wash was then performed in a protease-cleavage buffer containing 50 mM Tris pH 7.4, 150 mM NaCl, 1 mM EDTA, 2 mM DTT, and 0.25 M sucrose. This wash buffer was also called the "final wash buffer." After the wash buffer was carefully removed from the bead mixture, 10 µl of the final wash buffer collected from the last step was mixed with the beads, and 0.4 µl of the appropriate protease such as PreScission™ Protease (Amersham, NJ) was added to the mixture. A pipette was then used to gently suspend the beads. This bead mixture/suspension was then allowed to sit overnight at 4°C. To recover the cleaved mature ORF protein product, 20 µl of the final wash buffer was added and entire liquid fraction (without the beads) recovered by pipetting (after allowing the beads to settle), or by filtering through a sintered frit.

Aliquots of the recovered liquid fraction (containing the purified mature protein) were analysed by ELISA and/or Coomassie/Silver Staining of SDS-PAGE gels, in order to quantify the level of expression of the mature protein.

To stabilize the recovered mature ORF protein, a solution of 10 mg/ml purified BSA in PBS was added to the purified protein solution so that the final concentration of BSA became about 1 mg/ml. The protein sample was then dialyzed in PBS and filter-sterilized for storage. Western blot analysis can be done from aliquots recovered throughout various steps along the purification procedure to assess, for example, the level of protein expression, and to determine whether or not the protein translated corresponds to the protein expected to be encoded by the cDNA of interest, both in terms of its length and its sequence. The protein can also be used in future characterization studies, such as biological activity measurements, mass-spectrometry, and post-translational modification assays. To produce additional protein from the same mRNA template, the single Bilayer Reaction can be repeated multiple times, and the purification and formulation can be scaled accordingly.

Typically, sixteen Bilayer reactions (set up as described above) will produce sufficient biologically active protein for testing in most typical assays such as biological activity assays. Since these reactions are done in 96 well plates, this expression system is suitable for high-throughput assays in which multiple cDNAs of interested can be translated simultaneously in separate wells. Once a cDNA is shown capable to encode a specific protein of interest in wheat germ extracts, it can be desirable to express larger amounts of protein than those typically obtainable with this expression system. It can also be desirable to compare the post-translational modifications that a given protein undergoes in different cell systems, for example those that occur in a plant-based system such as the wheat germ lysates, with those that occur in a mammalian system (*e.g*. CHO cells, 293 cells, PER.C6^{®} cells).

### Evaluation of the Expression Levels of Various Signal-Peptide-Less Mature Proteins

Column 3 ("Highest Expressors") of Table 3 summarizes the results of a high-throughput expression experiment aimed at comparing the expression levels of various proteins of the invention, without their endogenous signal peptide and under standardized conditions. Starting with a set of cDNAs that included those encoding the full-length proteins listed in Table 1 and Appendix A, mature ORF templates were prepared as described in detail in the previous paragraphs, to express the mature version of each protein (i.e., the protein without its endogenous signal peptide). After purification, the expression levels were quantified by Coomassie-stained SDS-PAGE, silver stained SDS-PAGE, or quantitative Western Blot using an Anti-V5 antibody relative to purified V5-tagged protein standards, and 56 of the "highest expressors" were ranked from 1 (high) to 56 (low) based on their expression levels, relatively to each other. Under these standardized conditions, among the "highest expressors" of column 3/Table 3, the very highest expressor (ranked 1) was the mature version of the beta-subunit of prolyl 4-hydroxylase, (CLN00517790); a moderate expressor (ranked 20) was the mature version of the long form of alpha I collagen type IX (CLN 00517648); and the lowest expressor (ranked 56) was WFIKKN-related protein (CLN 00463474).

### Example 2. Identification of Leader Sequence-Containing Proteins that are Secreted at High Levels from Mammalian Cells.

The next set of assays aimed at comparing proteins on the basis of the amounts that could be recovered from the conditioned media (i.e., on the basis of "secretion"). The cDNAs used for Example 1, table 1 and table 2, were subcloned into modified versions of the pTT mammalian expression vector, and the proteins were expressed with their endogenous signal peptides/leader sequences, in mammalian cells. After quantifying the levels of the resulting protein present in the conditioned media, proteins were ranked again, this time from "high secretors" to "moderate secretors" to "low secretors."

Later on, this information served as the baseline to assess whether one could improve secretion of a protein by re-engineering its signal peptide/leader sequence. This "re-engineering" was done by replacing the endogenous signal peptide of a "low secretor" protein, with that of a "moderate" or "high secretor."

In order to proceed with the above re-engineering, the amino acid sequence corresponding to the signal peptide/leader sequence of each of the proteins of the invention had first to be identified (Appendix A, Table 1 and 2). Based on a defined set of attributes, cDNAs from an existing library can be predicted to encode secretable proteins bioinformatically. For example, a signal peptide is typically encoded by the first 6-27 amino acid codons (18-81 nucleotides) of the ORF, and it usually begins with 1-4 polar amino acids, followed by a stretch of hydrophobic amino acids, and then followed by a short region of charged amino acids just before the site where the secretion-related cleavage takes place. Using these attributes, together with other physical characteristics, cDNAs can be predicted to encode secretable proteins while the identities of the proteins may remain unknown. The results of one of such analysis done on our complete cDNA library are summarized on Appendix A, and Tables 1 and 2. A current limitation still is that one can not predict whether or not the presence of a putative signal peptide/leader sequence allows a protein containing said leader sequence to be secreted *in vitro* or *in vivo*, and what the efficiency of this process will be.

### Preparation of the Expression Vectors for High-Throughput Screening of Leader Sequences

In order to identify signal peptides or secretory leader sequences that yield high secretion levels in proteins, a set of cDNAs predicted to encode secretable proteins (using a cDNA library existing in house and the methods described above) were subcloned into one of several modified version of the pTT5 expression vector (FIG. 3) using subcloning techniques similar to those described in detail in Example 1. Some of the modified vectors contained cleavable tags (Vectors A and C, FIGS. 4 and 5) in frame with a C-terminal V5 and HisX8 epitope tag (Vector A, B and C, FIGS 4 and 5), or in frame with an Fc domain sequence (Vector D and E, FIG. 6). The presence of a HisX8 tag (which consists of a group of eight His residues), allows for purification of the recombinantly-expressed proteins using standard Nickel column-based technologies familiar to those skilled in the art, and commercially available (*e.g*. Qiagen Inc., CA). When long-term selection for stable transfectants was necessary, the proteins were also expressed in vectors such as the pTT2p vector shown in FIG. 7.

The plasmid DNAs for each cDNA clone inserted into pTT5 were purified using the QIAGEN™ TURBO™ DNA system in 96-well plates. The DNA concentration for each clone was determined by absorbance at 260 nm, and subsequently adjusted, for example, to a concentration of 50 µg/ml in a suitable buffer. The expression/secretion assays were done after the resulting pTT5-based constructs were transiently transfected into 293T cells (ATCC^{®}, VA) using a high-throughput 96-well system. These steps are described next.

### High-throughput Transfection in 96-well Plates

For transient transfection of ten 96-well plates, 10 µl of each cDNA plasmid were combined with 50 µl of GIBCO® OPTI-MEM I™ (GIBCO, Gaithersburg, MD, Cat#:319-85-070) in separate wells (one for each cDNA) of a round-bottom 96-well polystyrene plate. This plate was named the "master transfection plate." Then, 37.5 µl of each OPTI-MEM I™/cDNA mix were pre-incubated for 5 minutes with 2.5 µl of FUGENE™ 6 transfection reagent (Roche Applied Science, Palo Alto, CA, cat#1988387) in separate wells (one for each cDNA) of another round-bottom 96-well polystyrene plate. The mixture was then incubated at room temperature for about 30 minutes, resulting in one "transfection complex" per cDNA.

Each transfection complex was subsequently diluted by the addition of 100 µl of OPTI-MEM I™, mixed several times by repeated pipetting, and then transferred 20 µl at a time into ten separate wells. Each well was on a separate 96 well flat bottom poly-lysine-coated plate (Becton Dickinson, Rockville, MD, cat# 356461) to facilitate collection of samples for up to 10 different assays after transfection. Each plate contained up to 96 different cDNAs.

Two hundred microliters of a suspension of 2 x 10⁵ cell/ml of 293T cells in DMEM medium (containing 10% FBS, penicillin and streptomycin) were then added to each well. The different mixtures of cells and diluted transfection complex were allowed to incubate at 37°C in 5 % Coy. After approximately 40 hours, the medium was removed from the wells by aspiration, the cells were briefly washed with 150 µl PBS, and new prewarmed medium was added.

To prepare the set of transfected cells used for the purpose of assaying the expression and secretion levels of each protein, 150 µl of fresh HYQ-PF™ CHO Liquid Soy medium (Hyclone, Logan, UT, Cat# SH30359.02) were added to each well,

To prepare the set of transfected cells used for the purpose of assaying the activity of the secreted protein, 150 µl fresh DMEM medium containing 5% FBS, penicillin and streptomycin were added to the wells instead of the HYQ-PF™ CHO Liquid Soy, and the resulting mixtures were incubated at 37°C in 5 % CO₂.

After an additional 48 hours, during which the various cDNA expressed their respective secretable proteins, the culture supernatants from all ten 96-well plates were harvested and, when appropriate, combined into a single sterile deep-well plate, covered with a sterile lid. The deep-well plates were centrifuged at 1,400 RPM for 10 minutes to pellet any loose cells or cell debris. The supernatants were then transferred to new sterile deep-well plates so that the level of protein released into the conditioned media (i.e. secreted protein) could be measured. This was achieved by Western blot using anti-V5-HRP antibody and sandwich ELISA using the anti-penta-HIS antibody as a capture step and anti-V5-HRP to detect expression and measure expression levels relative to purified V5His standard. The layer of cells, which remained attached to the plates, was solubilized with 0.2% SDS, 0.5% NP-40 in PBS; the resulting cell lysates were used to assay the levels of protein in the cell lysates by ELISA.

In the first set of screening assays, a subset of leader sequences were identified that were shown to correlate with high secretion levels of the proteins they belonged to. The results of a high-throughput secretion assay, done following the steps just described are shown in Figures 8 and 9. Using high-throughput expression of cDNAs in the 293T cells, several cDNAs were identified that lead to high secretion levels. A total of 56 cDNA (previously ranked as the highest expressors among our complete library of cDNAs for secretable proteins) were screened in this assay. Their identity, respective position in each lane, and relative expression level in the conditioned media, are all summarized in Table 3, columns 2 and 4. Column 2 quantified the concentration of each protein that was secreted into the conditioned media, relatively to the concentration of one or more standards that were separated in adjacent lanes (BSA for FIGS. 8 and 9). Typically, secretion levels do correlate to expression levels (compare colum 2 to column 2), but not always. For example, the "highest expressor" (ranked 1 on column 3) is also the "highest secretor" (secreted protein concentration of 32 µg/mL) according to Table 3. These results correspond to the full length protein beta subunitprolyl 4-hydroxylase (column 11). On the other hand, several proteins were secreted at the level of about 4 µg/mL (column 2), but had ranked between 16 and 21 on expression (column 3). The long form of alpha I collagen type IX (encoded by CLN00517648) is among the latter.

### Example 3. A Set of Leader Sequences from High-Secretors is Useful for Converting Low-Secretors into High-Secretors.

The high-throughput assay described in detail in Example 2 provided a panel of cDNAs from the "highest expressor" proteins with levels of secretion which varied from "low secretor proteins" to "high secretor proteins." For a summary of their identity and properties, see Tables 1, 2 and 3. The next question was whether the signal peptide/leader sequences of the high-secretors were transferable into other proteins. More importantly, we asked whether the secretion of "low secretor proteins" could be improved by replacing their endogenous leader sequence with one taken from any one of "high secretor proteins" of the invention. To this end, a series of experiments were conducted, using standard subcloning techniques, transfection and expression methods essentially as described in detail in Examples 1 and 2. One of these experiments is exemplified next.

The signal peptide/leader sequence from CLN00517648 was used to replace the signal peptide/leader sequence of a panel of proteins, which in the initial sets of high-throughput expression and high-throughput secretion assays had been shown to be low expressing proteins, low secretor proteins, or both. The proteins encoded by the resulting re-engineered cDNAs, which carried the heterologous leader sequence of the high secretor clone CLN00517648 instead of their own endogenous leader sequence, were found to have become high secretor proteins from what otherwise had been low expressor/low secretor proteins. Indeed, the signal peptide/leader sequence of CLN00517648 is capable of enhancing the secretion of type I TM proteins and type II proteins. Some specific examples of proteins whose secretion was improved by this process include cDNA constructs encoding the following proteins: human CD30 Ligand, SCDFR1, Ox40 Ligand, all of which were engineered to replace their endogenous signal peptide/leader sequence with that of CLN00517648 according to the process described in Examples 1 and 2. Moreover, the total level of expression of the modified proteins was also increased by this substitution. This was determined both by quantified the total levels of protein in both cell lysates and conditioned media. Thus, the signal peptide/leader sequence from CLN00517648 can enhance both the expression and the secretion of low expressor proteins.

The high-throughput results described above, showing improvements in secretion and/or expression levels of low secretors and/or low expressors by replacing their endogenous leader sequence with that of either CLN00517648 or of another protein (heterologous leader sequence) selected from the list of "highest expressors" (see Table 3, column 3), were further confirmed using the scale-up procedures described in Example 4.

### Example 4. Scale-Up Process for Expression of Leader Sequence-Containing Proteins in 293-6E Cells

An alternative to the 96-well high-throughput transfection-expression assay is one in which both the transfection and the expression are done in larger scale protocols. These can use, for example, 293-6E cells provided by Y. Durocher grown in shaker flasks rather than 96-well plates. For the high-throughput process, the 293-6E cells can be treated with the same reagents and subject to the same conditions as the ones used for the 293T cells except that PEI is used for DNA transfection in shake flasks instead of Fugene 6.

For the scale-up process, the 293-6E cells were grown in polycarbonate Erlenmeyer flasks fitted with a vented screw cap and rotated on a table top shaker at 100 RPM in FREESTYLE™ Medium (INVITROGEN®, Carlsbad, CA) at 37°C in 5% CO₂. The cell densities in those flasks were maintained in a range from 0.5 to 3 x 10⁶ cells/ml. Typically 50 ml cultures were grown in 250 ml flasks. One day before transfection, 293-6E cells were diluted into fresh FREESTYLE™ Medium to a cell density of about 0.6 x 10⁶ cells/ml. On the day of transfection, the cells were predicted to be in the log phase, which is characterized by a cell density range of 0.8 to 1.5 x 10⁶ cells/ml. The volumes of the log-phase cell cultures were adjusted so that their cell densities were about 10⁶ cells/ml.

For each cDNA, a different transfection mix was prepared. To prepare each transfection mix, 2.5 ml sterile PBS were added to two 15-ml tubes. The first tube also contained 50 µg DNA. The second tube also contained 100 µl PEI solution, which includes 1 mg/ml sterile stock solution of linear 25 kDa Polyethylenimine, pH 7.0 (from Polysciences, Warrington, WI). The solutions in the two tubes were then combined and allowed to incubate together for 15 minutes at room temperature, yielding the transfection complex. The transfection complex was then transferred to a 293-6E suspension culture and allowed to grow for 4 -6 days at 37°C in 5%CO₂; this process was repeated for each cDNA.

To determined protein secretion levels, culture supernatants were analyzed by Western blot. Samples (15 µl per cDNA) were resolved by SDS-PAGE on 26-lane CRITERION™ gels (Bio-Rad, Inc., Hercules, CA) and transferred to nitrocellulose membranes. The membranes were blocked, and probed with an antibody against the specific epitope introduced at the cloning step. For example, for proteins tagged with a V5 and/or a HisX8 epitope, either an anti-VS or an anti-HisX8 epitope antibody, conjugated to HRP (INVITROGEN®, Carlsbad, CA), was used. The HRP signal was developed using standard HRP chemiluminescence substrates (ECL Detection Kit, Amersham).

Secretion levels were determined by comparing the intensity of signal obtained for each secreted protein to that of one of three purified mass standards (for example, 15 µl of standards at 8, 33, and 133 ng/ml) that were loaded into separate lanes of the same gels. The comparison involved determining the area of the bands present on either the Coomassie-stained gel, the silver-stained gel, or the Western blot; this process was done with a image scanner and NIH *Image* freeware, which can be downloaded from Scion Corporation website. Various protein standards were used. Examples include a V5-HisX6-tagged Delta-like protein 1 extracellular protein, a V5-HisX6-tagged CSF-1 Receptor extracellular domain, and/or a POSITOPE™ (INVITROGEN®, Carlsbad, CA, cat#: R900-50) containing a V5-HisX6 tag. These standards can be expressed separately using, for example, a baculovirus expression system, and purified to >90% purity.

Figure 2 exemplifies the results of a large-scale expression experiment in which the cDNAs (including the V5H8 epitopes) of twenty clones were subcloned into a pTT5 vector (FIG. 5). The resulting clones were transfected into 293T cells, using the methods herein described. The levels of secreted protein in 15-µl samples of conditioned media were assessed by a Western Blot. Two V5 His standards were mixed in each standards and loaded into the right-hand lanes according to the following concentrations: (1) the higher molecular weight, V5-Hisx5 tagged Delta-like protein 1 extracellular protein, loaded at 16, 66 and 266 ng/ml; and (2) the lower molecular weight, V5-Hisx6 tagged CSF-1 extracellular domain, loaded at 8,33, and 133 ng/ml. An anti-V5 antibody (Invitrogen, CA) was used for the Western Blot. From this Western Blot experiment, the clone expressing a protein encoded by CLN00717648 produced the highest level of secreted protein in the conditioned media. These results were confirmed by large-scale expression in 293-6E cells.

### Example 5. Classification of the Signal Peptides/Leader Sequences of the Invention on the Basis of their Ability to Enhance Secretion and/or Expression of Heterologous Proteins

The combined results from the experiments described in Examples 1-4, suggest a classification of the leader sequences of the invention according to their ability to, in their role as heterologous leader sequences, improve secretion and/or expression of the proteins they are inserted into. The leader sequences are accordingly classified under categories such as "high secretor signal peptide/secretory leaders," "moderate secretory signal peptide/secretory leaders," or "low secretory signal peptide/secretory leader sequences."

Because the secretion levels and the increases in secretion caused by the heterologous polypeptide of the invention is separate and distinct from the expression levels of the resulting polypeptides, the resulting polypeptides were also ranked on the basis of their expression levels on a relative scale that served to rank all the proteins of the invention (Tables 1-3 and Appendix A) relatively to each other. These rankings were made for expression and secretion levels in either wheat germ extracts, or mammalian cells (*see* Examples 1-3).

Moreover, whereas the above classification is based on the results obtained from using *in vitro* assays, the classification extends to results that can be obtained while expressing the proteins of the invention *in vivo.* As already discussed in Example 1, the signal peptides/leaders sequences of the invention can be assayed for their ability to be used to improve the *in vivo* expression of heterologous proteins they are attached to. For example, any of the leader sequences described in Table 2 can be operatively linked to an heterologous protein using cloning methods essentially as described in Examples 1 and 2. The resulting cDNA construct can then be electroporated or microinjected into embryonic stem (ES) cells (for example, mouse or pig ES cells), which are then used, according to standard methods known to those skilled in the art, for generating transgenic animals (*e.g*. mice or pigs). Depending on the protein, and on other properties of the cDNA construct (for example, the specific promoter used to drive expression of the recombinant protein), the secreted recombinant protein can be assayed from bodily fluids such as, for example, blood, milk, saliva, and its expression levels quantified. The assay can be done such that two recombinant proteins are expressed that vary only by their signal peptide (i.e., comparing endogenous signal peptide and heterologous signal peptide of the invention).

It is possible that the signal peptide/leader sequences of the invention do not fall into the same categories when, instead of being used for protein expression *in vitro* they are used for protein expression *in vivo.* However, the results from the *in vitro* assays described herein should serve as guidelines for choosing which particular signal peptide/leader sequence one can use in order to achieve the desired levels of protein expression both *in vitro* and *in vivo.*

The specification is most thoroughly understood in light of the following references.
1. Agrawal, S. et al. eds. (1998) Antisense Research and Application (Handbook of Experimental Pharmacology. v. 131). Springer-Verlag NY, Inc.
2. Andreeff, M. et al. eds. (1999) Introduction to Fluorescence In Situ Hybridization: Principles and Clinical Applications. John Wiley & Sons, Inc., New York, NY.
3. Ansel, H.C. et al. eds. (1999) Pharmaceutical Dosage Forms and Drug Delivery Systems. 7th ed. Lippencott Williams &Wilkins Publishers.
4. Beigelman, L. et al. (1995) Nucleic Acids Res. 23:4434-4442.
5. Chen, S.Y. et al. (1994) Hum. Gene Ther. 5:595-601.
6. Cheng, W.F. et al (2001) J. Clin. Invest. 108:669-678.
7. Chien, C. et al (1991) Proc. Natl. Acad. Sci. 88:9578-9581.
8. Coligan, J.E. et al. eds. (2002) Current Protocols in Immunology, vols. 1-4, including quarterly suppl.) John Wiley & Sons, Inc., New York, NY.
9. Deutscher, M.P. et al. eds. (1990) Guide to Protein Purification: Methods in Enzymology. (Methods in Enzymology Series, v. 182). Academic Press.
10. Dieffenbach, C.W. et al. eds. (1995) PCR Primer: A Laboratory Manual. Cold Spring Harbor Laboratory Press.
11. Durocher, Y. et al. (2002) Nucleic Acids Res. 30(2) e9.
12. Fields, S. et al. (1989) Nature 340:245-246.
13. Fukuhara, A. et al. (2004) Sciencexpress @ www.sciencexpress.org/16 December 2004/Page 1/10.1126/science.1097243.
14. Furth, P.A. et al. (1992) Anal. Biochem. 205:365-368.
15. Gaudilliere, B. et al. (2002) J. Biol. Chem. 277:46442-46446.
16. Gennaro, A., ed. (2000) Remington: The Science and Practice of Pharmacy. 20th ed. Lippincott, Williams, & Wilkins.
17. Gorman, C.M. et al. (1982) Proc. Natl. Acad. Sci. 79:6777-6781.
18. Grosschedl, R. et al. (1985) Cell 41:885-897.
19. Grosveld, F. et al. eds. (1992) Transgenic Animals. 1st ed. Academic Press.
20. Harlow, E. et al. eds. (1988) Antibodies: A Laboratory Manual. Cold Spring Harbor Laboratory.
21. Harlow, E. et al. eds. (1998) Using Antibodies: A Laboratory Manual: Portable Protocol No. I. Cold Spring Harbor Laboratory.
22. Hartmann, G. et al. eds. (1999) Manual of Antisense Methodology (Perspectives in Antisense Science). 1st ed. Kluwer Law International.
23. Hassanzadeh, G.H.G. et al. (1998) FEBS Lett. 437:75-80.
24. Heiser, A. et al. (2002) J. Clin. Invest. 109:409-417.
25. Hirschberg, C. (1987) Annu. Rev. Biochem. 56:63-87.
26. Hoogenboom, H.R. et al. (1998) Immunotechnology 4:1-20.
27. Howard, G.C. et al. (2000) Basic Methods in Antibody Production and Characterization. CRC Press.
28. Jameson, D.M. et al. (1995) Methods Enzymol. 246:283-300.
29. Jia, S.H. et al. (2004) J. Clin. Investigation 113(9): 1318-1327.
30. Jones, P. ed. (1998a) Vectors: Cloning Applications: Essential Techniques, John Wiley & Sons, Inc., New York, NY.
31. Jones, P. ed. (1998b) Vectors: Expression Systems: Essential Techniques, John Wiley & Sons, Inc., New York, NY.
32. Jost, C.R. et al. (1994) J. Biol. Chem. 269:26,267-26,273.
33. Kabat, E.A. et al. (1991) J. Immunol. 147:1709-1719.
34. Kibbe, A.H., ed. (2000) Handbook of Pharmaceutical Excipients. 3rd ed. Pharmaceutical Press.
35. Kirkpatrick, K.L. et al. (2001) Eur. J. Surg. Oncol. 27:754-760.
36. Knutson, K.L. et al. (2001) J. Clin. Invest. 107:477-484.
37. Kolonin, M.G. et al. (1998) Proc. Natl. Acad. Sci. 95:14,266-14,271.
38. Liu, A.Y. et al. (1987) Proc. Natl. Acad. Sci. 84:3439-3443.
39. Liu, A.Y. et al. (1987) J. Immunol. 139:3521-3526.
40. Machiels, J.P. et al. (2002) Semin. Oncol. 29:494-502.
41. Massie, B. et al. (1998) J. Virol., 72, 2289-2296).
42. Matz, M.V. et al. (1999) Nat. Biotechnol. 17:969-973.
43. Mayer, B.J. (2001) J. Cell Sci. 114:1253-1263.
44. Milligan, J.F. et al. (1993) J. Med. Chem. 36:1923-1937.
45. Mitchell, D.A. et al. (2000) J. Clin. Invest. 106:1065-1069.
46. Mitsumoto, Y. et al. (1991) Biochem. Biophys. Res. Commun. 175: 652-9.
47. Mitsumoto, Y. et al. (1992) J. Biol. Chem. 267: 4957-4962.
48. Okayama, H. et al. (1983) Mol. Cell. Biol. 3:280-289.
49. O'Neil, N.J. et al. (2001) Am. J. Pharmacogenomics 1:45-53.
50. Peelle, B. et al. (2001) J. Protein Chem. 20:507-519.
51. Pertl, U. et al. (2003) Blood 101:649-654.
52. Phillips, M.I., ed. (1999) Antisense Technology, Part A. Methods in Enzymology Vol. 313. Academic Press, Inc.
53. Phillips, M.I., ed. (1999) Antisense Technology, Part B. Methods in Enzymology Vol. 314. Academic Press, Inc.
54. Pinkert, C.A., ed. (1994) Transgenic Animal Technology: A Laboratory Handbook. Academic Press.
55. Remington, J.P. (1985) Remington's Pharmaceutical Sciences. 17th ed. Mack Publishing Co.
56. Samal, B. et al. (1994). Mol. Cell. Biol. 14(2): 1431-1437.
57. Sambrook, J. et al. eds. (1989) Molecular Cloning, A Laboratory Manual. 2nd ed. Cold Spring Harbor Laboratory Press.
58. Schoen, F. J. (1994) Robbins Pathologic Basis of Disease. W.B. Saunders Co., Philadelphia, PA.
59. Stein, C.A. et al. eds. (1998) Applied Antisense Oligonucleotide Technology. Wiley-Liss.
60. Tang, D.C. et al. (1992) Nature 356:152-154.
61. Wagner, R.W. et al. (1996) Nat. Biotechnol. 14:840-844.
62. Wagner, R.W. et al. (1993) Science 260:1510-1513.
63. Xu, C.W. et al. (1997) Proc. Natl. Acad. Sci. (USA) 94:12473-12478.
64. Xu, Y. et al. (1999) Proc. Natl. Acad. Sci. 96:151-156.
65. Yu, Z. et al. (2002) J. Clin. Invest. 110:289-294.
66. Zallipsky, S. (1995) Bioconjugate Chem., 6:150-165.
67. Zhu, J. et al. (1997) Proc. Natl. Acad. Sci. 94:13,063-13,068.
68. Zavyalov, et al. (1997) AP 105(3):161-186.

### Sequence Listing

Applicants include a Sequence Listing provided in both electronic and paper format as Appendix A.

### Industrial Applicability

The leader sequences, heterologous secreted polypeptides, nucleic acids, vectors, host cells and methods of making these find use in a number of investigative, diagnostic, and therapeutic applications.

**Table 1**

| **FP ID** | **Source ID** | **Annotation** |
|---|---|---|
| HG1018265 | collagen_leader_seq | collagen alpha 1(IX) chain precursor, long splice form - human |
| HG1018268 | 112907:21594845_1-17 | Alpha-2-antiplasmin precursor (Alpha-2-plasmin inhibitor) |
| HG1018269 | 112907:21594845_1-13 | Alpha-2-antiplasmin precursor (Alpha-2-plasmin inhibitor) |
| HG1018270 | 112907:21594845_1-19 | Alpha-2-antiplasmin precursor (Alpha-2-plasmin inhibitor) |
| HG1018271 | 112907:21594845_1-16 | Alpha-2-antiplasmin precursor (Alpha-2-plasmin inhibitor) |
| HG1018272 | 112907:21594845_1-15 | Alpha-2-antiplasmin precursor (Alpha-2-plasmin inhibitor) |
| HG1018274 | 13325208:13325207_1-30 | Trinucleotide repeat containing 5 [Homo sapiens] |
| HG1018275 | 13325208:13325207_1-25 | Trinucleotide repeat containing 5 [Homo sapiens] |
| HG1018276 | 13325208:13325207_1-33 | Trinucleotide repeat containing 5 [Homo sapiens] |
| HG1018277 | 13325208:13325207_1-24 | Trinucleotide repeat containing 5 [Homo sapiens] |
| HG1018278 | 13325208:13325207_1-26 | Trinucleotide repeat containing 5 [Homo sapiens] |
| HG1018279 | 13325208:13325207_1-32 | Trinucleotide repeat containing 5 [Homo sapiens] |
| HG1018280 | 13325208:13325207_1-27 | Trinucleotide repeat containing 5 [Homo sapiens] |
| HG1018281 | 13325208:13325207_1-23 | Trinucleotide repeat containing 5 [Homo sapiens] |
| HG1018282 | 13325208:13325207_1-35 | Trinucleotide repeat containing 5 [Homo sapiens] |
| HG1018284 | 13938307:13938306_1-24 | ARMET protein [Homo sapiens] |
| HG1018285 | 13938307:13938306_1-21 | ARMET protein [Homo sapiens] |
| HG1018287 | 14718453:14718452_1-19 | calumenin [Homo sapiens] |
| HG1018288 | 14718453:14718452_1-15 | calumenin [Homo sapiens] |
| HG1018289 | 14718453:14718452_1-17 | calumenin [Homo sapiens] |
| HG1018291 | 15929966:15929965_1-23 | COL9A1 protein [Homo sapiens] |
| HG1018293 | 16356651:16356650_1-21 | NBL1 [Homo sapiens] |
| HG1018294 | 16356651:16356650_1-17 | NBL1 [Homo sapiens] |
| HG1018296 | 18204192:18204191_1-19 | PACAP protein [Homo sapiens] |
| HG1018297 | 18204192:18204191_1-22 | PACAP protein [Homo sapiens] |
| HG1018298 | 18204192:18204191_1-18 | PACAP protein [Homo sapiens] |
| HG1018299 | 18204192:18204191_1-16 | PACAP protein [Homo sapiens] |
| HG1018300 | 18204192:18204191_1-14 | PACAP protein [Homo sapiens] |
| HG1018302 | 23503038:15778555_1-20 | Alpha-1B-glycoprotein precursor(Alpha-1-B glycoprotein) |
| HG1018303 | 23503038:157785555_1-16 | Alpha-1B-glycoprotein precursor (Alpha-1-B glycoprotein) |
| HG1018304 | 23503038:15778555_1-21 | Alpha-1B-glycoprotein precursor (Alpha-1-B glycoprotein) |
| HG1018306 | 27479535:27479534_1-24 | similar to Brain-specific angiogenesis inhibitor 2 precursor [Homo sapiens] |
| HG1018307 | 27479535:27479534_1-20 | similar to Brain-specific angiogenesis inhibitor 2 precursor [Homo sapiens] |
| HG1018308 | 27479535:27479534_1-26 | similar to Brain-specific angiogenesis inhibitor 2 precursor [Homo sapiens] |
| HG1018309 | 27479535:27479534_1-21 | similar to Brain-specific angiogenesis inhibitor 2 precursor [Homo sapiens] |
| HG1018310 | 27479535:27479534_1-23 | similar to Brain-specific angiogenesis inhibitor 2 precursor [Homo sapiens] |
| HG1018312 | 37182960:37182959_1-24 | SPOCK2 [Homo sapiens] |
| HG1018313 | 37182960:37182959_1-19 | SPOCK2 [Homo sapiens] |
| HG1018314 | 37182960:37182959_1-22 | SPOCK2 [Homo sapiens] |
| HG1018315 | 37182960:37182959_1-20 | SPOCK2 [Homo sapiens] |
| HG1018316 | 37182960:37182959_1-26 | SPOCK2 [Homo sapiens] |
| HG1018317 | 37182960:37182959_1-21 | SPOCK2 [Homo sapiens] |
| HG1018319 | 7437388:1208426_1-24 | protein disulfide-isomerase (EC 5341) ER60 precursor - human |
| HG1018320 | 7437388:1208426_1-23 | protein disulfide-isomerase (EC 5341) ER60 precursor - human |
| HG1018322 | NP_000286:NM_000295_1-24 | serine (or cysteine) proteinase inhibitor, clade A (alpha-1) |
| HG1018323 | NP_000286:NM_000295_1-18, | serine (or cysteine) proteinase inhibitor, clade A (alpha-1) |
| HG1018324 | NP_000286:NM_000295_ 1-23 | serine (or cysteine) proteinase inhibitor, clade A (alpha-1) |
| HG1018325 | NP_000286:NM_000295_1-17 | serine (or cysteine) proteinase inhibitor, clade A (alpha-1) |
| HG1018327 | NP_000396:NM_000405_1-23 | GM2 ganglioside activator precursor [Homo sapiens] |
| HG1018328 | NP_000396:NM_000405_1-18 | GM2 ganglioside activator precursor [Homo sapiens] |
| HG1018329 | NP_000396:NM_000405_1-25 | GM2 ganglioside activator precursor [Homo sapiens] |
| HG1018330 | NP_000396:NM_000405_1-20 | GM2 ganglioside activator precursor [Homo sapiens] |
| HG1018331 | NP_000396:NM_000405_1-21 | GM2 ganglioside activator precursor [Homo sapiens] |
| HG1018333 | NP_000495:NM_000504_1-23 | coagulation factor X precursor [Homo sapiens] |
| HG1018334 | NP_000495:NM_000504 1-19 | coagulation factor X precursor [Homo sapiens] |
| HG1018335 | NP_000495:NM_000504_1-20 | coagulation factor X precursor [Homo sapiens] |
| HG1018336 | NP_000495:NM_000504_1-15 | coagulation factor X precursor [Homo sapiens] |
| HG1018337 | NP_000495:NM_000504_1-21 | coagulation factor X precursor [Homo sapiens] |
| HG1018338 | NP_000495:NM_000504_1-17 | coagulation factor X precursor [Homo sapiens] |
| HG1018340 | NP_000573:NM_000582_1-18 | secreted phosphoprotein 1 (osteopontin, bone sialoprotein I, early) |
| HG1018341 | NP_000573:NM_000582_1-16 | secreted phosphoprotein 1 (osteopontin, bone sialoprotein I, early) |
| HG1018342 | NP_000573:NM_000582_1-15 | secreted phosphoprotein 1 (osteopontin, bone sialoprotein I, early) |
| HG 1018344 | NP_000574:NM_000583_1-16 | vitamin D-binding protein precursor [Homo sapiens] |
| HG1018345 | NP_000574:NM_000583_1-14 | vitamin D-binding protein precursor [Homo sapiens] |
| HG1018347 | NP_000591:NM_000600_1-25 | interleukin 6 (interferon, beta 2) [Homo sapiens] |
| HG1018348 | NP_000591:NM_000600_1-24 | interleukin 6 (interferon, beta 2) [Homo sapiens] |
| HG1018349 | NP_000591:NM_000600_1-27 | interleukin 6 (interferon, beta 2) [Homo sapiens] |
| HG1018351 | NP_000598:NM_000607_1-18 | orosomucoid 1 precursor [Homo sapiens] |
| HG1018353 | NP_000604:NM_000613_1-19 | hemopexin [Homo sapiens] |
| HG1018354 | NP_000604:NM_000613_1-25 | hemopexin [Homo sapiens] |
| HG1018355 | NP_000604:NM_000613_1-21 | hemopexin [Homo sapiens] |
| HG1018356 | NP_000604:NM_000613_1-23 | hemopexin [Homo sapiens] |
| HG1018357 | NP_000604:NM_000613_1-31 | hemopexin [Homo sapiens] |
| HG1018359 | NP_000726:NM 000735_1-26 | glycoprotein hormones, alpha polypeptide precursor [Homo sapiens] |
| HG1018360 | NP 000726:NM 000735_1-24 | glycoprotein hormones, alpha polypeptide precursor [Homo sapiens] |
| HG1018362 | NP_000884:NM_000893_1-18 | kininogen 1 [Homo sapiens] |
| HG1018363 | NP_000884:NM_000893 1-19 | kininogen 1 [Homo sapiens] |
| HG1018364 | NP_000884:NM_000893_1-16 | kininogen 1 [Homo sapiens] |
| HG1018365 | NP_000884:NM_000893_1-23 | kininogen 1 [Homo sapiens] |
| HG108367 | NP_000909:NM_000918_1-17 | prolyl 4-hydroxylase, beta subunit [Homo sapiens] |
| HG1018369 | NP_000930:NM_0009399_1-23 | proopiomelanocortin [Homo sapiens] |
| HG1018370 | NP_000930:NM_000939_1-26 | proopiomelanocortin [Homo sapiens] |
| HG1018372 | NP_000945:NM_000964_1-23 | prostaglandin D2 synthase 21kDa [Homo sapiens] |
| HG1018373 | NP_000945:NM_000954_1-22 | prostaglandin D2 synthase 21kDa [Homo sapiens] |
| HG1018374 | NP_000945:NM_000954_1-18 | prostaglandin D2 synthase 21kDa [Homo sapiens] |
| HG1018376 | NP_001176:NM_001185_1-18 | alpha-2-glycoprotein 1, zinc [Homo sapiens] |
| HG1018377 | NP_001176:NM_001185_1-20 | alpha-2-glycoprotein 1, zinc [Homo sapiens] |
| HG1018378 | NP_001176:NM_001185_1-21 | alpha-2-glycoprotein 1, zinc [Homo sapiens] |
| HG1018379 | NP_001176:NM 001185_1-17 | alpha-2-glycoprotein 1, zinc [Homo sapiens] |
| HG1018381 | NP_001266:NM_001275_1-18 | chromogranin A [Homo sapiens] |
| HG1018382 | NP_001266:NM_001275_1-15 | chromogranin A [Homo sapiens] |
| HG 1018383 | NP_001266:NM_00127 1-14 | chromogranin A [Homo sapiens] |
| HG1018385 | NP_001314:NM_001323_1-26 | cystatin M precursor [Homo sapiens] |
| HG1018386 | NP_001314:NM_001323_1-18 | cystatin M precursor [Homo sapiens] |
| HG1018387 | NP_001314:NM_001323_1-20 | cystatin M precursor [Homo sapiens] |
| HG1018388 | NP_001314:NM_001323_1-28 | cystatin M precursor [Homo sapiens] |
| HG1018389 | NP_001314:NM_001323_1-21 | cystatin M precursor [Homo sapiens] |
| HG1018390 | NP_001314:NM 001323 1-23 | cystatin M precursor [Homo sapiens] |
| HG1018392 | NP_001822:NM 001831 1-22 | clusterin isoform 1 [Homo sapiens] |
| HG1018393 | NP 001822:NM_001831_1-18 | clusterin isoform 1 [Homo sapiens] |
| HG1018394 | NP_001822:NM_001831_1-14 | clusterin isoform 1 [Homo sapiens] |
| HG 1018396 | NP_002206:NM_002215_1-24 | inter-alpha (globulin) inhibitor H1 [Homo sapiens] |
| HG1018397 | NP_002206:NM_002215_1-29 | inter-alpha (globulin) inhibitor H1 [Homo sapiens] |
| HG1018398 | NP_002206:NM_002215_1-30 | inter-alpha (globulin) inhibitor H1 [Homo sapiens] |
| HG1018399 | NP_002206:NM_002216_1-23 | inter-alpha (globulin) inhibitor H1 [Homo sapiens] |
| HG1018400 | NP_002206:NM_002215_1-31 | inter-alpha (globulin) inhibitor H1 [Homo sapiens] |
| HG1018402 | NP_002300:NM_002309_1-22 | leukemia inhibitory factor (cholinergic differentiation factor) |
| HG1018403 | NP_002300:NM_002309_1-23 | leukemia inhibitory factor (cholinergic differentiation factor) |
| HG1018405 | NP_002336:NM_002345_1-18 | lumican [Homo sapiens] |
| HG1018406 | NP_002336:NM_002345_1-15 | lumican [Homo sapiens] |
| HG1018407 | NP 002336:NM_002345_1-17 | lumican [Homo sapiens] |
| HG1018408 | NP_002336:NM_002345_1-14 | lumican [Homo sapiens] |
| HG1018410 | NP_002402:NM_002411_1-18 | secretoglobin, family 2A, member 2 [Homo sapiens] |
| HG1018412 | NP_002505:NM_002514_1-30 | nov precursor [Homo sapiens] |
| HG1018413 | NP_002505:NM_02514_1-32 | nov precursor [Homo sapiens] |
| HG1018414 | NP_002505:NM_002514_1-28 | nov precursor [Homo sapiens] |
| HG1018415 | NP_002505:NM_002514_1-27 | nov precursor [Homo sapiens] |
| HG1018416 | NP_002505:NM_002514_1-31 | nov precursor [Homo sapiens] |
| HG1018418 | NP_002892:NM_002901_1-26 | reticulocalbin 1 precursor [Homo sapiens] |
| HG1018419 | NP_002892:NM_002901_1-22 | reticulocalbin 1 precursor [Homo sapiens] |
| HG1018420 | NP_002892:NM_002901_1-29 | reticulocalbin 1 precursor [Homo sapiens] |
| HG1018421 | NP_002892:NM_002901_1-24 | reticulocalbin 1 precursor [Homo sapiens] |
| HG1018422 | NP_002892:NM_002901_1-23 | reticulocalbin 1 precursor [Homo sapiens] |
| HG1018424 | NP_002893:NM_002902_1-25 | reticulocalbin 2, EF-hand calcium binding domain [Homo sapiens] |
| HG1018425 | NP_002893:NM_002902_1-19 | reticulocalbin 2, EF-hand calcium binding domain [Homo sapiens] |
| HG1018426 | NP_002893:NM_002902_1-22 | reticulocalbin 2, EF-hand calcium binding domain [Homo sapiens] |
| HG1018427 | NP_002893:NM_002902_1-18 | reticulocalbin 2, EF-hand calcium binding domain [Homo sapiens] |
| HG1018428 | NP_002893:NM_002902_1-20 | reticulocalbin 2, EF-hand calcium binding domain [Homo sapiens] |
| HG1018429 | NP_002893:NM_002902_1-21 | reticulocalbin 2, EF-hand calcium binding domain [Homo sapiens] |
| HG1018430 | NP_002893:NM_002902_1-23 | reticulocalbin 2, EF-hand calcium binding domain [Homo sapiens] |
| HG1018432 | NP_005133:NM_005142_1-19 | gastric intrinsic factor (vitamin B synthesis) [Homo sapiens] |
| HG1018433 | NP_005133:NM_005142_1-18 | gastric intrinsic factor (vitamin B synthesis) [Homo sapiens] |
| HG1018434 | NP_005133:NM_005142_1-20 | gastric intrinsic factor (vitamin B synthesis) [Homo sapiens] |
| HG1018435 | NP_005133:NM_005142_1-24 | gastric intrinsic factor (vitamin B synthesis) [Homo sapiens] |
| HG1018436 | HP_005133:NM_00142_1-16 | gastric intrinsic factor (vitamin B synthesis) [Homo sapiens] |
| HG1018437 | NP_005133:NM_005142_1-17 | gastric intrinsic factor (vitamin B synthesis) [Homo sapiens] |
| HG1018438 | NP_005133:NM_005142_1-14 | gastric intrinsic factor (vitamin B synthesis) [Homo sapiens] |
| HG1018440 | NP_005445:NM_005454_1-17 | cerberus 1 [Homo sapiens] |
| HG1018442 | NP_005555:NM_005564_1-18 | lipocalin 2 (oncogene 24p3) [Homo sapiens] |
| HG 1018443 | NP_005555:NM_005564_1-20 | lipocalin 2 (oncogene 24p3) [Homo sapiens] |
| HG1018444 | NP_005555:NM_005564_1-15 | lipocalin 2 (oncogene 24p3) [Homo sapiens] |
| HG1018446 | NP_005690:NM_005699_1-29 | interleukin 18 binding protein isoform C precursor [Homo sapiens] |
| HG1018447 | NP_005690:NM_005699_1-24 | interleukin 18 binding protein isoform C precursor [Homo sapiens] |
| HG1018448 | NP_005690:NM_005699_1-28 | interleukin 18 binding protein isoform C precursor [Homo sapiens] |
| HG1018450 | NP_006560:NM_006569_1-19 | cell growth regulator with EF hand domain 1 [Homo sapiens] |
| HG1018451 | NP_006560:NM_006569_1-18 | cell growth regulator with EF hand domain 1 [Homo sapiens] |
| HG1018452 | NP_006560:NM_006569_1-21 | cell growth regulator with EF hand domain 1 [Homo sapiens] |
| HG1018454 | NP_006856:NM_006865_1-15 | leukocyte immunoglobulin-like receptor, subfamily A (without TM) |
| HG 1018456 | NP_036577:NM_012445_1-26 | spondin 2, extracellular matrix protein [Homo sapiens] |
| HG1018457 | NP_036577:NM_012445_1-25 | spondin 2, extracellular matrix protein [Homo sapiens] |
| HG1018458 | NP_036577:NM_012445_1-24 | spondin 2, extracellular matrix protein [Homo sapiens] |
| HG1018459 | NP_036577:NM_012445_1-28 | spondin 2, extracellular matrix protein [Homo sapiens] |
| HG1018461 | NP_055070:NM_014255_1-20 | transmembrane protein 4 [Homo sapiens] |
| HG1018462 | NP_055070:NM_01425_1-18 | transmembrane protein 4 [Homo sapiens] |
| HG1018463 | NP_055070:NM_014255_1-16 | transmembrane protein 4 [Homo sapiens] |
| HG1018465 | NP_055582:NM_014767_1-24 | sparc/osteonectin, cwcv and kazal-like domains proteoglycan |
| HG1018466 | NP_055582:NM_014767_1-19 | sparc/osteonectin, cwcv and kazal-like domains proteoglycan |
| HG1018467 | NP_055582:NM_014767_1-22 | sparc/osteonectin, cwcv and kazal-like domains proteoglycan |
| HG1018468 | NP_055582:NM_014767_1-20 | sparc/osteonectin, cwcv and kazal-like domains proteoglycan |
| HG1018469 | NP_055582:NM_014767_1-26 | sparc/osteonectin, cwcv and kazal-like domains proteoglycan |
| HG1018470 | NP_055582:NM_014767_1-21 | sparc/osteonectin, cwcv and kazal-like domains proteoglycan |
| HG1018472 | NP_055697:NM_014882_1-18 | Rho GTPase activating protein 25 isoform b [Homo sapiens] |
| HG1018474 | NP_056965:NM_015881_1-18 | dickkopf homolog 3 [Homo sapiens] |
| HG1018475 | NP_056965:NM_015881_1-19 | dickkopf homolog 3 [Homo sapiens] |
| HG1018476 | NP_056965:NM_015881_1-22 | dickkopf homolog 3 [Homo sapiens] |
| HG1018477 | NP_056965:NM_015881_1-16 | dickkopf homolog 3 [Homo sapiens] |
| HG1018478 | NP_056965:NM_015881_1-21 | dickkopf homolog 3 [Homo sapiens] |
| HG1018480 | NP_057603:NM_016519_1-26 | ameloblastin precursor [Homo sapiens] |
| HG1018481 | NP_057603:NM_016519_1-28 | ameloblastin precursor [Homo sapiens] |
| HG1018483 | NP_149439:NM_033183_1-18 | chorionic gonadotropin, beta polypeptide 8 recursor [Homo sapiens] |
| HG1018484 | NP_149439:NM_033183_1-20 | chorionic gonadotropin, beta polypeptide 8 |
| HG1018485 | NP_149439:NM_033183_1-16 | recursor [Homo sapiens] chorionic gonadotrophin, beta polypeptide 8 |
| HG1018487 | NP_644808:NM_139279_1-18 | recursor [Homo sapiens] multiple coagulation factor deficiency 2 [Homo |
| HG1018488 | NP_644808:NM_139279_1-20 | sapiens] multiple coagulation factor deficiency 2 |
| HG1018489 | NP_644808:NM_139279_1-26 | [Homo sapiens] multiple coagulation factor deficiency 2 |
| HG1018490 | NP_644808:NM_139279_1-23 | [Homo sapiens] multiple coagulation factor deficiency 2 |
| HG1018492 | NP_660295:NM_145252_1-13 | [Homo sapiens] similar to common salivary protein 1 |
| HG1018493 | NP_660295:NM_145252_1-16 | [Homo sapiens] similar to common salivary I |
| HG1018494 | NP_660295:NM_145252_1-14 | protein [Homo sapiens] similar to common salivary |
| HG1018495 | NP_660295:NM_145252_1-17 | protein 1 [Homo sapiens] similar to common salivary protein |
| HG1018497 | NP_689534:NM_152321_1-25 | 1 [Homo sapiens] hypothetical protein FLJ32115 [Homo |
| HG 1018498 | NP_689534:NM_152321_1-21 | sapiens] hypothetical protein FLJ32115 |
| HG1018500 | NP_689848:NM_152635_1-18 | [Homo sapiens] oncoprotein-induced transcript 3 |
| HG1018501 | NP_689848:NM_152635_1-16 | [Homo sapiens] oncoprotein-induced transcript |
| HG1018502 | NP_689848:NM_152635_1-15 | 3 [Homo sapiens] oncoprotein-induced |
| HG1018504 | NP_689968:NM_152755_1-21 | transcript 3 [Homo sapiens] hypothetical protein MGC40499 |
| HG1018506 | NP_766630:NM_173042_1-29 | [Homo sapiens] interleukin 18 binding protein isoform |
| HG1018507 | NP_766630:NM_173042_1-24 | A precursor [Homo sapiens] interleukin 18 binding |
| HG1018508 | NP_766630:NM_173042_1-28 | protein isoform A precursor [Homo sapiens] interleukin 18 binding |
| HG1018510 | NP_776214:NM_173842_1-23 | protein isoform A precursor [Homo sapiens] interleukin 1 |
| HG1018511 | NP_776214:NM_173842_1-25 | receptor antagonist isoform 1 precursor [Homo sapiens] interleukin 1 receptor |
| HG1018513 | NP_783165:NM_175575_1-32 | antagonist isoform 1 precursor [Homo sapiens] WFIKKN2 protein [Homo |
| HG1018514 | NP_783165:NM_175575_1-34 | sapiens] WFIKKN2 protein [Homo |
| HG1018515 | NP_783165:NM_175575_1-29 | sapiens] WFIKKN2 protein [Homo |
| HG1018516 | NP_783165:NM_175575_1-30 | sapiens] WFIKKN2 protein [Homo |
| HG1018517 | NP_783165:NM_175575_1-27 | sapiens] WFIKKN2 protein [Homo |
| HG1018857 | 27482680:27482679_1-26 | sapiens] similar to hypothetical |
| HG1018858 | 27482680:27482679_1-24 | protein 9330140G23 [Homo sapiens] similar to hypothetical protein 9330140G23 [Homo sapiens] |

**Table 2**

| **FP ID** | **SEQ.ID.NO. (P1)** | **Reference ID** | **Type** | **Secreted Protein** |
|---|---|---|---|---|
| HG1018265 | SEQ.ID.NO. 1 | collagen_leader_seq | leadersequence | collagen alpha 1 (IX) chain precursor, long splice form - human |
| HG1018266 | SEQ.ID.NO. 2 | CLN00517648 | full length | collagen alpha 1 (IX) chain precursor, long splice form - human |
| HG1018267 | SEQ.ID.NO. 3 | 112907:21594845 | full length | Alpha-2-antiplasmin precursor (Alpha-2-plasmin inhibitor) |
| HG1018268 | SEQ.ID.NO. 4 | 112907:21594845_1-17 | HMM_SP leader sequence | Alpha-2-antiplasmin precursor (Alpha-2-plasmin inhibitor) |
| HG1018269 | SEQ.ID.NO. 5 | 112907:21594845_1-13 | leadersequence | Alpha-2-antiplasmin precursor (Alpha-2-plasmin inhibitor) |
| HG1018270 | SEQ.ID.NO. 6 | 112907:21594845_1-19 | leader sequence | Alpha-2-antiplasmin precursor (AJpha-2-plasmin inhibitor) |
| HG1018271 | SEQ.ID.NO. 7 | 112907:21594845_1-16 | leadersequence | Alpha-2-anliplasmin precursor (Alpha-2-plasmin inhibitor) |
| HG1018272 | SEQ.ID.NO. 8 | 112907:21594845_1-15 | leader sequence | Alpha-2-antiplasmin precursor (Alpha-2-plasmin inhibitor) |
| HG1018273 | SEQ.ID.NO. 9 | 13325208:13325207 | full length | Trinucleotide repeat containing 5 [Homo sapiens] |
| HG1018274 | SEQ.ID.NO. 10 | 13325208:13325207_1-30 | HMM_SP leader sequence | Trinucleotide repeat containing 5 [Homo sapiens] |
| HG1018275 | SEQ.ID.NO. 11 | 13325208:13325207_1-25 | leadersequence | Trinucleotide repeat containing 5 [Homo sapiens] |
| HG1018276 | SEQ.ID.NO. 12 | 13325208:13325207_1-33 | leadersequence | Trinucleotide repeat containing 5 [Homo sapiens] |
| HG1018277 | SEQ.ID.NO. 13 | 13325208:13325207_1-24 | leader sequence | Trinucleotide repeat containing 5 [Homo sapiens] |
| HG1018278 | SEQ.ID.NO. 14 | 13325208:13325207_1-26 | leadersequence | Trinucleotide repeat containing 5 [Homo sapiens] |
| HG1018279 | SEQ.ID.NO. 15 | 13325208:13325207_1-32 | leadersequence | Trinucleotide repeat containing 5 [Homo sapiens] |
| HG1018280 | SEQ.ID.NO. 16 | 13325208:13325207_1-27 | leadersequence | Trinucleotide repeat containing 5 [Homo sapiens] |
| HG1018281 | SEQ.ID.NO. 17 | 13325208:13325207_1-23 | leader sequence | Trinucleotide repeat containing 5 [Homo |
| HG1018282 | SEQ.ID.NO. 18 | 13325208:13325207_1-35 | leader sequence | sapiens] Trinucleotide repeat containing 5 [Homo |
| HG1018283 | SEQ.ID.NO. 19 | 13938307:13938306 | full length | sapiens] ARMET protein [Homo sapiens] |
| HG1018284 | SEQ.ID.NO. 20 | 13938307:13938306_1-24 | HMM SP leader sequence | ARMET protein [Homo sapiens] |
| HG1018285 | SEQ.ID.NO. 21 | 13938307:13938306_1-21 | leader sequence | ARMET protein [Homo sapiens] |
| HG1018286 | SEQ.ID.NO. 22 | 14718453:14718452 | full length | calumenin [Homo |
| HG1018287 | SEQ.ID.NO. 23 | 14718453:14718452_1-19 | HMM SP leader sequence | sapiens] calumenin [Homo |
| HG1018288 | SEQ.ID.NO. 24 | 14718453:14718452_1-15 | leader sequence | sapiens] calumenin [Homo sapiens] |
| HG1018289 | SEQ.ID.NO. 25 | 14718453:14718452_1-17 | leader sequence | calumenin [Homo sapiens] |
| HG1018290 | SEQ.ID.NO. 26 | 15929966:15929965 | full length | COL9A1 protein |
| HG1018291 | SEQ.ID.NO. 27 | 15929966:15929965_1-23 | HMM SP leader sequence | [Homo sapiens] COL9A1 protein |
| HG1018292 | SEQ.ID.NO. 28 | 16356651:16356650 | full length | [Homo sapiens] NBL1 [Homo sapiens] |
| HG1018293 | SEQ.ID.NO. 29 | 16356651:16356650_1-21 | leadersequence | NBL1 [Homo |
| HG1018294 | SEQ.ID.NO. 30 | 16356651:16356650_1-17 | leadersequence | sapiens] NBL1 [Homo |
| HG1018295 | SEQ.ID.NO. 31 | 18204192:18204191 | full length | sapiens] PACAP protein |
| HG1018296 | SEQ.ID.NO. 32 | 18204192:18204191_1-19 | HMM_SP leader | [Homo sapiens] PACAP |
| HG1018297 | SEQ.ID.NO. 33 | 18204192:18204191_1-22 | sequence leader sequence | protein [Homo sapiens] PACAP protein |
| HG1018298 | SEQ.ID.NO. 34 | 18204192:18204191_1-18 | leader sequence | [Homo sapiens] PACAP |
| HG1018299 | SEQ.ID.NO. 35 | 18204192:18204191_1-16 | leadersequence | protein [Homo sapiens] PACAP protein |
| HG1018300 | SEQ.ID.NO. 36 | 18204192:18204191_1-14 | leadersequence | [Homo sapiens] PACAP protein [Homo |
| HG1018301 | SEQ.ID.NO. 37 | 23503038:15778555 | full length | sapiens] Alpha-1B-glycoprotein |
| HG1018302 | SEQ.ID.NO. 38 | 23503038:15778555_1-20 | leader sequence | precursor (Alpha-1-B glycoprotein) Alpha-1 B-glycoprotein |
| HG1018303 | SEQ.ID.NO. 39 | 23503038:15778555_1-16 | leader sequence | precursor (Alpha-1-B glycoprotein) Alpha-1B-glycoprotein |
| HG1018304 | SEQ.ID.NO. 40 | 23503038:15778555_1-21 | leadersequence | precursor (Alpha-1-Bglycoprotein) Alpha-1B-glycoprotein (Alpha-1-B |
| HG1018305 | SEQ.ID.NO. 41 | 27479535:27479534 | full length | precursor glycoprotein) similar to Brain-specific angiogenesis inhibitor 2 |
| HG1018306 | SEQ.ID.NO. 42 | 27479535:27479534_1-24 | HMM SP leader sequence | precursor [Homo sapiens] similar to Brain-specific angiogenesis inhibitor 2 precursor [Homo sapiens] |
| HG1018307 | SEQ.ID.NO. 43 | 27479535:27479534_1-20 | leader sequence | similar to Brain-specific angiogenesis inhibitor 2 precursor [Homo sapiens] |
| HG1018308 | SEQ.ID.NO. 44 | 27479535:27479534_1-26 | leader sequence | similar to Brain-specific angiogenesis inhibitor 2 precursor [Homo sapiens] |
| HG1018309 | SEQ.ID.NO. 45 | 27479535:27479534_1-21 | leader sequence | similar to Brain-specific angiogenesis inhibitor 2 precursor [Homo sapiens] |
| HG1018310 | SEQ.ID.NO. 46 | 27479535:27479534 1-23 | leadersequence | similar to Brain-specific angiogenesis inhibitor 2 precursor [Homo sapiens] |
| HG1018311 | SEQ.ID.NO. 47 | 37182960:37182959 | full length | SPOCK2 [Homo sapiens] |
| HG1018312 | SEQ.ID.NO. 48 | 37182960:37182959_1-24 | HMM_SP leader sequence | SPOCK2 [Homo sapiens] |
| HG1018313 | SEQ.ID.NO. 49 | 37182960:37182959_1-19 | leadersequence | SPOCK2 [Homo sapiens] |
| HG1018314 | SEQ.ID.NO. 50 | 37182960:37182959_1-22 | leader sequence | SPOCK2 [Homo sapiens] |
| HG1018315 | SEQ.ID.NO. 51 | 37182960:37182959_1-20 | leader sequence | SPOCK2 [Homo sapiens] |
| HG1018316 | SEQ.ID.NO. 52 | 37182960:37182959_1-26 | leader sequence | SPOCK2 [Homo sapiens] |
| HG1018317 | SEQ.ID.NO. 53 | 37182960:37182959_1-21 | leader sequence | SPOCK2 [Homo sapiens] |
| HG1018318 | SEQ.ID.NO. 54 | 7437388:1208426 | full length | protein disulfide-isomerase (EC 5341) ER60 precursor - human |
| HG1018319 | SEQ.ID.NO. 55 | 7437388:1208426_1-24 | HMM SP leader sequence | protein disulfide-isomerase (EC 5341) ER60 precursor- human |
| HG1018320 | SEQ.ID.NO. 56 | 7437388:1208426_1-23 | leadersequence | protein disulfide-isomerase (EC 5341) ER60 precursor- human |
| HG1018321 | SEQ.ID.NO. 57 | NP_000286:NM_000295 | full length | serine (or cysteine) proteinase inhibitor, clade A (alpha-1 |
| HG1018322 | SEQ.ID.NO. 58 | NP_000286:NM_000295_1-24 | HMM_SP leader sequence | serine (or cysteine) proteinase inhibitor, clade A (alpha-1 |
| HG1018323 | SEQ.ID.NO. 59 | NP_000286:NM_000295_1-18 | leader sequence | serine (or cysteine) proteinase inhibitor, clade A (alpha-1 |
| HG1018324 | SEQ.ID.NO. 60 | NP_000286:NM_000295_1-23 | leader sequence | serine (or cysteine) proteinase inhibitor, clade A (alpha-1 |
| HG1018325 | SEQ.ID.NO. 61 | NP_000286:NM_000295_1-17 | leader sequence | serine (or cysteine) proteinase inhibitor, clade A (alpha-1 |
| HG1018326 | SEQ.ID.NO. 62 | NP_000396:NM_000405 | full length | GM2 ganglioside activator precursor [Homo sapiens] |
| HG1018327 | SEQ.ID.NO. 63 | NP_000396:NM_000405_1-23 | HMM_SP leader sequence | ) GM2 ganglioside activator precursor [Homo sapiens] |
| HG1018328 | SEQ.ID.NO. 64 | NP_000396:NM_000405_1-18 | leader sequence | ) GM2 ganglioside activator precursor [Homo sapiens] |
| HG1018329 | SEQ.ID.NO. 65 | NP_000396:NM_000405_1-25 | leader sequence | ) GM2 ganglioside activator precursor [Homo sapiens] ) |
| HG1018330 | SEQ.ID.NO. 68 | NP_000396:NM_000405_1-20 | leader sequence | GM2 ganglioside activator precursor [Homo sapiens] ) |
| HG1018331 | SEQ.ID.NO. 67 | NP_000396:NM_000405_1-21 | leader sequence | GM2 ganglioside activator precursor [Homo sapiens] |
| HG1018332 | SEQ.ID.NO. 68 | NP_000495:NM_000504 | full length | ) coagulation factor X precursor [Homo sapiens] |
| HG1018333 | SEQ.ID.NO. 69 | NP_000495:NM_000504_1-23 | HMM_SP leader sequence | coagulation factor X precursor [Homo sapiens] |
| HG1018334 | SEQ.ID.NO. 70 | NP_000495:NM_000504_1-19 | leadersequence | coagulation factor X precursor [Homo sapiens] |
| HG1018335 | SEQ.ID.NO. 71 | NP_000495:NM_000504_1-20 | leader sequence | coagulation factor X precursor [Homo sapiens] |
| HG1018336 | SEQ.ID.NO. 72 | NP_000495:NM_000504_1-15 | leader sequence | coagulation factor X precursor [Homo sapiens] |
| HG1018337 | SEQ.ID.NO. 73 | NP_000495:NM_000504_1-21 | leader sequence | coagulation factor X precursor [Homo sapiens] |
| HG1018338 | SEQ.ID.NO. 74 | NP_000495:NM_000504_1-17 | leader sequence | coagulation factor X precursor [Homo sapiens] |
| HG1018339 | SEQ.ID.NO. 75 | NP_000573:NM_000582 | full length | secreted phosphoprotein 1 (osteoponlin, bone sialoprotein |
| HG1018340 | SEQ.ID.NO. 76 | NP_000573:NM_000582_1-18 | HMM_SP leader sequence | 1, early secreted phosphoprotein 1 (osteopontin, bone sialoprotein 1, |
| HG1018341 | SEQ.ID.NO. 77 | NP_000573:NM_000582_1-16 | leader sequence | early secreted phosphoprotein 1 (osteopontin, bone sialoprotein 1, |
| HG1018342 | SEO.ID.NO. 78 | NP_000573:NM_000582_1-15 | leader sequence | early secreted phosphoprotein 1 (osteopontin, bone sialoprotein 1, early |
| HG1018343 | SEQ.ID.NO. 79 | NP_000574:NM_000583 | full length | vitamin D-binding protein precursor [Homo sapiens] |
| HG1018344 | SEQ.ID.NO. 80 | NP_000574:NM_000583_1-16 | HMM_SP leader sequence | vitamin D-binding protein [Homo sapiens] |
| HG1018345 | SEQ.ID.NO. 81 | NP_000574:NM_000583_1-14 | leadersequence | precursor vitamin D-binding protein precursor [Homo sapiens] |
| HG1018346 | SEQ.ID.NO. 82 | NP_000591:NM_000600 | full length | interleukin 6 (interferon, beta 2) [Homo sapiens] |
| HG1018347 | SEQ.ID.NO. 83 | NP_000591:NM_000600_1-25 | HMM_SP leader sequence | interleukin 6 (interferon, beta 2) [Homo sapiens] |
| HG1018348 | SEQ.ID.NO. 84 | NP_000591:NM_000600_1-24 | leader sequence | interleukin 6 (interferon, beta 2) [Homo |
| HG1018349 | SEQ.ID.NO. 85 | NP_000591:NM_000600_1-27 | leader sequence | sapiens] interleukin 6 (interferon, beta 2) [Homo sapiens] |
| HG1018350 | SEO.ID.NO. 86 | NP_000598:NM_000607 | full length | orosomucoid 1 precursor [Homo sapiens] |
| HG1018351 | SEQ.ID.NO. 87 | NP_000598:NM_000607_1-18 | HMM_SP leader sequence | orosomucoid 1 precursor [Homo sapiens] |
| HG1018352 | SEQ.ID.NO. 88 | NP_000604:NM_000613 | full length | hemopexin [Homo sapiens] |
| HG1018353 | SEQ.ID.NO. 89 | NP_000604:NM_000613_1-19 | leader sequence | hemopexin [Homo sapiens] |
| HG1018354 | SEQ.ID.NO. 90 | NP_000604:NM_000613_1-25 | leader sequence | hemopexin [Homo sapiens] |
| HG1018355 | SEQ.ID.NO. 91 | NP_000604:NM_000613_1-21 | leader sequence | hemopexin [Homo sapiens] |
| HG1018356 | SEQ.ID.NO. 92 | NP_000604:NM_000613_1-23 | leader sequence | hemopexin [Homo sapiens] |
| HG1018357 | SEQ.ID.NO. 93 | NP_000604:NM_000613_1-31 | leader sequence | hemopexin [Homo sapiens] |
| HG1018358 | SEQ.ID.NO. 94 | NP_000726:NM_000735 | full length | glycoprotein hormones, alpha polypeptide precursor [Homo sapiens] |
| HG1018359 | SEQ.ID.NO. 95 | NP_000726:NM_000735_1-26 | HMM_SP leader sequence | glycoprotein hormones, alpha polypeptide precursor [Homo sapiens] |
| HG1018360 | SEQ.ID.NO. 96 | NP_000726:NM_000735_1-24 | leader sequence | glycoprotein hormones, alpha polypeptide precursor [Homo sapiens] |
| HG1018361 | SEQ.ID.NO. 97 | NP_000884:NM_000893 | full length | kininogen 1 [Homo sapiens] |
| HG1018362 | SEQ.ID.NO. 98 | NP_000884:NM_000893_1-18 | HMM_SP leader sequence | kininogen 1 [Homo sapiens] |
| HG1018363 | SEQ.ID.NO. 99 | NP 000884:NM_000893_1-19 | leader sequence | kininogen 1 [Homo sapiens] |
| HG1018364 | SEQ.ID.NO. 100 | NP_000884:NM_000893_1-16 | leader sequence | kininogen 1 [Homo sapiens] |
| HG1018365 | SEQ.ID.NO. 101 | NP_000884:NM_000893_1-23 | leader sequence | kininogen 1 [Homo sapiens] |
| HG1018366 | SEQ.ID.NO. 102 | NP_000909:NM_000918 | full length | prolyl 4-hydroxylase, beta subunit [Homo sapiens] |
| HG1018367 | SEQ.ID.NO. 103 | NP_000909:NM_000918_1-17 | leader Sequence HMM_SP leader sequence | prolyl 4-hydroxylase, beta subunit [Homo sapiens] |
| HG1018368 | SEQ.ID.NO. 104 | NP_000930:NM_000939 | full length | proopiomelanocortin [Homo sapiens] |
| HG1018369 | SEQ.ID.NO. 105 | NP_000930:NM_000939_1-23 | HMM_SP leader sequence | proopiomelanocortin [Homo sapiens] |
| HG1018370 | SEQ.ID.NO. 106 | NP_000930:NM_000939_1-26 | leader sequence | proopiomelanocortin [Homo sapiens] |
| HG1018371 | SEQ.ID.NO. 107 | NP_000945:NM_000954 | full length | prostaglandin D2 synthase 21kDa [Homo sapiens] |
| HG1018372 | SEQ.ID.NO. 108 | NP_000945:NM_000954_1-23 | HMM_SP leader sequence | prostaglandin D2 synthase 21kDa [Homo sapiens] |
| HG1018373 | SEQ.ID.NO. 109 | NP_ 000945:NM_000954_1-22 | leader sequence | prostaglandin D2 synthase 21kDa [Homo sapiens] |
| HG1018374 | SEQ.ID.NO. 110 | NP_000945:NM_000954_1-18 | leader sequence | prostaglandin D2 synthase 21kDa [Homo sapiens] |
| HG1018375 | SEQ.ID.NO. 111 | NP_001176:NM_001185 | full length | alpha-2-glycoprotein 1, zinc [Homo sapiens] |
| HG1018376 | SEQ.ID.NO. 112 | NP_001176:NM_001185_1-18 | leader sequence | alpha-2-glycoprotein 1, zinc [Homo sapiens] |
| HG1018377 | SEQ.ID.NO. 113 | NP_001176:NM_001185_1.20 | leader sequence | alpha-2-glycoprotein 1, zinc [Homo |
| HG1018378 | SEQ.ID.NO. 114 | NP_001176:NM_001185_1-21 | leader sequence | sapiens] alpha-2-glycoprotein 1, zinc [Homo |
| HG1018379 | SEQ.ID.NO. 115 | NP_001176:NM_001185_1-17 | leader sequence | sapiens] alpha-2-glycoprolein 1, zinc [Homo |
| HG1018380 | SEQ.ID.NO. 116 | NP_001266:NM_001275 | full length | sapiens] chromogranin A [Homo |
| HG1018381 | SEQ.ID.NO. 117 | NP_001266:NM_001275_1-18 | HMM_SP leader sequence | sapiens] chromogranin A [Homo |
| HG1018382 | SEQ.ID.NO. 118 | NP_001266:NM_001275_1-15 | leader sequence | sapiens] chromogranin A [Homo |
| HG1018383 | SEQ.ID.NO. 119 | NP_001266:NM_001275_1-14 | leader sequence | sapiens] chromogranin A [Homo sapiens] |
| HG1018384 | SEQ.ID.NO. 120 | NP_001314:NM_001323 | full length | cystatin M precursor [Homo sapiens] |
| HG1018385 | SEQ.ID.NO. 121 | NP_001314:NM_001323_1-26 | HMM_SP leader sequence | cystatin M precursor [Homo sapiens] |
| HG1018386 | SEQ.ID.NO. 122 | NP_001314:NM_001323_1-18 | leader sequence | cystatin M precursor [Homo sapiens] |
| HG1018387 | SEQ.ID.NO. 123 | NP_001314:NM_001323_1-20 | leader sequence | cystatin M precursor [Homo sapiens] |
| HG1018388 | SEQ.ID.NO. 124 | NP_001314:NM_001323_1-28 | leader sequence | cystatin M precursor [Homo sapiens] |
| HG1018389 | SEQ.ID.NO. 125 | NP_001314:NM_001323_1-21 | leader sequence | cystatin M precursor [Homo sapiens] |
| HG1018390 | SEQ.ID.NO. 126 | NP_001314:NM_001323_1-23 | leader sequence | cystatin M precursor [Homo sapiens] |
| HG1018391 | SEQ.ID.NO. 127 | NP_001822:NM_001831 | full length | clusterin isoform 1 [Homo sapiens] |
| HG1018392 | SEQ.ID.NO. 128 | NP_001822:NM_001831_1-22 | leader sequence | clusterin isoform 1 [Homo sapiens] |
| HG1018393 | SEQ.ID.NO. 129 | NP_001822:NM_001831_1-18 | leader sequence | clusterin Isoform 1 [Homo sapiens] |
| HG1018394 | SEQ.ID.NO. 130 | NP_001822:NM_001831_1-14 | leader sequence | clusterin isoform 1 [Homo sapiens] |
| HG1018395 | SEQ.ID.NO. 131 | NP_002206:NM_002215 | full length | inter-alpha (globulin) inhibitor H1 [Homo sapiens] |
| HG1018396 | SEQ.ID.NO. 132 | NP_002206:NM_002215_1-24 | leader sequence | inter-alpha (globulin) inhibitor H1 [Homo sapiens] |
| HG1018397 | SEQ.ID.NO. 133 | NP_002206:NM_002215_1-29 | leader sequence | inter-alpha (globulin) inhibitor H1 [Homo sapiens] |
| HG1018398 | SEQ.ID.NO. 134 | NP_002206:NM_002215_1-30 | leader sequence | inter-alpha (globulin) inhibitor H1 [Homo sapiens] |
| HG1018399 | SEQ.ID.NO. 135 | NP 002206:NM_002215_1-23 | leader sequence | inter-alpha (globulin) inhibitor H1 [Homo sapiens] |
| HG1018400 | SEQ.ID.NO. 136 | NP_002206:NM_002215_1-31 | leader sequence | inter-alpha (globulin) inhibitor H1 [Homo sapiens] |
| HG1018401 | SEQ.ID.NO. 137 | NP_002300:NM_002309 | full length | leukemia inhibitory factor (cholinergic differentiation factor) |
| HG1018402 | SEQ.ID.NO. 138 | NP_002300:NM_002309_1-22 | HMM_SP leader sequence | leukemia inhibitory factor (cholinergic differentiation factor) |
| HG1018403 | SEQ.ID.NO. 139 | NP_002300:NM_002309_1-23 | leader sequence | leukemia inhibitory factor (cholinergic differentiation factor) |
| HG1018404 | SEQ.ID.NO. 140 | NP_002336:NM_002345 | full length | lumican [Homo sapiens] |
| HG1018405 | SEQ.ID.NO. 141 | NP_002336:NM_002345_1-18 | HMM_SP leader sequence | lumican [Homo sapiens] |
| HG1018406 | SEQ.ID.NO. 142 | NP_002336:NM_002345_1-15 | leader sequence | lumican [Homo sapiens] |
| HG1018407 | SEQ.ID.NO. 143 | NP_002336:NM_002345_1-17 | leader sequence | lumican [Homo sapiens] |
| HG1018408 | SEQ.ID.NO. 144 | NP_002336:NM_002345_1-14 | leader sequence | lumican [Homo sapiens] |
| HG1018409 | SEQ.ID.NO. 145 | NP 002402:NM_002411 | full length | secretoglobin, family 2A, member 2 [Homo sapiens] |
| HG1018410 | SEQ.ID.NO. 146 | NP_002402:NM_002411_1-18 | HMM_SP leader sequence | secretoglobin, family 2A, member 2 [Homo sapiens] |
| HG1018411 | SEQ.ID.NO. 147 | NP_002505:NM_002514 | full length | nov precursor [Homo sapiens] |
| HG1018412 | SEQ.ID.NO. 148 | NP_002505:NM_002514_1-30 | HMM_SP leader sequence | nov precursor [Homo sapiens] |
| HG1018413 | SEQ.ID.NO. 149 | NP_002505:NM_002514_1-32 | leader sequence | nov precursor [Homo sapiens] |
| HG1018414 | SEQ.ID.NO. 150 | NP_002505:NM_002514_1-28 | leader sequence | nov precursor [Homo sapiens] |
| HG1018415 | SEQ.ID.NO. 151 | NP_002505:NM_002514_1-27 | leader sequence | nov precursor [Homo sapiens] |
| HG1018416 | SEQ.ID.NO. 152 | NP_002505:NM_002514_1-31 | leader sequence | nov precursor [Homo sapiens] |
| HG1018417 | SEQ.ID.NO. 153 | NP_002892:NM_002901 | full length | reticulocalbin 1 precursor [Homo sapiens] |
| HG1018418 | SEQ.ID.NO. 154 | NP_002692:NM_002901_1-26 | HMM_SP leader sequence | reticulocalbin 1 precursor [Homo sapiens] |
| HG1018419 | SEQ.ID.NO. 155 | NP_002892:NM_002901_1-22 | leader sequence | reticulocalbin 1 precursor [Homo sapiens] |
| HG1018420 | SEQ.ID.NO. 156 | NP_002892:NM_002901_1-29 | leader sequence | reticulocalbin 1 precursor [Homo sapiens] |
| HG1018421 | SEQ.ID.NO. 157 | NP_002892:NM_002901 1-24 | leader sequence | reticulocalbin 1 precursor [Homo sapiens] |
| HG1018422 | SEQ.ID.NO. 158 | NP_002892:NM_002901_1-23 | leader sequence | reticulocalbin 1 precursor [Homo sapiens] |
| HG1018423 | SEQ.ID.NO. 159 | NP_002893:NM_002902 | full length | reticulocalbin 2, EF-hand calcium binding domain [Homo sapiens] |
| HG1018424 | SEQ.ID.NO. 160 | NP_002893:NM_002902_1-25 | HMM_SP leader sequence | reticulocalbin 2, EF-hand calcium binding domain [Homo sapiens] |
| HG1018425 | SEQ.ID.NO. 161 | NP_002893:NM_002902_1-19 | leader sequence | reticulocalbin 2, EF-hand calcium binding domain [Homo sapiens] |
| HG1018426 | SEQ.ID.NO. 162 | NP_002893:NM_0029D2_1-22 | leader sequence | reticulocalbin 2, EF-hand calcium binding domain [Homo sapiens] |
| HG1018427 | SEQ.ID.NO. 163 | NP_002893:NM_002902_1-18 | leader sequence | reticulocalbin 2, EF-hand calcium binding domain [Homo sapiens] |
| HG1018428 | SEQ.ID.NO. 164 | NP_002893:NM_002902_1-20 | leader sequence | reticulor-albin 2, EF-hand calcium binding domain [Homo sapiens] |
| HG1018429 | SEQ.ID.NO. 165 | NP_002893:NM_002902_1-21 | leader sequence | reticulocalbin 2, EF-hand calcium binding domain [Homo sapiens] |
| HG1018430 | SEO.ID.NO. 166 | NP_002893:NM_002902_1-23 | leader sequence | reticulocalbin 2, EF-hand calcium binding domain [Homo sapiens] |
| HG1018431 | SEQ.ID.NO. 167 | NP_005133:NM_005142 | full length | gastric intrinsic factor (vitamin B synthesis) [Homo sapiens] |
| HG1018432 | SEQ.ID.NO. 168 | NP_005133:NM_005142_1-19 | HMM_SP leader sequence | gastric intrinsic factor (vitamin B synthesis) [Homo sapiens] |
| HG1018433 | SEQ.ID.NO. 169 | NP_005133:NM_005142_1-18 | leadersequence | gastric intrinsic factor (vitamin B synthesis) [Homo sapiens] |
| HG1018434 | SEQ.ID.NO. 170 | NP_005133:NM_005142 1-20 | leader sequence | gastric intrinsic factor (vitamin B synthesis) [Homo sapiens] |
| HG1018435 | SEQ.ID.NO. 171 | NP_005133:NM_005142_1-24 | leader sequence | gastric intrinsic factor (vitamin B synthesis) [Homo sapiens] |
| HG1018436 | SEQ.ID.NO. 172 | NP_005133:NM_005142_16 | leader sequence | gastric intrinsic factor (vitamin B synthesis) [Homo sapiens] |
| HG1018437 | SEQ.ID.NO. 173 | NP_005133:NM_005142_1-17 | leader sequence | gastric Intrinsic factor (vitamin B synthesis) [Homo sapiens] |
| HG1018438 | SEQ.ID.NO. 174 | NP_005133:NM_005142_1-14 | leadersequence | gastric intrinsic factor (vitamin B synthesis) [Homo sapiens] |
| HG1018439 | SEQ.ID.NO. 175 | NP_005445:NM_005454 | full length | cerberus 1 [Homo sapiens] |
| HG1018440 | SEQ.ID.NO. 176 | NP_005445:NM_005454_1-17 | HMM_SP leader sequence | cerberus 1 [Homo sapiens] |
| HG1018441 | SEQ.ID.NO. 177 | NP_005555:NM_005564 | full length | lipocalin 2 (oncogene 24p3) [Homo sapiens] |
| HG1018442 | SEQ.ID.NO- 178 | NP_005555:NM_005564_1-18 | HMM_SP leader sequence | lipocalin 2 (oncogene 24p3) [Homo sapiens] |
| HG1018443 | SEQ.ID.NO. 179 | NP_005555:NM_005564_1-20 | leader sequence | lipocalin 2 (oncogene 24p3) [Homo sapiens] |
| HG1018444 | SEQ.ID.NO. 180 | NP_005555:NM_005564_1-15 | leadersequence | lipocalin 2 (oncogene 24p3) [Homo sapiens] |
| HG1018445 | SEQ.ID.NO. 181 | NP_005690:NM 005699 | full length | interleukin 18 binding protein isoform C precursor [Homo sapiens] |
| HG1018446 | SEQ.ID.NO. 182 | NP_005690:NM_005699_1-29 | HMM_SP leader sequence | interleukin 1 binding protein isoform C precursor [Homo sapiens] |
| HG1018447 | SEQ.ID.NO. 183 | NP_005690:NM_005699_1-24 | leader sequence | interleukin 18 binding protein isoform C precursor [Homo sapiens] |
| HG1018448 | SEQ.ID.NO. 184 | NP_005690:NM_005699_1-28 | leader sequence | interleukin 18 binding protein isoform C precursor [Homo sapiens] |
| HG1018449 | SEQ.ID.NO. 185 | NP_006560:NM_006569 | full length | cell growth regulator with EF hand domain 1 [Homo sapiens] |
| HG1018450 | SEQ.ID.NO. 186 | NP_006560:NM_006569_1-19 | HMM SP leader sequence | cell growth regulator with EF hand domain 1 [Homo sapiens] |
| HG1018451 | SEQ.ID.NO. 187 | NP_006560:NM_006569_1-18 | leadersequence | cell growth regulator with EF hand domain 1 [Homo sapiens] |
| HG1018452 | SEQ.ID.NO. 188 | NP_006560:NM_006569_1-21 | leader sequence | cell growth regulator with EF hand domain 1 [Homo sapiens] |
| HG1018453 | SEQ.ID.NO. 189 | NP_006856:NM_006865 | full length | leukocyte immunoglobulin-like receptor, subfamily A (without TM) |
| HG1018454 | SEQ.ID.NO.190 | NP_006856:NM_006865_1-15 | HMM_SP leader sequence | leukocyte immunoglobulin-like receptor, subfamily A (without TM) |
| HGT018455 | SEQ.ID.NO.191 | NP_036577:NM_012445 | full length | spondin 2, extracellular matrix protein [Homo sapiens] |
| HG1018456 | SEQ.ID.NO. 192 | NP_036577:NM_012445_1-26 | HMM_SP leader sequence | spondin 2, extracellular matrix protein [Homo sapiens] |
| HG1018457 | SEQ.ID.NO. 193 | NP_036577:NM_012445_1-25 | leadersequence | spondin 2, extracellular matrix protein [Homo sapiens] |
| HG1018458 | SEQ.ID.NO. 194 | NP_036577:NM_012445_1-24 | leader sequence | spondin 2, extracellular matrix protein [Homo sapiens] |
| HG1018459 | SEQ.ID.NO. 195 | NP_036577:NM_012445_1-28 | leader sequence | spondin 2, extracellular matrix protein [Homo sapiens] |
| HG1018460 | SEQ.ID.NO. 196 | NP_055070:NM_014255 | full length | transmembrane protein 4 [Homo sapiens] |
| HG1018461 | SEQ.ID.NO. 197 | NP_055070;NM_014255_1-20 | HMM_SP leader sequence | transmembrane protein 4 [Homo sapiens] |
| HG1018462 | SEQ.ID.NO. 198 | NP_055070:NM_014255_1-18 | leader sequence | transmembrane protein 4 [Homo sapiens] |
| HG1018463 | SEQ.ID.NO. 199 | NP_055070:NM_014255_1-16 | leader sequence | transmembrane protein 4 [Homo sapiens] |
| HG1018464 | SEQ.ID.NO. 200 | NP_055582:NM_014767 | full length | sparc/osteonectin, cwcv and kazal-like domains proteoglycan |
| HG1018465 | SEQ.ID.NO. 201 | NP_055582:NM_014767_1-24 | HMM_SP leader sequence | sparc/osteonectin, cwcv and kazal-like domains proteoglycan |
| HG1018466 | SEQ.ID.NO. 202 | NP_055582:NM 014767_1-19 | leader sequence | sparc/osteonectin, cwcv and kazal-like domains proteoglycan |
| HG1018467 | SEQ.ID.NO. 203 | NP_055582:NM_014767_1-22 | leader sequence | sparc/osteonectin, cwcv and kazal-like domains proteoglycan |
| HG1018468 | SEQ.ID.NO. 204 | NP_055582:NM_014767_1-20 | leader sequence | sparc/osteonectin, cwcv and kazal-like domains proteoglycan |
| HG1018469 | SEQ.ID.NO. 205 | NP_055582:NM_014767_1-26 | leader sequence | sparc/osteoneclin, cwcv and kazal-like domains proteoglycan |
| HG1018470 | SEQ.ID.NO. 206 | NP_055582:NM_014767_1-21 | leader sequence | sparc/osteonectin, cwcv and kazal-like domains proteoglycan |
| HG1018471 | SEQ.ID.NO. 207 | NP_055697:NM_014882 | full length | Rho GTPase activating protein 25 isoform b [Homo sapiens] |
| HG1018472 | SEQ.ID.NO. 208 | NP_055697:NM_014882_1-18 | HMM_SP leader sequence | Rho GTPase activating protein 25 isoform b [Homo sapiens] |
| HG1018473 | SEQ.ID.NO. 209 | NP_056965:NM_015881 | full length | dickkopf homolog 3 [Homo sapiens] |
| HG1018474 | SEQ.ID.NO. 210 | NP_056965:NM_015881_1-18 | HMM_SP leader sequence | dickkopf homolog 3 [Homo sapiens] |
| HG1018475 | SEQ.ID.NO. 211 | NP_056965:NM_015881_1-19 | leader sequence | dickkopf homolog 3 [Homo sapiens] |
| HG1018476 | SEQ.ID.NO. 212 | NP_056965:NM_015881_1-22 | leader sequence | dickkopf homolog 3 [Homo sapiens] |
| HG1018477 | SEQ.ID.NO. 213 | NP_056965:NM_015881_1-16 | leadersequence | dickkopf homolog 3 [Homo sapiens] |
| HG1018478 | SEQ.ID.NO. 214 | NP_056965:NM_01581_1-21 | leadersequence | dickkopf homolog 3 [Homo sapiens] |
| HG1018479 | SEQ.ID.NO.215 | NP_057603:NM_016519 | full length | ameloblastin precursor [Homo sapiens] |
| HG1018480 | SEQ.ID.NO. 216 | NP_057603:NM_016519_1-26 | leadersequence | ameloblastin precursor [Homo sapiens] |
| HG1018481 | SEQ.ID.NO. 217 | NP_057603:NM_016519_1-28 | leader sequence | ameloblastin precursor [Homo sapiens] |
| HG1018482 | SEQ.ID.NO. 218 | NP_149439:NM_033183 | full length | chorionic gonadotropin, beta polypeptide 8 recursor [Homo sapiens] |
| HG1018483 | SEQ.ID.NO. 219 | NP_149439:NM_033183_1-18 | HMM_SP leader sequence | chorionic gonadotropin, beta polypeptide 8 recursor [Homo sapiens] |
| HG1018484 | SEQ.ID.NO. 220 | NP_149439:NM_033183_1-20 | leadersequence | chorionic gonadotropin, beta polypeptide 8 recursor IHomo sapiens] |
| HG1018485 | SEQ.ID.NO. 221 | NP_149439:NM_033183_1-16 | leadersequence | chorionic gonadotropin, beta polypeptide 8 recursor [Homo sapiens] |
| HG1018486 | SEQ.ID.NO. 222 | NP_644808:NM_139279 | full length | multiple coagulation factor deficiency 2 [Homo sapiens] |
| HG1018487 | SEQ.ID.NO. 223 | NP_644808:NM_139279_1-18 | leadersequence | multiple coagulation factor deficiency 2 [Homo sapiens] |
| HG1018488 | SEQ.ID.NO. 224 | NP_644808:NM_139279_1-20 | leader sequence | multiple coagulation factor deficiency 2 [Homo sapiens] |
| HG1018489 | SEQ.ID.NO. 225 | NP_644808:NM_139279_1-26 | leader sequence | multiple coagulation factor deficiency 2 [Homo sapiens] |
| HG1018490 | SEQ.ID.NO. 226 | NP_644808:NM_139279_1-23 | leader sequence, | multiple coagulation factor deficiency 2 [Homo sapiens] |
| HG1018491 | SEQ.ID.NO. 227 | NP_660295:NM_145252 | full length | similar to common salivary protein 1 [Homo sapiens] |
| HG1018492 | SEQ.ID.NO. 228 | NP_660295:NM_145252_1-13 | leader sequence | similar to common salivary protein 1 [Homo sapiens] |
| HG1018493 | SEQ.ID.NO. 229 | NP_660295:NM_145252_1-16 | leader sequence | similar to common salivary protein 1 [Homo sapiens] |
| HG1018494 | SEQ.ID.NO. 230 | NP_660295:NM_145252_1-14 | leader sequence | similar to common salivary protein 1 [Homo sapiens] |
| HG1018495 | SEQ.ID.NO. 231 | NP_660295:NM_145252_1-17 | leader sequence | similar to common salivary protein 1 [Homo sapiens] |
| HG1018496 | SEQ.ID.NO. 232 | NP_689534:NM_152321 | full length | hypothetical protein FLJ32115 [Homo sapiens] |
| HG1018497 | SEQ.ID.NO. 233 | NP_689534:NM_152321_1-25 | HMM_SP leader sequence | hypothetical protein FLJ32115 [Homo sapiens] |
| HG1018498 | SEQ.ID.NO. 234 | NP_689534:NM_152321_1-21 | leader sequence | hypothetical protein FLJ32115 [Homo sapiens] |
| HG1018499 | SEQ.ID.NO. 235 | NP_689848:NM_152635 | full length | oncoprotein-induced transcript 3 [Homo sapiens] |
| HG1018500 | SEQ.ID.NO. 236 | NP_689848:NM_152635_1-18 | HMM SP leader sequence | oncoprotein-induced transcript 3 [Homo sapiens] |
| HG1018501 | SEQ.ID.NO. 237 | NP_689848:NM_152635_1-16 | leader sequence | oncoprotein-induced transcript 3 [Homo sapiens] |
| HG1018502 | SEQ.ID.NO. 238 | NP_689848:NM_152635_1-15 | leader sequence | oncoprotein-induced transcript 3 [Homo sapiens] |
| HG1018503 | SEQ.ID.NO. 239 | NP_689968:NM_152755 | full length | hypothetical protein MGC40499 [Homo sapiens] |
| HG1018504 | SEQ.ID.NO. 240 | NP_689968:NM_152755 1-21 | HMM SP leader sequence | hypothetical protein MGC40499 [Homo sapiens] |
| HG1018505 | SEQ.IO.NO. 241 | NP_766630:NM_173042 | full length | interleukin 18 binding protein isoform A precursor [Homo sapiens] |
| HG1018506 | SEQ.ID.NO. 242 | NP_766630:NM_173042_1-29 | HMM_SP leader sequence | interleukin 18 binding protein isoform A precursor [Homo sapiens] |
| HG1018507 | SEQ.ID.NO. 243 | NP_766630:NM_173042_1-24 | leader sequence | interleukin 18 binding protein isoform A precursor [Homo sapiens] |
| HG1018508 | SEQ.ID.NO. 244 | NP_766630:NM_173042_1-28 | leader sequence | interleukin 18 binding protein isoform A precursor [Homo sapiens] |
| HG1018509 | SEQ.ID.NO. 245 | NP_776214:NM_173842 | full length | interleukin 1 receptor antagonist isoform 1 [Homo |
| HG1018510 | SEQ.ID.NO. 246 | NP_776214:NM_173842_1-23 | HMM_SP leader sequence | precursor sapiens] interleukin 1 receptor antagonist isoform 1 [Homo |
| HG1018511 | SEQ.ID.NO. 247 | NP_776214:NM_173842_1-25 | leadersequence | precursor sapiens] interleukin 1 receptor antagonist isoform 1 precursor [Homo sapiens]. |
| HG1018512 | SEQ.ID.NO. 248 | NP_783165:NM_175575 | full length | WFIKKN2 protein [Homo sapiens] |
| HG1018513 | SEQ.ID.NO. 249 | NP_783165:NM_175575 1-32 | HMM_SP leader sequence | WFIKKN2 protein [Homo sapiens] |
| HG1018514 | SEQ.ID.NO. 250 | NP_783165:NM_175575 1-34 | leader sequence | WFIKKN2 protein [Homo sapiens] |
| HG1018515 | SEQ.ID.NO. 251 | NP_783165:NM_175575_1-29 | leader sequence | WFIKKN2 protein [Homo sapiens] |
| HG1018516 | SEQ.ID.NO. 252 | NP_783165:NM_175575_1-30 | leader sequence | WFIKKN2 protein [Homo sapiens] |
| HG1018517 | SEQ.ID.NO. 253 | NP_783165:NM_175575_1 -27 | leader sequence | WFIKKN2 protein [Homo sapiens] |
| HG1018856 | SEQ.ID.NO. 254 | 27482680:27482679 | full length | similar to hypothetical protein 9330140G23 [Homo sapiens] |
| HG1018857 | SEQ.ID.NO. 255 | 27482680:27482679_1-26 | HMM_ SP leader sequence | similar to hypothetical protein 9330140G23 [Homo sapiens] |
| HG1018858 | SEQ.ID.NO. 256 | 27482680:27482679 1-24 | leader sequence | similar to hypothetical protein 9330140G23 [Homo sapiens] |

**Table 3**

| Clone ID | Coomassle mg/ml | Highest Expressor (1=high, 56=low) | Band Detected by Silver Staining | Daltons | Gel Lane | Internal Designation (Secretable Protein) | Protein ID | FP ID | Source ID | Secretable Protein |
|---|---|---|---|---|---|---|---|---|---|---|
| CLN00441787 | 0 | 39 | Yes | 46720 | Gel 1_01 | serine (or cysteine) proteinase Inhibitor, clade A (alpha-1) | NP_000286 | HG1018321 | NP_000266:NM_000295 | serine (or cysteine) proteinase inhibitor, clade A (alpha-1) |
| CLN00441737 | 0 | 53 | No | 47897 | 1_02 | kininogen | NP_000884 | HG1018361 | NP_000884:NM_000893 | kininogen 1 [Homo sapiens] |
| CLN00441827 | 0 | 47 | No | 35770 | Gel 1_03 | spondin 2, extracellular matrix protein | NP_036577 | HG1018455 | NP_036577:NM_012445 | spondin 2, extracellular matrix protein [Homo sapiens] |
| CLN00517648 | 4 | 20 | Yes | 35507 | Get 1_04 | collagen type IX, alpha I | 15929966 | HG1018290 | 15929966:15929965 | COL9A1 protein [Homo sapiens] |
| CLN00517790 | 32 | 1 | Yes | 57113 | Gel 1_05 | pro-collagen proline, 2-oxoglutarate 4-dioxygenase (proline) | NP_000909 | HG1018366 | NP_000909:NM_000918 | prolyl 4-hydroxylase, beta subunit [Homo sapiens] |
| CLN00523549 | 4 | 16 | Yes | 30478 | Gel 1_06 | hypothetical protein FLJ32115 | NP_689534 | HG1018496 | NP_689534:NM_152321 | hypothetical protein FW32115 [Homo sapiens] |
| CLN00528299 | 0 | 33 | No | 47459 | Gel 1_07 | leukocyte immunoglobulin-like receptor, subfamily A (without TM) | NP_006856 | HG1018453 | NP_006856:NM_006865 | leukocyte immunoglobulin-like receptor, subfamily A (without TM) |
| CLN00535083 | 0 | 50 | No | 10498 | Gel 1_08 | secretoglobin, family 2A, member 2 | NP_002402 | HG1018409 | NP_002402:MM_002411 | secretoglobin, family 2A, member 2 [Homo sapiens] |
| CLN00535396 | 0 | 27 | No | 16510 | Gel 1_09 | cystatin E/M | NP_001314 | HG1018384 | NP_001314:NM_001323 | cystatin M precursor [Homo sapiens] |
| CLN00535143 | 15 | 5 | Yes | 20054 | Gel 1_10 | interleukin I receptor antagonist | NP_776214 | HG1018509 | NP_776214:NM_173842 | interleukin 1 receptor antagonis isoform 1 precursor [Homo sapiens] |
| CLN00535158 | 16 | 3 | Yes | 36874 | Gel 1_11 | reticulocalbin 2, EF-hand calcium binding domain | NP_002893 | HG1018423 | NP_002893:NM_002902 | reticulocalbin 2, EF-hand calcium binding domain [Homo sapiens] |
| CLN00535164 | 8 | 11 | Yes | 33841 | Gel 1_12 | secreted phosphoprotein 1 (osteopontin, bone sialoprotein 1, early) | NP_000573 | HG1018339 | NP_000573:NM_000582 | secreted phosphoprotein 1 (osteopontin, bone sialoprotein I, early) |
| CLN00535348 | 10 | 7 | Yes | 38888 | Gel 1_13 | reliculocalbin, EF-hand calcium binding domain | NP_002892 | HG1018417 | NP_002892:NM_002901 | reticulocalbin 1 precursor [Homo sapiens] |
| CLN00535063 | 8 | 10 | Yes | 38288 | Gel 1_14 | dickkopf homolog 3 (Xenopus laevis) | NP_056965 | HG1018473 | NP_056965:NM_015881 | dickkopf homolog 3 [Homo sapiens] |
| CLN00546486 | 0 | 46 | No | 54562 | Gel 2_01 | serine (or cysteine) protease inhibitor, clade F (alpha-2) | 112907 | HG1018267 | 112907:21594845 | Alpha-2-antiplasmin precursor (Alpha-2-plasmin inhibitor) |
| CLN00547185 | 0 | 48 | Yes | 21696 | Gel 2_02 | interleukin 1B binding protein | NP_005690 | HG1018505 | NP_66630:NM_173042 | Interleukin 18 binding protein isoform A precursor [Homo sapiens] |
| CLN00547321 | 0 | 44 | Yes | 20801 | Gel 2_03 | GM2 ganglioside activator protein | NP_000396 | HG1018326 | NP_000396:NM_000405 | GM2 ganglioside activator precursor [Homo sapiens] |
| CLN00547449 | 0 | 31 | Yes | 19407 | Gel 2_04 | neuroblastoma, suppression of tumorigenicity 1 | 16356651 | HG1018292 | 16356651:16356650 | NBL1 [Homo sapiens] |
| CLN00547246 | 8 | 13 | Yes | 21027 | Gel 2_05 | prostaglandin D2 synthase 21 kDa (brain) | NP_000945 | HG1018371 | NP_000945:NM_000954 | prostaglandin D2 synthase 21kDa [Homo sapiens] |
| CLN00547343 | 10 | 6 | Yes | 20651 | Gel 2_06 | transmembrane protein 4 | NP_055070 | HG1018460 | NP_055070:NM_014255 | transmembrane protein 4 [Homo sapiens] |
| CLN00551143 | 2 | 25 | Yes | 23717 | Gel 2_07 | Interleukin 6 (interferon, beta 2) | NP_000591 | HG1018346 | NP_000591:NM_000800 | interleukin 6 (interferon, beta 2) [Homo sapiens] |
| CLN00561179 | 0 | 51 | No | 51673 | Gel 2_08 | hemopaxin | NP_000604 | HG1018352 | NP_000604:NM_000613 | hemopexin [Homo sapiens] |
| CLN00580797 | 6 | 15 | Yes | 31975 | Gel 2_09 | cell growth regulator with EF hand domain 1 | NP_006560 | HG1018449 | NP:006560:NM_006569 | cell growth regulator with EF hand domain 1 [Homo sapiens] |
| CLN00581051 | 15 | 4 | Yes | 37133 | 2_10 | calumenin | 14718453 | HG1018286 | 14718453:14718452 | calumenin [Homo sapiens] |
| CLN00580821 | 6 | 14 | Yes | 50685 | Gel 2_11 | chromogranin A (parathyroid secretory protein 1) | NP_001266 | HG1018380 | NP_001266:NM_001275 | chromogranin A [Homo sapiens] |
| CLN00603545 | 0 | 37 | Yes | 20962 | Gel 2_12 | similar to ARMET protein precursor (Arginine-rich protein) | | | | |
| CLN00604186 | 4 | 18 | Yes | 20963 | Gel 2_13 | proapoptotic caapase adaptor protein | 18204192 | HG1018295 | 18204192:18204191 | PACAP protein [Homo sapiens] |
| CLN00604306 | 8 | 12 | Yes | 22663 | Gel 2_14 | lipocalin 2 (oncogene 24p3) | NP_005555 | HG1018441 | NP_005555:NM_005564 | lipocalin 2 (oncogene 24p3) [Homo sapiens] |
| CLN00604193 | 4 | 19 | Yes | 23510 | Gel 3_01 | orosomucoid 1 | NP_000598 | HG1018350 | NP_000598:NM_000607 | orosomucoid 1 precursor [Homo sapiens] |
| CLN00604144 | 0 | 32 | No | 17737 | Gel 3_02 | chorionic gonadotropin, beta polypeptide 7 | NP_149439 | HG1018482 | NP_149439:NM_033183 | chorionic gonadotropin, beta polypeptide 8 precursor [Homo sapiens] |
| CLN00604170 | 2 | 26 | Yes | 13074 | Gel 3_03 | glycoprotein hormones, alpha polypeptide | NP_000726 | HG1018358 | NP_000726:NM_000735 | glycoprotein homones, alpha polypeptide precursor [Homo sapiens] |
| CLN00622839 | 0 | 34 | No | 18878 | 3_04 | salivary protein 1 | NP_660295 | HG1018491 | NP_660295:NM_145252 | [Homo sapiens] |
| CLN00622803 | 4 | 17 | Yes | 38426 | Gel 3_05 | lumican | NP_002336 | HG1018404 | NP_002336:NM_002345 | lumican [Homo sapiens] |
| CLN00622755 | 0 | 35 | No | 29422 | Gel 3_06 | proopiomelanocortin (adrenocorticotropin/beta-lipotropin) | NP_000930 | HG1018368 | NP_000930:NM_000939 | proopiomelanocortin [Homo sapiens] |
| CLN00622763 | 0 | 41 | No | 39159 | Gel 3_07 | nephroblastoma overexpressed gene | NP_002502 | HG1018411 | NP_002505:NM_002514 | nov precursor [Homo sapiens] |
| CLN00622719 | 8 | 8 | Yes | 53046 | Gel 3_08 | group-specific component (vitamin D binding protein) | NP_000574 | HG1018343 | NP_000574:NM_000583 | vitamin D-binding protein precursor [Homo sapiens] |
| CLN00622726 | 20 | 2 | Yes | 34257 | Gel 3_09 | alpha-2-glycoprotein 1, zinc | NP_001176 | HG1018375 | NP_001176:NM_001185 | alpha-2-glycoprotein 1, zinc [Homo sapiens] |
| CLN00624913 | 0 | 40 | No | 20865 | Gel 3_10 | interleukin 18 binding protein | NP_766630 | HG1018445 | NP_005690:NM_005699 | interleukin 18 binding protein isoform C precursor [Homo sapiens] |
| CLN00625401 | 0 | 43 | No | 56793 | Gel 3_11 | glucose regulated protein, 58 kDa | 7437388 | HG1018318 | 7437388:1208426 | protein disulfide-isomerase (EC 5341) ER60 precursor human |
| CLN00649118 | 0 | 30 | Yes | 22006 | Gel 3_12 | leukemia inhibitory factor (cholinergic diffemeliation factor) | NP_002300 | HG1018401 | NP_002300:NM_002309 | leukocyte immunoglobulin-like receptor, subfamily A (without TM) |
| CLN00649021 | 0 | 45 | No | 30746 | Gel 3_13 | trinucleotide repeat containing 5 | 13325208 | HG1018273 | 13325208:13325207 | Trinucleotide repeat containing 5 [Homo sapiens] |
| CLN00649291 | 0 | 36 | No | 30082 | Gel 3_14 | cerberus 1 homolog, cysteine knot superfamily (Xenopus laevis) | NP_005445 | HG1018439 | NP_005445:NM_005454 | cerberus 1 [Homo sapiens] |
| CLN00658769 | 2 | 24 | Yes | 16389 | Gel 4_01 | multiple coagulation factor deviciency 2 | NP_644808 | HG1018486 | NP_644808:NM_139279 | multiple coagulation factor deficiency 2 [Homo sapiens] |
| CLN00658997 | 4 | 21 | Yes | 52491 | Gel 4_02 | clusterine (complement lysis inhibitor, SP-40, 40, sulfated) | NP_001822 | HG1018391 | NP_001822:NM001831 | clusterin isoform 1 [Homo sapiens] |
| CLN00658649 | 0 | 28 | Yes | 20699 | Gel 4_03 | arginine-rich, mutated in early stage tumors | 13938307 | HG1018283 | 13938307:13938306 | ARMET protein [Homo sapiens] |
| CLN00649094 | 8 | 9 | Yes | 101396 | Gel 4_04 | inter-alpha (globulin) inhibitor, H1 polypeptide | NP_002206 | HG1018395 | NP_002206:NM_002215 | inter-alpha (globulin) inhibitor H1 [Homo sapiens] |
| CLN00649247 | 2 | 23 | Yes | 28308 | Gel 4_05 | hypothetical protein MGC40499 | NP_689968 | HG1018503 | NP_689968:NM_152755 | hypothetical protein MGC40499 [Homo sapiens] |
| CLN00439078 | 0 | 49 | Yes | 45422 | Gel 4_06 | gastric intrinsic factor (vitamin B synthesis) | NP_005133 | HG1018431 | NP_005133:NM_005142 | gastric intrinsic factor (vitamin B synthesis) [Homo sapiens] |
| CLN00438878 | 0 | 52 | No | 72426 | Gel 4_07 | Rho GTPase activating protein 25 | NP_055697 | HG1018471 | NP_055697:NM_014882 | Rho GTPase activating protein 25 isoform b [Homo sapiens] |
| CLN00438933 | not tested | 54 | not tested | 63902 | Gel 4_08 | similar to Brain-specific angiogenesis inhibitor 2 precursor | 27479535 | HG1018305 | 27479535:27479534 | similar to Brain-specific angiogenesis inhibitor 2 precursor [Homo sapiens] |
| CLN00463475 | 2 | 22 | Yes | 54206 | Gel 4_09 | alpha-1-B glycoprotein | 23503038 | HG1018301 | 23503038:15778555 | Alpha-1B-glycoprotein precursor (Alpha-1-B glycoprotein) |
| CLN00463575 | 0 | 42 | No | 48280 | Gel 4_10 | ameloblasfin, enamel matrix protein | NP_057603 | HG1018479 | NP_057603:NM_016519 | ameloblastin precursor [Homo sapiens] |
| CLN00463328 | 0 | 38 | No | 54728 | Gel 4_11 | coagulation factor X | NP_000495 | HG1018332 | NP_000495:NM_000504 | coagulation factor X precursor [Homo sapiens] |
| CLN00463625 | 0 | 29 | No | 46826 | Gel 4_12 | sparc/osteoonectin, cwcv and kazal-like domains proteinglycan | 37182960 | HG1018311 | 37182960:37182959 | SPOCK2 [Homo sapiens] |
| CLN00463338 | not tested | 55 | not tested | 60017 | Gel 4_13 | oncoprotein induced transcript 3 | NP_689848 | HG1018499 | NP_689848:NM_152635 | oncoprotein-induced transcript 3 [Homo sapiens] |
| CLN00463474 | not tested | 56 | not tested | 63936 | Gel 4_14 | WFIKKN-related protein | NP_783165 | HG1018512 | NP_783165:NM_175575 | WFIKKN2 protein [Homo sapiens] |

SEQ.ID.NO. 1      HG1018265P1 collagen_leader_seq
MKTCWKIPVFFFVCSFLEPWASA

SEQ.ID.NO. 2   .  HG1018266P1 CLN00517648
MKTCWKIPVFFFVCSFLEPWASAAVKRRPRFPVNSNSNGGNELCPKIRIGQDDLPGFDLI
SQFQVDKAASRRAIQRVVGSATLQVAYKLGNNVDFRIPTRNLYPSGLPEEYSFLTTFRMT
GSTLKKNWNIWQIQDSSGKEQVGIKINGQTQSVVFSYKGLDGSLQTAAFSNLSSLFDSQW
HKIMIGVERSSATLFVDCNRIESLPIKPRGPIDIDGFAVLGKLADNPQVSVPFELQWMLI
HCDPLRPRRETCHELPARITPSQTTDERGPPGEQGPPGPPGPPGVPGIDGIDGDRGPKGP
PGPPGPAGEPGKPGAPGKPGTPGADTSPYPAFLYKVVIEGKPIPNPLLGLDSTRTGHHHH
HHHHGGQ

SEQ.ID.NO. 3      HG1018267P1 112907:21594845
MALLWGLLVLSWSCLQGPCSVFSPVSAMEPLGRQLTSGPNQEQVSPLTLLKLGNQEPGGQ
TALKSPPGVCSRDPTPEQTHRLARAMMAFTADLFSLVAQTSTCPNLILSPLSVALALSHL
ALGAQNHTLQRLQQVLHAGSGPCLPHLLSRLCQDLGPGAFRLAARMYLQKGFPIKEDFLE
QSEQLFGAKPVSLTGKQEDDLANINQWVKEATEGKIQEFLSGLPEDTVLLLLNAIHFQGF
WRNKFDPSLTQRDSFHLDEQFTVPVEMMQARTYPLRWFLLEQPEIQVAHFPFKNNMSFVV
LVPTHFEWNVSQVLANLSWDTLHPPLVWERPTKVRLPKLYLKHQMDLVATLSQLGLQELF
QAPDLRGISEQSLVVSGVQHQSTLELSEVGVEAAAATSIAMSRMSLSSFSVNRPFLFFIF
EDTTGLPLFVGSVRNPNPSAPRELKEQQDSPGNKDFLQSLKGFPRGDKLFGPDLKLVPPM
EEDYPQFGSPK

SEQ.ID.NO. 4      HG1018268P1 112907:21594845_1-17
MALLWGLLVLSWSCLQG

SEQ.ID.NO. 5      HG1018269P1 112907:21594845_1-13
MALLWGLLVLSWS

SEQ.ID.NO. 6      HG1018270P1 112907:21594845_1-19
MALLWGLLVLSWSCLQGPC

SEQ.ID.NO. 7      HG1018271P1 112907:21594845_1-16
MALLWGLLVLSWSCLQ

SEQ.ID.NO. 8      HG1018272P1 112907:21594845_1-15
MALLWGLLVLSWSCL

SEQ.ID.NO. 9      HG1018273P1 13325208:13325207
MDSMPEPASRCLLLLPLLLLLLLLPAPELGPSQAGAEENDWVRLPSKCEVCKYVAVELK
SAFEETGKTKEVIGTGYGILDQKASGVKYTKSDLRLIEVTETICKRLLDYSLHKERTGSN
RFAKGMSETFETLHNLVHKGVKVVMDIPYELWNETSAEVADLKKQCDVLVEEFEEVIEDW
YRNHQEEDLTEFLCANHVLKGKDTSCLAEQWSGKKGDTAALGGKKSKKKSSRAKAAGGRS
SSSKQRKELGGLEGDPSPEEDEGIQKASPLTHSPPDEL

SEQ.ID.NO. 10     HG1018274P1 13325208:13325207_1-30
MDSMPEPASRCLLLLPLLLLLLLLPAPEL

SEQ.ID.NO. 11     HG1018275P1 13325208:13325207_1-25
MDSMPEPASRCLLLLPLLLLLLLL

SEQ.ID.NO. 12     HG1018276P1 13325208:13325207_1-33
MDSMPEPASRCLLLLPLLLLLLLLPAPELGPS

SEQ.ID.NO. 13     HG1018277P1 13325208:13325207_1-24
MDSMPEPASRCLLLLPLLLLLLL

SEQ.ID.NO. 14     HG1018278P1 13325208:13325207_1-26
MDSMPEPASRCLLLLPLLLLLLLLP

SEQ.ID.NO. 15     HG1018279P1 13325208:13325207_1-32
MDSMPEPASRCLLLLPLLLLLLLLPAPELGP

SEQ.ID.NO. 16     HG1018280P1 13325208:13325207_1-27
MDSMPEPASRCLLLLPLLLLLLLLPA

SEQ.ID.NO. 17     HG1018281P1 13325208:13325207_1-23
MDSMPEPASRCLLLLPLLLLLLL

SEQ.ID.NO. 18     HG1018282P1 13325208:13325207_1-35
MDSMPEPASRCLLLLPLLLLLLLLPAPELGPSQA

SEQ.ID.NO. 19     HG1018283P1 13938307:13938306
MRRMWATQGLAVALALSVLPGSRALRPGDCEVCISYLGRFYQDLKDRDVTFSPATIENEL
IKFCREARGKENRLCYYIGATDDAATKIINEVSKPLAHHIPVEKICEKLKKKDSQICELK
YDKQIDLSTVDLKKLRVKELKKILDDWGETCKGCAEKSDYIRKINELMPKYAPKAASART
DL

SEQ.ID.NO. 20     HG1018284P1 13938307:13938306_1-24
MRRMWATQGLAVALALSVLPGSRA

SEQ.ID.NO. 21     HG1018285P1 13938307:13938306_1-21
MRRMWATQGLAVALALSVLPG

SEQ.ID.NO. 22     HG1018286P1 14718453:14718452
MDLRQFLMCLSLCTAFALSKPTEKKDRVHHEPQLSDKVHNDAQSFDYDHDAFLGAEEAKT
FDQLTPEESKERLGMIVDKIDADKDGFVTEGELKSWIKHAQKKYIYDNVENQWQEFDMNQ
DGLISWDEYRNVTYGTYLDDPDPDDGFNYKQMMVRDERRFKMADKDGDLIATKEEFTAFL

HPEEYDYMKDIVVQETMEDIDKNADGFIDLEEYIGDMYSHDGNTDEPEWVKTEREQFVEF
RDKNRDGKMDKEETKDWILPSDYDHAEAEARHLVYESDQNKDGKLTKEEIVDKYDLFVGS
QATDFGEALVRHDEF

SEQ.ID.NO. 23     HG1018287P1 14718453:14718452_1-19
MDLRQFLMCLSLCTAFALS

SEQ.ID.NO. 24     HG1018288P1 14718453:14718452_1-15
MDLRQFLMCLSLCTA

SEQ.ID.NO. 25     HG1018289P1 14718453:14718452_1-17
MDLRQFLMCLSLCTAFA

SEQ.ID.NO. 26     HG1018290P1 15929966:15929965
MKTCWKIPVFFFVCSFLEPWASAAVKRRPRFPVNSNSNGGNELCPKIRIGQDDLPGFDLI
SQFQVDKAASRRAIQRVVGSATLQVAYKLGNNVDFRIPTRNLYPSGLPEEYSFLTTFRMT
GSTLKKNWNIWQIQDSSGKEQVGIKINGQTQSVVFSYKGLDGSLQTAAFSNLSSLFDSQW
HKIMIGVERSSATLFVDCNRIESLPIKPRGPIDIDGFAVLGKLADNPQVSVPFELQWMLI
HCDPLRPRRETCHELPARITPSQTTDERGPPGEQGPPGPPGPPGVPGIDGIDGDRGPKGP
PGPPGPAGEPGKPGAPGKPGTPGADTSP

SEQ.ID.NO. 27     HG1018291P1 15929966:15929965_1-23
MKTCWKIPVFFFVCSFLEPWASA

SEQ.ID.NO. 28     HG1018292P1 16356651:16356650
MMLRVLVGAVLPAMLLAAPPPINKLALFPDKSAWCEAKNITQIVGHSGCEAKSIQNRACL
GQCFSYSVPNTFPQSTESLVHCDSCMPAQSMWEIVTLECPGHEEVPRVDKLVEKILHCSC
QACGKEPSHEGLSVYVQGEDGPGSQPGTHPHPHPHPHPGGQTPEPEDPPGAPHTEEEGAE
D

SEQ.ID.NO. 29     HG1018293P1 16356651:16356650_1-21
MMLRVLVGAVLPAMLLAAPPP

SEQ.ID.NO. 30     HG1018294P1 16356651:16356650_1-17
MMLRVLVGAVLPAMLLA

SEQ.ID.NO. 31     HG1018295P1 18204192:18204191
MRLSLPLLLLLLGAWAIPGGLGDRAPLTATAPQLDDEEMYSAHMPAHLRCDACRAVAYQM
WQNLAKAETKLHTSNSGGRRELSELVYTDVLDRSCSRNWQDYGVREVDQVKRLTGPGLSE
GPEPSISVMVTGGPWPTRLSRTCLHYLGEFGEDQIYEAHQQGRGALEALLCGGPQGACSE
KVSATREEL

SEQ.ID.NO. 32     HG1018296P1 18204192:18204191_1-19
MRLSLPLLLLLLGAWAIPG

SEQ.ID.NO. 33 HG1018297P1 18204192:18204191_1-22
MRLSLPLLLLLLGAWAIPGGLG

SEQ.ID.NO. 34 HG1018298P1 18204192:18204191_1-18
MRLSLPLLLLLLGAWAIP

SEQ.ID.NO. 35 HG1018299P1 18204192:18204191_1-16
MRLSLPLLLLLLGAWA

SEQ.ID.NO. 36 HG1018300P1 18204192:18204191_1-14
MRLSLPLLLLLLGA

SEQ.ID.NO. 37 HG1018301P1 23503038:15778555










SEQ.ID.NO. 38 HG1018302P1 23503038:15778555_1-20
MSMLVVFLLLWGVTWGPVTE

SEQ.ID.NO. 39 HG1018303P1 23503038:15778555_1-16
MSMLVVFLLLWGVTWG

SEQ.ID.NO. 40 HG1018304P1 23503038:15778555_1-21
MSMLVVFLLLWGVTWGPVTEA

SEQ.ID.NO. 41 HG1018305P1 27479535:27479534
MRALRDRAGLLLCVLLLAALLEAALGLPVKKPRLRGPRPGSLTRLAEVSASPDPRPLKEE
EEAPLLPRTHLQAEPHQHGCWTVTEPAAMTPGNATPPRTPEVTPLRLELQKLPGLANTTL
STPNPDTQASASPDPRPLREEEEARLLPRTHLQAELHQHGCWTVTEPAALTPGNATPPRT
QEVTPLLLELQKLPELVHATLSTPNPDNQVTIKVVEDPQAEVSIDLLAEPSNPPPQDTLS
WLPALWSFLWGDYKGEEKDRAPGEKGEEKEEDEDYPSEDIEGEDQEDKEEDEEEQALWFN
GTTDNWDQGWLAPGDWVFKDSVSYDYEPQKEWSPWSPCSGNCSTGKQQRTRPCGYGCTAT
ETRTCDLPSCPGTEDKDTLGLPSEEWKLLARNATDMHDQDVDSCEKWLNCKSDFLIKYLS
QMLRDLPSCPCAYPLEAMDSPVSLQDEHQGRSFRWRDASGPRERLDIYQPTARFCLRSML
SGESSTLAAQHCCYDEDSRLLTRGKGAGMPNLISTDFSPKLHFKFDTTPWILCKGDWSRL
HAVLPPNNGRACTDNPLEEEYLAQLQEAKEY

SEQ.ID.NO. 42     HG1018306P1 27479535:27479534_1-24
MRALRDRAGLLLCVLLLAALLEAA

SEQ.ID.NO. 43     HG1018307P1 27479535:27479534_1-20
MRALRDRAGLLLCVLLLAAL

SEQ.ID.NO. 44     HG1018308P1 27479535:27479534_1-26
MRALRDRAGLLLCVLLLAALLEAALG

SEQ.ID.NO. 45     HG1018309P1 27479535:27479534_1-21
MRALRDRAGLLLCVLLLAALL

SEQ.ID.NO. 46     HG1018310P1 27479535:27479534_1-23
MRALRDRAGLLLCVLLLAALLEA

SEQ.ID.NO. 47     HG1018311P1 37182960:37182959









SEQ.ID.NO. 48     HG1018312P1 37182960:37182959_1-24
MRAPGCGRLVLPLLLLAAAALAEG

SEQ.ID.NO. 49     HG1018313P1 37182960:37182959_1-19
MRAPGCGRLVLPLLLLAAA

SEQ.ID.NO. 50     HG1018314P1 37182960:37182959_1-22
MRAPGCGRLVLPLLLLAAAALA

SEQ.ID.NO. 51     HG1018315P1 37182960:37182959_1-20
MRAPGCGRLVLPLLLLAAAA

SEQ.ID.NO. 52     HG1018316P1 37182960:37182959_1-26
MRAPGCGRLVLPLLLLAAAALAEGDA

SEQ.ID.NO. 53     HG1018317P1 37182960:37182959_1-21
MRAPGCGRLVLPLLLLAAAAL

SEQ.ID.NO. 54      HG1018318P1 7437388:1208426
MRLRRLALFPGVALLLAAGRLVAASDVLELTDDNFESRISDTGSAGLMLVEFFAPWCGHC
KRLAPEYEAAATRLKGIVPLAKVDCTANTNTCNKYGVSGYPTLKIFRDGEEAGAYDGPRT
ADGIVSHLKKQAGPASVPLRTEEEFKKFISDKDASIVGFFDDSFSEAHSEFLKAASNLRD
NYRFAHTNVESLVNEYDDNGEGIILFRPSHLTNKFEDKTVAYTEQKMTSGKIKKFIQENI
FGICPHMTEDNKDLIQGKDLLIAYYDVDYEKNAKGSNYWRNRVMMVAKKFLDAGHKLNFA
VASRKTFSHELSDFGLESTAGEIPVVAIRTAKGEKFVMQEEFSRDGKALERFLQDYFDGN
LKRYLKSEPIPESNDGPVKVVVAENFDEIVNNENKDVLIEFYAPWCGHCKNLEPKYKELG
EKLSKDPNIVIAKMDATANDVPSPYEVRGFPTIYFSPANKKLNPKKYEGGRELSDFISYL
QREATNPPVIQEEKPKKKKKAQEDL

SEQ.ID.NO. 55      HG1018319P1 7437388:1208426_1-24
MRLRRLALFPGVALLLAAGRLVAA

SEQ.ID.NO. 56      HG1018320P1 7437388:1208426_1-23
MRLRRLALFPGVALLLAAGRLVA

SEQ.ID.NO. 57      HG1018321P1 NP_000286:NM_000295
MPSSVSWGILLLAGLCCLVPVSLAEDPQGDAAQKTDTSHHDQDHPTFNKITPNLAEFAFS
LYRQLAHQSNSTNIFFSPVSIATAFAMLSLGTKADTHDEILEGLNFNLTEIPEAQIHEGF
QELLRTLNQPDSQLQLTTGNGLFLSEGLKLVDKFLEDVKKLYHSEAFTVNFGDTEEAKKQ
INDYVEKGTQGKIVDLVKELDRDTVFALVNYIFFKGKWERPFEVKDTEEEDFHVDQVTTV
KVPMMKRLGMFNIQHCKKLSSWVLLMKYLGNATAIFFLPDEGKLQHLENELTHDIITKFL
ENEDRRSASLHLPKLSITGTYDLKSVLGQLGITKVFSNGADLSGVTEEAPLKLSKAVHKA
VLTIDEKGTEAAGAMFLEAIPMSIPPEVKFNKPFVFLMIDQNTKSPLFMGKVVNPTQK

SEQ.ID.NO. 58      HG1018322P1 NP_000286:NM_000295_1-24
MPSSVSWGILLLAGLCCLVPVSLA

SEQ.ID.NO. 59      HG1018323P1 NP_000286:NM_000295_1-18
MPSSVSWGILLLAGLCCL

SEQ.ID.NO. 60      HG1018324P1 NP_000286:NM_000295_1-23
MPSSVSWGILLLAGLCCLVPVSL

SEQ.ID.NO. 61      HG1018325P1 NP_000286:NM_000295_1-17
MPSSVSWGILLLAGLCC

SEQ.ID.NO. 62      HG1018326P1 NP_000396:NM_000405
MQSLMQAPLLIALGLLLATPAQAHLKKPSQLSSFSWDNCDEGKDPAVIRSLTLEPDPIVV
PGNVTLSVVGSTSVPLSSPLKVDLVLEKEVAGLWIKIPCTDYIGSCTFEHFCDVLDMLIP
TGEPCPEPLRTYGLPCHCPFKEGTYSLPKSEFVVPDLELPSWLTTGNYRIESVLSSSGKR
LGCIKIAASLKGI

SEQ.ID.NO. 63     HG1018327P1 NP_000396:NM_000405_1-23
MQSLMQAPLLIALGLLLATPAQA

SEQ.ID.NO. 64     HG1018328P1 NP_000396:NM_000405_1-18
MQSLMQAPLLIALGLLLA

SEQ.ID.NO. 65     HG1018329P1 NP_000396:NM_000405_1-25
MQSLMQAPLLIALGLLLATPAQAHL

SEQ.ID.NO. 66     HG1018330P1 NP_000396:NM_000405_1-20
MQSLMQAPLLIALGLLLATP

SEQ.ID.NO. 67     HG1018331P1 NP_000396:NM_000405_1-21
MQSLMQAPLLIALGLLLATPA

SEQ.ID.NO. 68     HG1018332P1 NP_000495:NM_000504










SEQ.ID.NO. 69     HG1018333P1 NP_000495:NM_000504_1-23
MGRPLHLVLLSASLAGLLLLGES

SEQ.ID.NO. 70     HG1018334P1 NP_000495:NM_000504_1-19
MGRPLHLVLLSASLAGLLL

SEQ.ID.NO. 71     HG1018335P1 NP_000495:NM_000504_1-20
MGRPLHLVLLSASLAGLLLL

SEQ.ID.NO. 72     HG1018336P1 NP_000495:NM_000504_1-15
MGRPLHLVLLSASLA

SEQ.ID.NO. 73     HG1018337P1 NP_000495:NM_000504_1-21
MGRPLHLVLLSASLAGLLLLG

SEQ.ID.NO. 74     HG1018338P1 NP_000495:NM_000504_1-17
MGRPLHLVLLSASLAGL

SEQ.ID.NO. 75 HG1018339P1 NP_000573:NM_000582
MRIAVICFCLLGITCAIPVKQADSGSSEEKQLYNKYPDAVATWLNPDPSQKQNLLAPQTL
PSKSNESHDHMDDMDDEDDDDHVDSQDSIDSNDSDDVDDTDDSHQSDESHHSDESDELVT
DFPTDLPATEVFTPVVPTVDTYDGRGDSVVYGLRSKSKKFRRPDIQYPDATDEDITSHME
SEELNGAYKAIPVAQDLNAPSDWDSRGKDSYETSQLDDQSAETHSHKQSRLYKRKANDES
NEHSDVIDSQELSKVSREFHSHEFHSHEDMLVVDPKSKEEDKHLKFRISHELDSASSEVN

SEQ.ID.NO. 76 HG1018340P1 NP_000573:NM_000582_1-18
MRIAVICFCLLGITCAIP

SEQ.ID.NO. 77 HG1018341P1 NP_000573:NM_000582_1-16
MRIAVICFCLLGITCA

SEQ.ID.NO. 78 HG1018342P1 NP_000573:NM_000582_1-15
MRIAVICFCLLGITC

SEQ.ID.NO. 79 HG1018343P1 NP_000574:NM_000583









SEQ.ID.NO. 80 HG1018344P1 NP_000574:NM_000583_1-16
MKRVLVLLLAVAFGHA

SEQ.ID.NO. 81 HG1018345P1 NP_000574:NM_000583_1-14
MKRVLVLLLAVAFG

SEQ.ID.NO. 82 HG1018346P1 NP_000591:NM_000600
MNSFSTSAFGPVAFSLGLLLVLPAAFPAPVPPGEDSKDVAAPHRQPLTSSERIDKQIRYI
LDGISALRKETCNKSNMCESSKEALAENNLNLPKMAEKDGCFQSGFNEETCLVKIITGLL
EFEVYLEYLQNRFESSEEQARAVQMSTKVLIQFLQKKAKNLDAITTPDPTTNASLLTKLQ
AQNQWLQDMTTHLILRSFKEFLQSSLRALRQM

SEQ.ID.NO. 83 HG1018347P1 NP_000591:NM_000600_1-25
MNSFSTSAFGPVAFSLGLLLVLPAA

SEQ.ID.NO. 84 HG1018348P1 NP_000591:NM_000600_1-24
MNSFSTSAFGPVAFSLGLLLVLPA

SEQ.ID.NO. 85 HG1018349P1 NP_000591:NM_000600_1-27
MNSFSTSAFGPVAFSLGLLLVLPAAFP

SEQ.ID.NO. 86 HG1018350P1 NP_000598:NM_000607
MALSWVLTVLSLLPLLEAQIPLCANLVPVPITNATLDQITGKWFYIASAFRNEEYNKSVQ
EIQATFFYFTPNKTEDTIFLREYQTRQDQCIYNTTYLNVQRENGTISRYVGGQEHFAHLL
ILRDTKTYMLAFDVNDEKNWGLSVYADKPETTKEQLGEFYEALDCLRIPKSDVVYTDWKK
DKCEPLEKQHEKERKQEEGES

SEQ.ID.NO. 87 HG1018351P1 NP_000598:NM_000607_1-18
MALSWVLTVLSLLPLLEA

SEQ.ID.NO. 88 HG1018352P1 NP_000604:NM_000613
MARVLGAPVALGLWSLCWSLAIATPLPPTSAHGNVAEGETKPDPDVTERCSDGWSFDATT
LDDNGTMLFFKGEFVWKSHKWDRELISERWKNFPSPVDAAFRQGHNSVFLIKGDKVWVYP
PEKKEKGYPKLLQDEFPGIPSPLDAAVECHRGECQAEGVLFFQGDREWFWDLATGTMKER
SWPAVGNCSSALRWLGRYYCFQGNQFLRFDPVRGEVPPRYPRDVRDYFMPCPGRGHGHRN
GTGHGNSTHHGPEYMRCSPHLVLSALTSDNHGATYAFSGTHYWRLDTSRDGWHSWPIAHQ
WPQGPSAVDAAFSWEEKLYLVQGTQVYVFLTKGGYTLVSGYPKRLEKEVGTPHGIILDSV
DAAFICPGSSRLHIMAGRRLWWLDLKSGAQATWTELPWPHEKVDGALCMEKSLGPNSCSA
NGPGLYLIHGPNLYCYSDVEKLNAAKALPQPQNVTSLLGCTH

SEQ.ID.NO. 89 HG1018353P1 NP_000604:NM_000613_1-19
MARVLGAPVALGLWSLCWS

SEQ.ID.NO. 90 HG1018354P1 NP_000604:NM_000613_1-25
MARVLGAPVALGLWSLCWSLAIATP

SEQ.ID.NO. 91 HG1018355P1 NP_000604:NM_000613_1-21
MARVLGAPVALGLWSLCWSLA

SEQ.ID.NO. 92 HG1018356P1 NP_000604:NM_000613_1-23
MARVLGAPVALGLWSLCWSLAIA

SEQ.ID.NO. 93 HG1018357P1 NP_000604:NM_000613_1-31
MARVLGAPVALGLWSLCWSLAIATPLPPTSA

SEQ.ID.NO. 94 HG1018358P1 NP_000726:NM_000735
MDYYRKYAAIFLVTLSVFLHVLHSAPDVQDCPECTLQENPFFSQPGAPILQCMGCCFSRA
YPTPLRSKKTMLVQKNVTSESTCCVAKSYNRVTVMGGFKVENHTACHCSTCYYHKS

SEQ.ID.NO. 95 HG1018359P1 NP_000726:NM_000735_1-26
MDYYRKYAAIFLVTLSVFLHVLHSAP

SEQ.ID.NO. 96     HG1018360P1 NP_000726:NM_000735_1-24
MDYYRKYAAIFLVTLSVFLHVLHS

SEQ.ID.NO. 97     HG1018361P1 NP_000884:NM_000893
MKLITILFLCSRLLLSLTQESQSEEIDCNDKDLFKAVDAALKKYNSQNQSNNQFVLYRIT
EATKTVGSDTFYSFKYEIKEGDCPVQSGKTWQDCEYKDAAKAATGECTATVGKRSSTKFS
VATQTCQITPAEGPVVTAQYDCLGCVHPISTQSPDLEPILRHGIQYFNNNTQHSSLFMLN
EVKRAQRQVVAGLNFRITYSIVQTNCSKENFLFLTPDCKSLWNGDTGECTDNAYIDIQLR
IASFSQNCDIYPGKDFVQPPTKICVGCPRDIPTNSPELEETLTHTITKLNAENNATFYFK
IDNVKKARVQVVAGKKYFIDFVARETTCSKESNEELTESCETKKLGQSLDCNAEVYVVPW
EKKIYPTVNCQPLGMISLMKRPPGFSPFRSSRIGEIKEETTSHLRSCEYKGRPPKAGAEP
ASEREVS

SEQ.ID.NO. 98     HG1018362P1 NP_000884:NM_000893_1-18
MKLITILFLCSRLLLSLT

SEQ.ID.NO. 99     HG1018363P1 NP_000884:NM_000893_1-19
MKLITILFLCSRLLLSLTQ

SEQ.ID.NO. 100     HG1018364P1 NP_000884:NM_000893_1-16
MKLITILFLCSRLLLS

SEQ.ID.NO. 101     HG1018365P1 NP_000884:NM_000893_1-23
MKLITILFLCSRLLLSLTQESQS

SEQ.ID.NO. 102     HG1018366P1 NP_000909:NM_000918










SEQ.ID.NO. 103     HG1018367P1 NP_000909:NM_000918_1-17
MLRRALLCLAVAALVRA

SEQ.ID.NO. 104     HG1018368P1 NP_000930:NM_000939
MPRSCCSRSGALLLALLLQASMEVRGWCLESSQCQDLTTESNLLECIRACKPDLSAETPM
FPGNGDEQPLTENPRKYVMGHFRWDRFGRRNSSSSGSSGAGQKREDVSAGEDCGPLPEGG
PEPRSDGAKPGPREGKRSYSMEHFRWGKPVGKKRRPVKVYPNGAEDESAEAFPLEFKREL

TGQRLREGDGPDGPADDGAGAQADLEHSLLVAAEKKDEGPYRMEHFRWGSPPKDKRYGGF
MTSEKSQTPLVTLFKNAIIKNAYKKGE

SEQ.ID.NO. 105     HG1018369P1 NP_000930:NM_000939_1-23
MPRSCCSRSGALLLALLLQASME

SEQ.ID.NO. 106     HG1018370P1 NP_000930:NM_000939_1-26
MPRSCCSRSGALLLALLLQASMEVRG

SEQ.ID.NO. 107     HG1018371P1 NP_000945:NM_000954
MATHHTLWMGLALLGVLGDLQAAPEAQVSVQPNFQQDKFLGRWFSAGLASNSSWLREKKA
ALSMCKSVVAPATDGGLNLTSTFLRKNQCETRTMLLQPAGSLGSYSYRSPHWGSTYSVSV
VETDYDQYALLYSQGSKGPGEDFRMATLYSRTQTPRAELKEKFTAFCKAQGFTEDTIVFL
PQTDKCMTEQ

SEQ.ID.NO. 108     HG1018372P1 NP_000945:NM_000954_1-23
MATHHTLWMGLALLGVLGDLQAA

SEQ.ID.NO. 109     HG1018373P1 NP_000945:NM_000954_1-22
MATHHTLWMGLALLGVLGDLQA

SEQ.ID.NO. 110     HG1018374P1 NP_000945:NM_000954_1-18
MATHHTLWMGLALLGVLG

SEQ.ID.NO. 111     HG1018375P1 NP_001176:NM_001185
MVRMVPVLLSLLLLLGPAVPQENQDGRYSLTYIYTGLSKHVEDVPAFQALGSLNDLQFFR
YNSKDRKSQPMGLWRQVEGMEDWKQDSQLQKAREDIFMETLKDIVEYYNDSNGSHVLQGR
FGCEIENNRSSGAFWKYYYDGKDYIEFNKEIPAWVPFDPAAQITKQKWEAEPVYVQRAKA
YLEEECPATLRKYLKYSKNILDRQDPPSVVVTSHQAPGEKKKLKCLAYDFYPGKIDVHWT
RAGEVQEPELRGDVLHNGNGTYQSWVVVAVPPQDTAPYSCHVQHSSLAQPLVVPWEAS

SEQ.ID.NO. 112     HG1018376P1 NP_001176:NM_001185_1-18
MVRMVPVLLSLLLLLGPA

SEQ.ID.NO. 113     HG1018377P1 NP_001176:NM_001185_1-20
MVRMVPVLLSLLLLLGPAVP

SEQ.ID.NO. 114     HG1018378P1 NP_001176:NM_001185_1-21
MVRMVPVLLSLLLLLGPAVPQ

SEQ.ID.NO. 115     HG1018379P1 NP_001176:NM_001185_1-17
MVRMVPVLLSLLLLLGP

SEQ.ID.NO. 116     HG1018380P1 NP_001266:NM_001275

MRSAAVLALLLCAGQVTALPVNSPMNKGDTEVMKCIVEVISDTLSKPSPMPVSQECFETL
RGDERILSILRHQNLLKELQDLALQGAKERAHQQKKHSGFEDELSEVLENQSSQAELKEA
VEEPSSKDVMEKREDSKEAEKSGEATDGARPQALPEPMQESKAEGNNQAPGEEEEEEEEA
TNTHPPASLPSQKYPGPQAEGDSEGLSQGLVDREKGLSAEPGWQAKREEEEEEEEEAEAG
EEAVPEEEGPTVVLNPHPSLGYKEIRKGESRSEALAVDGAGKPGAEEAQDPEGKGEQEHS
QQKEEEEEMAVVPQGLFRGGKSGELEQEEERLSKEWEDSKRWSKMDQLAKELTAEKRLEG
QEEEEDNRDSSMKLSFRARAYGFRGPGPQLRRGWRPSSREDSLEAGLPLQVRGYPEEKKE
EEGSANRRPEDQELESLSAIEAELEKVAHQLQALRRG

SEQ.ID.NO. 117    HG1018381P1 NP_001266:NM_001275_1-18
MRSAAVLALLLCAGQVTA

SEQ.ID.NO. 118    HG1018382P1 NP_001266:NM_001275_1-15
MRSAAVLALLLCAGQ

SEQ.ID.NO. 119    HG1018383P1 NP_001266:NM_001275_1-14
MRSAAVLALLLCAG

SEQ.ID.NO. 120    HG1018384P1 NP_001314:NM_001323
MARSNLPLALGLALVAFCLLALPRDARARPQERMVGELRDLSPDDPQVQKAAQAAVASYN
MGSNSIYYFRDTHIIKAQSQLVAGIKYFLTMEMGSTDCRKTRVTGDHVDLTTCPLAAGAQ
QEKLRCDFEVLVVPWQNSSQLLKHNCVQM

SEQ.ID.NO. 121    HG1018385P1 NP_001314:NM_001323_1-26
MARSNLPLALGLALVAFCLLALPRDA

SEQ.ID.NO. 122    HG1018386P1 NP_001314:NM_001323_1-18
MARSNLPLALGLALVAFC

SEQ.ID.NO. 123    HG1018387P1 NP_001314:NM_001323_1-20
MARSNLPLALGLALVAFCLL

SEQ.ID.NO. 124    HG1018388P1 NP_001314:NM_001323_1-28
MARSNLPLALGLALVAFCLLALPRDARA

SEQ.ID.NO. 125    HG1018389P1 NP_001314:NM_001323_1-21
MARSNLPLALGLALVAFCLLA

SEQ.ID.NO. 126    HG1018390P1 NP_001314:NM_001323_1-23
MARSNLPLALGLALVAFCLLALP

SEQ.ID.NO. 127    HG1018391P1 NP_001822:NM_001831
MMKTLLLFVGLLLTWESGQVLGDQTVSDNELQEMSNQGSKYVNKEIQNAVNGVKQIKTLI
EKTNEERKTLLSNLEEAKKKKEDALNETRESETKLKELPGVCNETMMALWEECKPCLKQT

CMKFYARVCRSGSGLVGRQLEEFLNQSSPFYFWMNGDRIDSLLENDRQQTHMLDVMQDHF
SRASSIIDELFQDRFFTREPQDTYHYLPFSLPHRRPHFFFPKSRIVRSLMPFSPYEPLNF
HAMFQPFLEMIHEAQQAMDIHFHSPAFQHPPTEFIREGDDDRTVCREIRHNSTGCLRMKD
QCDKCREILSVDCSTNNPSQAKLRRELDESLQVAERLTRKYNELLKSYQWKMLNTSSLLE
QLNEQFNWVSRLANLTQGEDQYYLRVTTVASHTSDSDVPSGVTEVVVKLFDSDPITVTVP
VEVSRKNPKFMETVAEKALQEYRKKHREE

SEQ.ID.NO. 128    HG1018392P1 NP_001822:NM_001831_1-22
MMKTLLLFVGLLLTWESGQVLG

SEQ.ID.NO. 129    HG1018393P1 NP_001822:NM_001831_1-18
MMKTLLLFVGLLLTWESG

SEQ.ID.NO. 130    HG1018394P1 NP_001822:NM_001831_1-14
MMKTLLLFVGLLLT

SEQ.ID.NO. 131    HG1018395P1 NP_002206:NM_002215
MDGAMGPRGLLLCMYLVSLLILQAMPALGSATGRSKSSEKRQAVDTAVDGVFIRSLKVNC
KVTSRFAHYVVTSQVVNTANEAREVAFDLEIPKTAFISDFAVTADGNAFIGDIKDKVTAW
KQYRKAAISGENAGLVRASGRTMEQFTIHLTVNPQSKVTFQLTYEEVLKRNHMQYEIVIK
VKPKQLVHHFEIDVDIFEPQGISKLDAQASFLPKELAAQTIKKSFSGKKGHVLFRPTVSQ
QQSCPTCSTSLLNGHFKVTYDVTRDEICDLLVANNHFAHFFAPQNLTNMNKNVVFVIDIS
GSMRGQKVKQTKEALLKILGDMQPGDYFDLVLFGTRVQSWKGSLVQASEANLQAAQDFVR
GFSLDEATNLNGGLLRGIEILNQVQESLPELSNHASILIMLTDGDPTEGVTDRSQILKNV
RNAIRGRFPLYNLGFGHNVDFNFLEVMSMENNGRAQRIYEDHDATQQLQGFYSQVAKPLL
VDVDLQYPQDAVLALTQNHHKQYYEGSEIVVAGRIADNKQSSFKADVQAHGEGQEFSITC
LVDEEEMKKLLRERGHMLENHVERLWAYLTIQELLAKRMKVDREVRANLSSQALRMSLDY
GFVTPLTSMSIRGMADQDGLKPTIDKPSEDSPPLEMLGPRRTFVLSALQPSPTHSSSNTQ
RLPDRVTGVDTDPHFIIHVPQKEDTLCFNINEEPGVILSLVQDPNTGFSVNGQLIGNKAR
SPGQHDGTYFGRLGIANPATDFQLEVTPQNITLNPGFGGPVFSWRDQAVLRQDGVVVTIN
KKRNLVVSVDDGGTFEVVLHRVWKGSSVHQDFLGFYVLDSHRMSARTHGLLGQFFHPIGF
EVSDIHPGSDPTKPDATMVVRNRRLTVTRGLQKDYSKDPWHGAEVSCWFIHNNGAGLIDG
AYTDYIVPDIF

SEQ.ID.NO. 132    HG1018396P1 NP_002206:NM_002215_1-24
MDGAMGPRGLLLCMYLVSLLILQA

SEQ.ID.NO. 133    HG1018397P1 NP_002206:NM_002215_1-29
MDGAMGPRGLLLCMYLVSLLILQAMPALG

SEQ.ID.NO. 134    HG1018398P1 NP_002206:NM_002215_1-30
MDGAMGPRGLLLCMYLVSLLILQAMPALGS

SEQ.ID.NO. 135    HG1018399P1 NP_002206:NM_002215_1-23

MDGAMGPRGLLLCMYLVSLLILQ

SEQ.ID.NO. 136    HG1018400P1 NP_002206:NM_002215_1-31
MDGAMGPRGLLLCMYLVSLLILQAMPALGSA

SEQ.ID.NO. 137    HG1018401P1 NP_002300:NM_002309
MKVLAAGVVPLLLVLHWKHGAGSPLPITPVNATCAIRHPCHNNLMNQIRSQLAQLNGSAN
ALFILYYTAQGEPFPNNLDKLCGPNVTDFPPFHANGTEKAKLVELYRIVVYLGTSLGNIT
RDQKILNPSALSLHSKLNATADILRGLLSNVLCRLCSKYHVGHVDVTYGPDTSGKDVFQK
KKLGCQLLGKYKQIIAVLAQAF

SEQ.ID.NO. 138    HG1018402P1 NP_002300:NM_002309_1-22
MKVLAAGVVPLLLVLHWKHGAG

SEQ.ID.NO. 139    HG1018403P1 NP_002300:NM_002309_1-23
MKVLAAGVVPLLLVLHWKHGAGS

SEQ.ID.NO. 140    HG1018404P1 NP_002336:NM_002345
MSLSAFTLFLALIGGTSGQYYDYDFPLSIYGQSSPNCAPECNCPESYPSAMYCDELKLKS
VPMVPPGIKYLYLRNNQIDHIDEKAFENVTDLQWLILDHNLLENSKIKGRVFSKLKQLKK
LHINHNNLTESVGPLPKSLEDLQLTHNKITKLGSFEGLVNLTFIHLQHNRLKEDAVSAAF
KGLKSLEYLDLSFNQIARLPSGLPVSLLTLYLDNNKISNIPDEYFKRFNALQYLRLSHNE
LADSGIPGNSFNVSSLVELDLSYNKLKNIPTVNENLENYYLEVNQLEKFDIKSFCKILGP
LSYSKIKHLRLDGNRISETSLPPDMYECLRVANEVTLN

SEQ.ID.NO. 141    HG1018405P1 NP_002336:NM_002345_1-18
MSLSAFTLFLALIGGTSG

SEQ.ID.NO. 142    HG1018406P1 NP_002336:NM_002345_1-15
MSLSAFTLFLALIGG

SEQ.ID.NO. 143    HG1018407P1 NP_002336:NM_002345_1-17
MSLSAFTLFLALIGGTS

SEQ.ID.NO. 144    HG1018408P1 NP_002336:NM_002345_1-14
MSLSAFTLFLALIG

SEQ.ID.NO. 145    HG1018409P1 NP_002402:NM_002411
MKLLMVLMLAALSQHCYAGSGCPLLENVISKTINPQVSKTEYKELLQEFIDDNATTNAID
ELKECFLNQTDETLSNVEVFMQLIYDSSLCDLF

SEQ.ID.NO. 146    HG1018410P1 NP_002402:NM_002411_1-18
MKLLMVLMLAALSQHCYA

SEQ.ID.NO. 147    HG1018411P1 NP_002505:NM_002514
MQSVQSTSFCLRKQCLCLTFLLLHLLGQVAATQRCPPQCPGRCPATPPTCAPGVRAVLDG
CSCCLVCARQRGESCSDLEPCDESSGLYCDRSADPSNQTGICTAVEGDNCVFDGVIYRSG
EKFQPSCKFQCTCRDGQIGCVPRCQLDVLLPEPNCPAPRKVEVPGECCEKWICGPDEEDS
LGGLTLAAYRPEATLGVEVSDSSVNCIEQTTEWTACSKSCGMGFSTRVTNRNRQCEMLKQ
TRLCMVRPCEQEPEQPTDKKGKKCLRTKKSLKAIHLQFKNCTSLHTYKPRFCGVCSDGRC
CTPHNTKTIQAEFQCSPGQIVKKPVMVIGTCTCHTNCPKNNEAFLQELELKTTRGKM

SEQ.ID.NO. 148    HG1018412P1 NP_002505:NM_002514_1-30
MQSVQSTSFCLRKQCLCLTFLLLHLLGQVA

SEQ.ID.NO. 149    HG1018413P1 NP_002505:NM_002514_1-32
MQSVQSTSFCLRKQCLCLTFLLLHLLGQVAAT

SEQ.ID.NO. 150    HG1018414P1 NP_002505:NM_002514_1-28
MQSVQSTSFCLRKQCLCLTFLLLHLLGQ

SEQ.ID.NO. 151    HG1018415P1 NP_002505:NM_002514_1-27
MQSVQSTSFCLRKQCLCLTFLLLHLLG

SEQ.ID.NO. 152    HG1018416P1 NP_002505:NM_002514_1-31
MQSVQSTSFCLRKQCLCLTFLLLHLLGQVAA

SEQ.ID.NO. 153    HG1018417P1 NP_002892:NM_002901
MARGGRGRRLGLALGLLLALVLAPRVLRAKPTVRKERVVRPDSELGERPPEDNQSFQYDH
EAFLGKEDSKTFDQLTPDESKERLGKIVDRIDNDGDGFVTTEELKTWIKRVQKRYIFDNV
AKVWKDYDRDKDDKISWEEYKQATYGYYLGNPAEFHDSSDHHTFKKMLPRDERRFKAADL
NGDLTATREEFTAFLHPEEFEHMKEIVVLETLEDIDKNGDGFVDQDEYIADMFSHEENGP
EPDWVLSEREQFNEFRDLNKDGKLDKDEIRHWILPQDYDHAQAEARHLVYESDKNKDEKL
TKEEILENWNMFVGSQATNYGEDLTKNHDEL

SEQ.ID.NO. 154    HG1018418P1 NP_002892:NM_002901_1-26
MARGGRGRRLGLALGLLLALVLAPRV

SEQ.ID.NO. 155    HG1018419P1 NP_002892:NM_002901_1-22
MARGGRGRRLGLALGLLLALVL

SEQ.ID.NO. 156    HG1018420P1 NP_002892:NM_002901_1-29
MARGGRGRRLGLALGLLLALVLAPRVLRA

SEQ.ID.NO. 157    HG1018421P1 NP_002892:NM_002901_1-24
MARGGRGRRLGLALGLLLALVLAP

SEQ.ID.NO. 158    HG1018422P1 NP_002892:NM_002901_1-23

MARGGRGRRLGLALGLLLALVLA

SEQ.ID.NO. 159    HG1018423P1 NP_002893:NM_002902
MRLGPRTAALGLLLLCAAAAGAGKAEELHYPLGERRSDYDREALLGVQEDVDEYVKLGHE
EQQKRLQAIIKKIDLDSDGFLTESELSSWIQMSFKHYAMQEAKQQFVEYDKNSDDTVTWD
EYNIQMYDRVIDFDENTALDDAEEESFRKLHLKDKKRFEKANQDSGPGLSLEEFIAFEHP
EEVDYMTEFVIQEALEEHDKNGDGFVSLEEFLGDYRWDPTANEDPEWILVEKDRFVNDYD
KDNDGRLDPQELLPWVVPNNQGIAQEEALHLIDEMDLNGDKKLSEEEILENPDLFLTSEA
TDYGRQLHDDYFYHDEL

SEQ.ID.NO. 160    HG1018424P1 NP_002893:NM_002902_1-25
MRLGPRTAALGLLLLCAAAAGAGKA

SEQ.ID.NO. 161    HG1018425P1 NP_002893:NM_002902_1-19
MRLGPRTAALGLLLLCAAA

SEQ.ID.NO. 162    HG1018426P1 NP_002893:NM_002902_1-22
MRLGPRTAALGLLLLCAAAAGA

SEQ.ID.NO. 163    HG1018427P1 NP_002893:NM_002902_1-18
MRLGPRTAALGLLLLCAA

SEQ.ID.NO. 164    HG1018428P1 NP_002893:NM_002902_1-20
MRLGPRTAALGLLLLCAAAA

SEQ.ID.NO. 165    HG1018429P1 NP_002893:NM_002902_1-21
MRLGPRTAALGLLLLCAAAAG

SEQ.ID.NO. 166    HG1018430P1 NP_002893:NM_002902_1-23
MRLGPRTAALGLLLLCAAAAGAG

SEQ.ID.NO. 167    HG1018431P1 NP_005133:NM_005142
MAWFALYLLSLLWATAGTSTQTQSSCSVPSAQEPLVNGIQVLMENSVTSSAYPNPSILIA
MNLAGAYNLKAQKLLTYQLMSSDNNDLTIGHLGLTIMALTSSCRDPGDKVSILQRQMENW
APSSPNAEASAFYGPSLAILALCQKNSEATLPIAVRFAKTLLANSSPFNVDTGAMATLAL
TCMYNKIPVGSEEGYRSLFGQVLKDIVEKISMKIKDNGIIGDIYSTGLAMQALSVTPEPS
KKEWNCKKTTDMILNEIKQGKFHNPMSIAQILPSLKGKTYLDVPQVTCSPDHEVQPTLPS
NPGPGPTSASNITVIYTINNQLRGVELLFNETINVSVKSGSVLLVVLEEAQRKNPMFKFE
TTMTSWGLVVSSINNIAENVNHKTYWQFLSGVTPLNEGVADYIPFNHEHITANFTQY

SEQ.ID.NO. 168    HG1018432P1 NP_005133:NM_005142_1-19
MAWFALYLLSLLWATAGTS

SEQ.ID.NO. 169    HG1018433P1 NP_005133:NM_005142_1-18

MAWFALYLLSLLWATAGT

SEQ.ID.NO. 170    HG1018434P1 NP_005133:NM_005142_1-20
MAWFALYLLSLLWATAGTST

SEQ.ID.NO. 171    HG1018435P1 NP_005133:NM_005142_1-24
MAWFALYLLSLLWATAGTSTQTQS

SEQ.ID.NO. 172    HG1018436P1 NP_005133:NM_005142_1-16
MAWFALYLLSLLWATA

SEQ.ID.NO. 173    HG1018437P1 NP_005133:NM_005142_1-17
MAWFALYLLSLLWATAG

SEQ.ID.NO. 174    HG1018438P1 NP_005133:NM_005142_1-14
MAWFALYLLSLLWA

SEQ.ID.NO. 175    HG1018439P1 NP_005445:NM_005454
MHLLLFQLLVLLPLGKTTRHQDGRQNQSSLSPVLLPRNQRELPTGNHEEAEEKPDLFVAV
PHLVATSPAGEGQRQREKMLSRFGRFWKKPEREMHPSRDSDSEPFPPGTQSLIQPIDGMK
MEKSPLREEAKKFWHHFMFRKTPASQGVILPIKSHEVHWETCRTVPFSQTITHEGCEKVV
VQNNLCFGKCGSVHFPGAAQHSHTSCSHCLPAKFTTMHLPLNCTELSSVIKVVMLVEECQ
CKVKTEHEDGHILHAGSQDSFIPGVSA

SEQ.ID.NO. 176    HG1018440P1 NP_005445:NM_005454_1-17
MHLLLFQLLVLLPLGKT

SEQ.ID.NO. 177    HG1018441P1 NP_005555:NM_005564
MPLGLLWLGLALLGALHAQAQDSTSDLIPAPPLSKVPLQQNFQDNQFQGKWYVVGLAGNA
ILREDKDPQKMYATIYELKEDKSYNVTSVLFRKKKCDYWIRTFVPGCQPGEFTLGNIKSY
PGLTSYLVRVVSTNYNQHAMVFFKKVSQNREYFKITLYGRTKELTSELKENFIRFSKYLG
LPENHIVFPVPIDQCIDG

SEQ.ID.NO. 178    HG1018442P1 NP_005555:NM_005564_1-18
MPLGLLWLGLALLGALHA

SEQ.ID.NO. 179    HG1018443P1 NP_005555:NM_005564_1-20
MPLGLLWLGLALLGALHAQA

SEQ.ID.NO. 180    HG1018444P1 NP_005555:NM_005564_1-15
MPLGLLWLGLALLGA

SEQ.ID.NO. 181    HG1018445P1 NP_005690:NM_005699
MRHNWTPDLSPLWVLLLCAHVVTLLVRATPVSQTTTAATASVRSTKDPCPSQPPVFPAAK

QCPALEVTWPEVEVPLNGTLSLSCVACSRFPNFSILYWLGNGSFIEHLPGRLWEGSTSRE
RGSTGTQLCKALVLEQLTPALHSTNFSCVLVDPEQVVQRHVVLAQLWVRSPRRGLQEQEE
LCFHMWGGKGGLCQSSL

SEQ.ID.NO. 182    HG1018446P1 NP_005690:NM_005699_1-29
MRHNWTPDLSPLWVLLLCAHVVTLLVRAT

SEQ.ID.NO. 183    HG1018447P1 NP_005690:NM_005699_1-24
MRHNWTPDLSPLWVLLLCAHVVTL

SEQ.ID.NO. 184    HG1018448P1 NP_005690:NM_005699_1-28
MRHNWTPDLSPLWVLLLCAHVVTLLVRA

SEQ.ID.NO. 185    HG1018449P1 NP_006560:NM_006569
MLPLTMTVLILLLLPTGQAAPKDGVTRPDSEVQHQLLPNPFQPGQEQLGLLQSYLKGLGR
TEVQLEHLSREQVLLYLFALHDYDQSGQLDGLELLSMLTAALAPGAANSPTTNPVILIVD
KVLETQDLNGDGLMTPAELINFPGVALRHVEPGEPLAPSPQEPQAVGRQSLLAKSPLRQE
TQEAPGPREEAKGQVEARRESLDPVQEPGGQAEADGDVPGPRGEAEGQAEAKGDAPGPRG
EAEGQAEAKGDAPGPRGEAGGQAEARENGEEAKELPGETLESKNTQNDFEVHIVQVENDE
I

SEQ.ID.NO. 186    HG1018450P1 NP_006560:NM_006569_1-19
MLPLTMTVLILLLLPTGQA

SEQ.ID.NO. 187    HG1018451P1 NP_006560:NM_006569_1-18
MLPLTMTVLILLLLPTGQ

SEQ.ID.NO. 188    HG1018452P1 NP_006560:NM_006569_1-21
MLPLTMTVLILLLLPTGQAAP

SEQ.ID.NO. 189    HG1018453P1 NP_006856:NM_006865
MTSILTVLICLGLSLDPRTHVQAGPLPKPTLWAEPGSVITQGSPVTLRCQGSLETQEYHL
YREKKTALWITRIPQELVKKGQFPILSITWEHAGRYCCIYGSHTAGLSESSDPLELVVTG
AYSKPTLSALPSPVVTSGGNVTIQCDSQVAFDGFILCKEGEDEHPQCLNSHSHARGSSRA
IFSVGPVSPSRRWSYRCYGYDSRAPYVWSLPSDLLGLLVPGVSKKPSLSVQPGPVVAPGE
KLTFQCGSDAGYDRFVLYKEWGRDFLQRPGRQPQAGLSQANFTLGPVSRSYGGQYTCSGA
YNLSSEWSAPSDPLDILITGQIRARPFLSVRPGPTVASGENVTLLCQSQGGMHTFLLTKE
GAADSPLRLKSKRQSHKYQAEFPMSPVTSAHAGTYRCYGSLSSNPYLLTHPSDPLELVVS
GAAETLSPPQNKSDSKAGE

SEQ.ID.NO. 190    HG1018454P1 NP_006856:NM_006865_1-15
MTSILTVLICLGLSL

SEQ.ID.NO. 191    HG1018455P1 NP_036577:NM_012445

MENPSPAAALGKALCALLLATLGAAGQPLGGESICSARAPAKYSITFTGKWSQTAFPKQY
PLFRPPAQWSSLLGAAHSSDYSMWRKNQYVSNGLRDFAERGEAWALMKEIEAAGEALQSV
HAVFSAPAVPSGTGQTSAELEVQRRHSLVSFVVRIVPSPDWFVGVDSLDLCDGDRWREQA
ALDLYPYDAGTDSGFTFSSPNFATIPQDTVTEITSSSPSHPANSFYYPRLKALPPIARVT
LVRLRQSPRAFIPPAPVLPSRDNEIVDSASVPETPLDCEVSLWSSWGLCGGHCGRLGTKS
RTRYVRVQPANNGSPCPELEEEAECVPDNCV

SEQ.ID.NO. 192    HG1018456P1 NP_036577:NM_012445_1-26
MENPSPAAALGKALCALLLATLGAAG

SEQ.ID.NO. 193    HG1018457P1 NP_036577:NM_012445_1-25
MENPSPAAALGKALCALLLATLGAA

SEQ.ID.NO. 194    HG1018458P1 NP_036577:NM_012445_1-24
MENPSPAAALGKALCALLLATLGA

SEQ.ID.NO. 195    HG1018459P1 NP_036577:NM_012445_1-28
MENPSPAAALGKALCALLLATLGAAGQP

SEQ.ID.NO. 196    HG1018460P1 NP_055070:NM_014255
MKGWGWLALLLGALLGTAWARRSQDLHCGACRALVDELEWEIAQVDPKKTIQMGSFRINP
DGSQSVVEVPYARSEAHLTELLEEICDRMKEYGEQIDPSTHRKNYVRVVGRNGESSELDL
QGIRIDSDISGTLKFACESIVEEYEDELIEFFSREADNVKDKLCSKRTDLCDHALHISHD
EL

SEQ.ID.NO. 197    HG1018461P1 NP_055070:NM_014255_1-20
MKGWGWLALLLGALLGTAWA

SEQ.ID.NO. 198    HG1018462P1 NP_055070:NM_014255_1-18
MKGWGWLALLLGALLGTA

SEQ.ID.NO. 199    HG1018463P1 NP_055070:NM_014255_1-16
MKGWGWLALLLGALLG

SEQ.ID.NO. 200    HG1018464P1 NP_055582:NM_014767
MRAPGCGRLVLPLLLLAAAALAEGDAKGLKEGETPGNFMEDEQWLSSISQYSGKIKHWNR
FRDEVEDDYIKSWEDNQQGDEALDTTKDPCQKVKCSRHKVCIAQGYQRAMCISRKKLEHR
IKQPTVKLHGNKDSICKPCHMAQLASVCGSDGHTYSSVCKLEQQACLSSKQLAVRCEGPC
PCPTEQAATSTADGKPETCTGQDLADLGDRLRDWFQLLHENSKQNGSASSVAGPASGLDK
SLGASCKDSIGWMFSKLDTSADLFLDQTELAAINLDKYEVCIRPFFNSCDTYKDGRVSTA
EWCFCFWREKPPCLAELERIQIQEAAKKKPGIFIPSCDEDGYYRKMQCDQSSGDCWCVDQ
LGLELTGTRTHGSPDCDDIVGFSGDFGSGVGWEDEEEKETEEAGEEAEEEEGEAGEADDG
GYIW

SEQ.ID.NO. 201    HG1018465P1 NP_055582:NM_014767_1-24
MRAPGCGRLVLPLLLLAAAALAEG

SEQ.ID.NO. 202    HG1018466P1 NP_055582:NM_014767_1-19
MRAPGCGRLVLPLLLLAAA

SEQ.ID.NO. 203    HG1018467P1 NP_055582:NM_014767_1-22
MRAPGCGRLVLPLLLLAAAALA

SEQ.ID.NO. 204    HG1018468P1 NP_055582:NM_014767_1-20
MRAPGCGRLVLPLLLLAAAA

SEQ.ID.NO. 205    HG1018469P1 NP_055582:NM_014767_1-26
MRAPGCGRLVLPLLLLAAAALAEGDA

SEQ.ID.NO. 206    HG1018470P1 NP_055582:NM_014767_1-21
MRAPGCGRLVLPLLLLAAAAL

SEQ.ID.NO. 207    HG1018471P1 NP_055697:NM_014882
MSLGQSACLFLSIARSRSVMTGEQMAAFHPSSTPNPLERPIKMGWLKKQRSIVKNWQQRY
FVLRAQQLYYYKDEEDTKPQGCMYLPGCTIKEIATNPEEAGKFVFEIIPASWDQNRMGQD
SYVLMASSQAEMEEWVKFLRRVAGTPCGVFGQRLDETVAYEQKFGPHLVPILVEKCAEFI
LEHGRNEEGIFRLPGQDNLVKQLRDAFDAGERPSFDRDTDVHTVASLLKLYLRDLPEPVV
PWSQYEGFLLCGQLTNADEAKAQQELMKQLSILPRDNYSLLSYICRFLHEIQLNCAVNKM
SVDNLATVIGVNLIRSKVEDPAVIMRGTPQIQRVMTMMIRDHEVLFPKSKDIPLSPPAQK
NDPKKAPVARSSVGWDATEDLRISRTDSFSSMTSDSDTTSPTGQQPSDAFPEDSSKVPRE
KPGDWKMQSRKRTQTLPNRKCFLTSAFQGANSSKMEIFKNEFWSPSSEAKAGEGHRRTMS
QDLRQLSDSQRTSTYDNVPSLPGSPGEEASALSSQACDSKGDTLASPNSETGPGKKNSGE
EEIDSLQRMVQELRKEIETQKQMYEEQIKNLEKENYDVWAKVVRLNEELEKEKKKSAALE
ISLRNMERSREDVEKRNKALEEEVKEFVKSMKEPKTEA

SEQ.ID.NO. 208    HG1018472P1 NP_055697:NM_014882_1-18
MSLGQSACLFLSIARSRS

SEQ.ID.NO. 209    HG1018473P1 NP_056965:NM_015881
MQRLGATLLCLLLAAAVPTAPAPAPTATSAPVKPGPALSYPQEEATLNEMFREVEELMED
TQHKLRSAVEEMEAEEAAAKASSEVNLANLPPSYHNETNTDTKVGNNTIHVHREIHKITN
NQTGQMVFSETVITSVGDEEGRRSHECIIDEDCGPSMYCQFASFQYTCQPCRGQRMLCTR
DSECCGDQLCVWGHCTKMATRGSNGTICDNQRDCQPGLCCAFQRGLLFPVCTPLPVEGEL
CHDPASRLLDLITWELEPDGALDRCPCASGLLCQPHSHSLVYVCKPTFVGSRDQDGEILL
PREVPDEYEVGSFMEEVRQELEDLERSLTEEMALGEPAAAAAALLGGEEI

SEQ.ID.NO. 210    HG1018474P1 NP_056965:NM_015881_1-18
MQRLGATLLCLLLAAAVP

SEQ.ID.NO. 211    HG1018475P1 NP_056965:NM_015881_1-19
MQRLGATLLCLLLAAAVPT

SEQ.ID.NO. 212    HG1018476P1 NP_056965:NM_015881_1-22
MQRLGATLLCLLLAAAVPTAPA

SEQ.ID.NO. 213    HG1018477P1 NP_056965:NM_015881_1-16
MQRLGATLLCLLLAAA

SEQ.ID.NO. 214    HG1018478P1 NP_056965:NM_015881_1-21
MQRLGATLLCLLLAAAVPTAP

SEQ.ID.NO. 215    HG1018479P1 NP_057603:NM_016519
MSASKIPLFKMKDLILILCLLEMSFAVPFFPQQSGTPGMASLSLETMRQLGSLQRLNTLS
QYSRYGFGKSFNSLWMHGLLPPHSSLPWMRPREHETQQYEYSLPVHPPPLPSQPSLKPQQ
PGLKPFLQSAAATTNQATALKEALQPPIHLGHLPLQEGELPLVQQQVAPSDKPPKPELPG
VDFADPQGPSLPGMDFPDPQGPSLPGLDFADPQGSTIFQIARLISHGPMPQNKQSPLYPG
MLYVPFGANQLNAPARLGIMSSEEVAGGREDPMAYGAMFPGFGGMRPGFEGMPHNPAMGG
DFTLEFDSPVAATKGPENEEGGAQGSPMPEANPDNLENPAFLTELEPAPHAGLLALPKDD
IPGLPRSPSGKMKGLPSVTPAAADPLMTPELADVYRTYDADMTTSVDFQEEATMDTTMAP
NSLQTSMPGNKAQEPEMMHDAWHFQEP

SEQ.ID.NO. 216    HG1018480P1 NP_057603:NM_016519_1-26
MSASKIPLFKMKDLILILCLLEMSFA

SEQ.ID.NO. 217    HG1018481P1 NP_057603:NM_016519_1-28
MSASKIPLFKMKDLILILCLLEMSFAVP

SEQ.ID.NO. 218    HG1018482P1 NP_149439:NM_033183
MEMFQGLLLLLLLSMGGTWASKEPLRPRCRPINATLAVEKEGCPVCITVNTTICAGYCPT
MTRVLQGVLPALPQVVCNYRDVRFESIRLPGCPRGVNPVVSYAVALSCQCALCRRSTTDC
GGPKDHPLTCDDPRFQDSSSSKAPPPSLPSPSRLPGPSDTPILPQ

SEQ.ID.NO. 219    HG1018483P1 NP_149439:NM_033183_1-18
MEMFQGLLLLLLLSMGGT

SEQ.ID.NO. 220    HG1018484P1 NP_149439:NM_033183_1-20
MEMFQGLLLLLLLSMGGTWA

SEQ.ID.NO. 221    HG1018485P1 NP_149439:NM_033183_1-16
MEMFQGLLLLLLLSMG

SEQ.ID.NO. 222    HG1018486P1 NP_644808:NM_139279

MTMRSLLRTPFLCGLLWAFCAPGARAEEPAASFSQPGSMGLDKNTVHDQEHIMEHLEGVI
NKPEAEMSPQELQLHYFKMHDYDGNNLLDGLELSTAITHVHKEEGSEQAPLMSEDELINI
IDGVLRDDDKNNDGYIDYAEFAKSLQ

SEQ.ID.NO. 223    HG1018487P1 NP_644808:NM_139279_1-18
MTMRSLLRTPFLCGLLWA

SEQ.ID.NO. 224    HG1018488P1 NP_644808:NM_139279_1-20
MTMRSLLRTPFLCGLLWAFC

SEQ.ID.NO. 225    HG1018489P1 NP_644808:NM_139279_1-26
MTMRSLLRTPFLCGLLWAFCAPGARA

SEQ.ID.NO. 226    HG1018490P1 NP_644808:NM_139279_1-23
MTMRSLLRTPFLCGLLWAFCAPG

SEQ.ID.NO. 227    HG1018491P1 NP_660295:NM_145252
MLLLLTLALLGGPTWAGKMYGPGGGKYFSTTEDYDHEITGLRVSVGLLLVKSVQVKLGDS
WDVKLGALGGNTQEVTLQPGEYITKVFVAFQAFLRGMVMYTSKDRYFYFGKLDGQISSAY
PSQEGQVLVGIYGQYQLLGIKSIGFEWNYPLEEPTTEPPVNLTYSANSPVGR

SEQ.ID.NO. 228    HG1018492P1 NP_660295:NM_145252_1-13
MLLLLTLALLGGP

SEQ.ID.NO. 229    HG1018493P1 NP_660295:NM_145252_1-16
MLLLLTLALLGGPTWA

SEQ.ID.NO. 230    HG1018494P1 NP_660295:NM_145252_1-14
MLLLLTLALLGGPT

SEQ.ID.NO. 231    HG1018495P1 NP_660295:NM_145252_1-17
MLLLLTLALLGGPTWAG

SEQ.ID.NO. 232    HG1018496P1 NP_689534:NM_152321
MEAAPSRFMFLLFLLTCELAAEVAAEVEKSSDGPGAAQEPTWLTDVPAAMEFIAATEVAV
IGFFQDLEIPAVPILHSMVQKFPGVSFGISTDSEVLTHYNITGNTICLFRLVDNEQLNLE
DEDIESIDATKLSRFIEINSLHMVTEYNPVTVIGLFNSVIQIHLLLIMNKASPEYEENMH
RYQKAAKLFQGKILFILVDSGMKENGKVISFFKLKESQLPALAIYQTLDDEWDTLPTAEV
SVEHVQNFCDGFLSGKLLKENRESEGKTPKVEL

SEQ.ID.NO. 233    HG1018497P1 NP_689534:NM_152321_1-25
MEAAPSRFMFLLFLLTCELAAEVAA

SEQ.ID.NO. 234    HG1018498P1 NP_689534:NM_152321_1-21

MEAAPSRFMFLLFLLTCELAA

SEQ.ID.NO. 235    HG1018499P1 NP_689848:NM_152635
MPPFLLLTCLFITGTSVSPVALDPCSAYISLNEPWRNTDHQLDESQGPPLCDNHVNGEWY
HFTGMAGDAMPTFCIPENHCGTHAPVWLNGSHPLEGDGIVQRQACASFNGNCCLWNTTVE
VKACPGGYYVYRLTKPSVCFHVYCGHFYDICDEDCHGSCSDTSECTCAPGTVLGPDRQTC
FDENECEQNNGGCSEICVNLKNSYRCECGVGRVLRSDGKTCEDVEGCHNNNGGCSHSCLG
SEKGYQCECPRGLVLSEDNHTCQVPVLCKSNAIEVNIPRELVGGLELFLTNTSCRGVSNG
THVNILFSLKTCGTVVDVVNDKIVASNLVTGLPKQTPGSSGDFIIRTSKLLIPVTCEFPR
LYTISEGYVPNLRNSPLEIMSRNHGIFPFTLEIFKDNEFEEPYREALPTLKLRDSLYFGI
EPVVHVSGLESLVESCFATPTSKIDEVLKYYLIRDGCVSDDSVKQYTSRDHLAKHFQVPV
FKFVGKDHKEVFLHCRVLVCGVLDERSRCAQGCHRRMRRGAGGEDSAGLQGQTLTGGPIR
IDWED

SEQ.ID.NO. 236    HG1018500P1 NP_689848:NM_152635_1-18
MPPFLLLTCLFITGTSVS

SEQ.ID.NO. 237    HG1018501P1 NP_689848:NM_152635_1-16
MPPFLLLTCLFITGTS

SEQ.ID.NO. 238    HG1018502P1 NP_689848:NM_152635_1-15
MPPFLLLTCLFITGT

SEQ.ID.NO. 239    HG1018503P1 NP_689968:NM_152755
MGPVRLGILLFLFLAVHEAWAGMLKEEDDDTERLPSKCEVCKLLSTELQAELSRTGRSRE
VLELGQVLDTGKRKRHVPYSVSETRLEEALENLCERILDYSVHAERKGSLRYAKGQSQTM
ATLKGLVQKGVKVDLGIPLELWDEPSVEVTYLKKQCETMLEEFEDIVGDWYFHHQEQPLQ
NFLCEGHVLPAAETACLQETWTGKEITDGEEKTEGEEEQEEEEEEEEEEGGDKMTKTGSH
PKLDREDL

SEQ.ID.NO. 240    HG1018504P1 NP_689968:NM_152755_1-21
MGPVRLGILLFLFLAVHEAWA

SEQ.ID.NO. 241    HG1018505P1 NP_766630:NM_173042
MRHNWTPDLSPLWVLLLCAHVVTLLVRATPVSQTTTAATASVRSTKDPCPSQPPVFPAAK
QCPALEVTWPEVEVPLNGTLSLSCVACSRFPNFSILYWLGNGSFIEHLPGRLWEGSTSRE
RGSTGTQLCKALVLEQLTPALHSTNFSCVLVDPEQVVQRHVVLAQLWAGLRATLPPTQEA
LPSSHSSPQQQG

SEQ.ID.NO. 242    HG1018506P1 NP_766630:NM_173042_1-29
MRHNWTPDLSPLWVLLLCAHVVTLLVRAT

SEQ.ID.NO. 243    HG1018507P1 NP_766630:NM_173042_1-24
MRHNWTPDLSPLWVLLLCAHVVTL

SEQ.ID.NO. 244    HG1018508P1 NP_766630:NM_173042_1-28
MRHNWTPDLSPLWVLLLCAHVVTLLVRA

SEQ.ID.NO. 245    HG1018509P1 NP_776214:NM_173842
MEICRGLRSHLITLLLFLFHSETICRPSGRKSSKMQAFRIWDVNQKTFYLRNNQLVAGYL
QGPNVNLEEKIDVVPIEPHALFLGIHGGKMCLSCVKSGDETRLQLEAVNITDLSENRKQD
KRFAFIRSDSGPTTSFESAACPGWFLCTAMEADQPVSLTNMPDEGVMVTKFYFQEDE

SEQ.ID.NO. 246    HG1018510P1 NP_776214:NM_173842_1-23
MEICRGLRSHLITLLLFLFHSET

SEQ.ID.NO. 247    HG1018511P1 NP_776214:NM_173842_1-25
MEICRGLRSHLITLLLFLFHSETIC

SEQ.ID.NO. 248    HG1018512P1 NP_783165:NM_175575
MWAPRCRRFWSRWEQVAALLLLLLLLGVPPRSLALPPIRYSHAGICPNDMNPNLWVDAQS
TCRRECETDQECETYEKCCPNVCGTKSCVAARYMDVKGKKGPVGMPKEATCDHFMCLQQG
SECDIWDGQPVCKCKDRCEKEPSFTCASDGLTYYNRCYMDAEACSKGITLAVVTCRYHFT
WPNTSPPPPETTMHPTTASPETPELDMAAPALLNNPVHQSVTMGETVSFLCDVVGRPRPE
ITWEKQLEDRENVVMRPNHVRGNVVVTNIAQLVIYNAQLQDAGIYTCTARNVAGVLRADF
PLSVVRGHQAAATSESSPNGTAFPAAECLKPPDSEDCGEEQTRWHFDAQANNCLTFTFGH
CHRNLNHFETYEACMLACMSGPLAACSLPALQGPCKAYAPRWAYNSQTGQCQSFVYGGCE
GNGNNFESREACEESCPFPRGNQRCRACKPRQKLVTSFCRSDFVILGRVSELTEEPDSGR
ALVTVDEVLKDEKMGLKFLGQEPLEVTLLHVDWACPCPNVTVSEMPLIIMGEVDGGMAML
RPDSFVGASSARRVRKLREVMHKKTCDVLKEFLGLH

SEQ.ID.NO. 249    HG1018513P1 NP_783165:NM_175575_1-32
MWAPRCRRFWSRWEQVAALLLLLLLLGVPPRS

SEQ.ID.NO. 250    HG1018514P1 NP_783165:NM_175575_1-34
MWAPRCRRFWSRWEQVAALLLLLLLLGVPPRSLA

SEQ.ID.NO. 251    HG1018515P1 NP_783165:NM_175575_1-29
MWAPRCRRFWSRWEQVAALLLLLLLLGVP

SEQ.ID.NO. 252    HG1018516P1 NP_783165:NM_175575_1-30
MWAPRCRRFWSRWEQVAALLLLLLLLGVPP

SEQ.ID.NO. 253    HG1018517P1 NP_783165:NM_175575_1-27
MWAPRCRRFWSRWEQVAALLLLLLLLG

SEQ.ID.NO. 254    HG1018856P1 27482680:27482679
MWCASPVAVVAFCAGLLVSHPVLTQGQEAGGRPGADCEVCKEFLNRFYKSLIDRGVNFSL
DTIEKELISFCLDTKGKENRLCYYLGATKDAATKILSEVTRPMSVHMPAMKICEKLKKLD
SQICELKYEKTLDLASVDLRKMRVAELKQILHSWGEECRACAEKTDYVNLIQELAPKYAA
THPKTEL

SEQ.ID.NO. 255    HG1018857P1 27482680:27482679_1-26
MWCASPVAVVAFCAGLLVSHPVLTQG

SEQ.ID.NO. 256    HG1018858P1 27482680:27482679_1-24
MWCASPVAVVAFCAGLLVSHPVLT

## Claims

1. A heterologous polypeptide comprising a secretory leader and a second polypeptide, wherein the secretory leader is operably linked to the N-terminus of the second polypeptide, wherein the secretory leader is not so linked to the second polypeptide in nature, and wherein the secretory leader comprises a leader sequence of a secretable protein, and the secretable protein is collagen type IX alpha 1 chain.

2. The heterologous polypeptide of claim 1, wherein the secretory leader comprises an amino acid sequence of SEQ ID NO: 27.

3. The heterologous polypeptide of claim 1 or 2, wherein the second polypeptide is selected from a second secretable polypeptide, an extracellular portion of a transmembrane protein, and a soluble receptor.

4. The heterologous polypeptide of claim 1, 2 or 3, wherein the second secretable polypeptide is selected from a growth factor, a cytokine, a lymphokine, an interferon, a hormone, a stimulatory factor, an inhibitory factor, and splice variants thereof.

5. The heterologous polypeptide of claim 1, 2 or 3, further comprising a fusion partner.

6. The heterologous polypeptide of claim 5, wherein the fusion partner is a polymer.

7. The heterologous polypeptide of claim 6, wherein the polymer is a third molecule, and wherein the third molecule is selected from polyethylene glycol and all or part of human serum albumin, fetuin A, fetuin B and Fc.

8. An isolated nucleic acid molecule comprising a polynucleotide sequence, wherein the polynucleotide sequence encodes an amino acid sequence of a heterologous polypeptide according to any one of claims 1 to 7, wherein the leader sequence is encoded by a first polynucleotide and the second polypeptide is encoded by a second polynucleotide, the first polynucleotide and the second polynucleotide are operably linked to facilitate secretion of the heterologous polypeptide from a cell, and wherein the first and second polynucleotide are not so linked in nature.

9. A nucleic acid molecule encoding a heterologous polypeptide, comprising a first polynucleotide that encodes the leader sequence of collagen type IX alpha 1 chain and a second polynucleotide that encodes a second polypeptide, wherein the first polynucleotide and the second polynucleotide are operably linked to facilitate secretion of the heterologous polypeptide from a cell, and wherein the first and second polynucleotide are not so linked in nature.

10. The nucleic acid molecule of claim 9, wherein the leader sequence of collagen type IX alpha 1 chain comprises an amino acid sequence of SEQ ID NO: 27.

11. The nucleic acid of claim 9 or 10, wherein the second polypeptide is selected from a secretable polypeptide, an extracellular portion of a transmembrane protein, and a soluble receptor.

12. The nucleic acid molecule of claim 8 or 9, further comprising a third polynucleotides, wherein the third polynucleotide is a Kozak sequence or a fragment thereof that is situated at its 5' end.

13. The nucleic acid molecule of claim 12, further comprising a fourth polynucleotide, wherein the fourth polynucleotide comprises a restriction enzyme-cleavable sequence at its 3' end.

14. The nucleic acid molecule of claim 13, further comprising a fifth polynucleotide that encodes a tag.

15. The nucleic acid molecule of claim 14, wherein the tag is a purification tag.

16. The nucleic acid molecule of claim 14, wherein the tag is selected from V5, HisX6V(SEQ ID NO: 257), HisX8 (SEQ ID NO: 258, an avidin molecule, and a biotin molecule.

17. The nucleic acid molecule of claim 13, further comprising a sixth polynucleotide that encodes a second enzyme-cleavable sequence that can be cleaved by a second enzyme, wherein the second cleavable sequence is situated upstream of the tag if the tag is situated at the C-terminus of the heterologous polypeptide, or downstream of the tag if the tag is situated at the N-terminus of the heterologous polypeptide.

18. The nucleic acid molecule of claim 17, wherein the second enzyme is thrombin or TEV from a tobacco virus.

19. A vector comprising the nucleic acid molecule of any one of claims 8-18, further comprising an origin of replication and a selectable marker.

20. The vector of claim 19, wherein the origin of replication is selected from SV40 ori, Pol ori, EBNA ori, and pMB1 ori.

21. The vector of claim 19, wherein the selectable marker is an antibiotic resistance gene.

22. The vector of claim 21, wherein the antibiotic resistance is selected from puromycin resistance, kanamycin resistance, and ampicillin resistance.

23. A recombinant host cell comprising a cell and the heterologous polypeptide of any of claims 1-7, the nucleic acid molecule of any of claims 8-18, or the vector of any one of claims 19-22.

24. The recombinant host cell of claim 23, wherein the cell is a eukaryotic cell.

25. The recombinant host cell of claim 24, wherein the cell is a mammalian cell.

26. A method of producing a secreted polypeptide, comprising:
(a) providing the nucleic acid molecule of any of claims 8-18; and
(b) expressing the nucleic acid molecule in an expression system.

27. The method of claim 26, wherein the expression system is a cellular expression system and the cell is a mammalian cell.

28. The method of claim 27, wherein the mammalian cell is a 293 cell line.

29. The method of claim 27, wherein the mammalian cell is a PER.C6^{®} cell line.

30. The method of claim 27, wherein the mammalian cell is a CHO cell line.

31. The method of claim 28, wherein the 293 cell is a 293-T cell.

32. The method of claim 28, wherein the 293 cell is a 293-6E cell.

## Patentansprüche

1. Heterologes Polypeptid, umfassend einen Sekretionsleader und ein zweites Polypeptid, worin der Sekretionsleader operabel an den N-Terminus des zweiten Polypeptids gebunden ist, worin der Sekretionsleader in der Natur nicht derart an das zweite Polypeptid gebunden ist und worin der Sekretionsleader eine Leadersequenz eines sekretierbaren Proteins umfasst und das sekretierbare Protein die α1-Kette von Kollagen Typ IX ist.

2. Heterologes Polypeptid nach Anspruch 1, worin der Sekretionsleader eine Aminosäuresequenz von Seq.-ID Nr. 27 umfasst.

3. Heterologes Polypeptid nach Anspruch 1 oder 2, worin das zweite Polypeptid aus einem zweiten sekretierbaren Polypeptid, einem extrazellulären Abschnitt eines Transmembranproteins und einem löslichen Rezeptor ausgewählt ist.

4. Heterologes Polypeptid nach Anspruch 1, 2 oder 3, worin das zweite sekretierbare Polypeptid aus einem Wachstumsfaktor, einem Zytokin, einem Lymphokin, einem Interferon, einem Hormon, einem stimulierenden Faktor, einem Hemmungsfaktor und Spleißvarianten davon ausgewählt ist.

5. Heterologes Polypeptid nach Anspruch 1, 2 oder 3, das weiters einen Fusionspartner umfasst.

6. Heterologes Polypeptid nach Anspruch 5, worin der Fusionspartner ein Polymer ist.

7. Heterologes Polypeptid nach Anspruch 6, worin das Polymer ein drittes Molekül ist und worin das dritte Molekül aus Polyethylenglykol und der Gesamtheit oder einem Teil von Human-Serumalbumin, Fetuin A, Fetuin B und Fc ausgewählt ist.

8. Isoliertes Nucleinsäuremolekül, das eine Polynucleotidsequenz umfasst, worin die Polynucleotidsequenz für eine Aminosäuresequenz eines heterologen Polypeptids nach einem der Ansprüche 1 bis 7 kodiert, worin für die Leadersequenz ein erstes Polynucleotid und für das zweite Polypeptid ein zweites Polynucleotid kodiert, wobei das erste Polynucleotid und das zweite Polynucleotid operabel verbunden sind, um die Sekretion des heterologen Polypeptids aus einer Zelle zu erleichtern, und worin das erste und das zweite Polynucleotid in der Natur nicht auf diese Weise verbunden sind.

9. Für ein heterologes Polypeptid kodierendes Nucleinsäuremolekül, das ein erstes Polynucleotid, das für die Leadersequenz der α1-Kette von Kollagen Typ IX kodiert, und ein zweites Polynucleotid umfasst, das für ein zweites Polypeptid kodiert, worin das erste Polynucleotid und das zweite Polynucleotid operabel verbunden sind, um die Sekretion des heterologen Polypeptids aus eine Zelle zu erleichtern, und worin das erste und das zweite Polynucleotid in der Natur nicht auf diese Weise verbunden sind.

10. Nucleinsäuremolekül nach Anspruch 9, worin die Leadersequenz von der α1-Kette von Kollagen Typ IX eine Aminosäuresequenz von Seq.-ID Nr. 27 umfasst.

11. Nucleinsäure nach Anspruch 9 oder 10, worin das zweite Polypeptid aus einem sekretierbaren Polypeptid, einem extrazellulären Anteil eines Transmembranproteins und einem löslichen Rezeptor ausgewählt ist.

12. Nucleinsäuremolekül nach Anspruch 8 oder 9, das weiters ein drittes Polynucleotid umfasst, worin das dritte Polynucleotid eine an seinem 5'-Ende gelegene Kozak-Sequenz oder ein Fragment davon ist.

13. Nucleinsäuremolekül nach Anspruch 12, das weiters ein viertes Polynucleotid umfasst, worin das vierte Polynucleotid an seinem 3'-Ende eine mittels Restriktionsenzym spaltbare Sequenz umfasst.

14. Nucleinsäuremolekül nach Anspruch 13, das weiters ein fünftes Polynucleotid umfasst, das für eine Markierung kodiert.

15. Nucleinsäuremolekül nach Anspruch 14, worin die Markierung eine Reinigungsmarkierung ist.

16. Nucleinsäuremolekül nach Anspruch 14, worin die Markierung aus V5, HisX6V (Seq.-ID Nr. 257), HisX8 (Seq.-ID Nr. 258), einem Avidinmolekül und einem Biotinmolekül ausgewählt ist.

17. Nucleinsäuremolekül nach Anspruch 13, das weiters ein sechstes Polynucleotid umfasst, das für eine zweite enzymspaltbare Sequenz kodiert, die von einem zweiten Enzym gespalten werden kann, worin die zweite spaltbare Sequenz, wenn die Markierung am C-Terminus des heterologen Polypeptids liegt, stromauf von der Markierung oder, wenn die Markierung am N-Terminus des heterologen Polypeptids liegt, stromab von der Markierung liegt.

18. Nucleinsäuremolekül nach Anspruch 17, worin das zweite Enzym Thrombin oder TEV aus einem Tabakvirus ist.

19. Vektor, der ein Nucleinsäuremolekül nach einem der Ansprüche 8 bis 18 umfasst und weiters einen Replikationsstartpunkt und einen selektierbaren Marker umfasst.

20. Vektor nach Anspruch 19, worin der Replikationsstartpunkt aus SV40 ori, Pol ori, EBNA ori und pMB1 ori ausgewählt ist.

21. Vektor nach Anspruch 19, worin der selektierbare Marker ein Antibiotikaresistenz-Gen ist.

22. Vektor nach Anspruch 21, worin die Antibiotikaresistenz aus Puromycinresistenz, Kanamycinresistenz und Ampicillinresistenz ausgewählt ist.

23. Rekombinante Wirtszelle, umfassend eine Zelle und das heterologe Polypeptid nach einem der Ansprüche 1 bis 7, das Nucleinsäuremolekül nach einem der Ansprüche 8 bis 18 oder den Vektor nach einem der Ansprüche 19 bis 22.

24. Rekombinante Wirtszelle nach Anspruch 23, worin die Zelle eine eukaryotische Zelle ist.

25. Rekombinante Wirtszelle nach Anspruch 24, worin die Zelle eine Säugetierzelle ist.

26. Verfahren zur Produktion eines sekretierten Polypeptids, umfassend:
(a) das Bereitstellen eines Nucleinsäuremoleküls nach einem der Ansprüche 8 bis 18; und
(b) das Exprimieren des Nucleinsäuremoleküls in einem Expressionssystem.

27. Verfahren nach Anspruch 26, worin das Expressionssystem ein zelluläres Expressionssystem und die Zelle eine Säugetierzelle ist.

28. Verfahren nach Anspruch 27, worin die Säugetierzelle eine 293-Zelllinie ist.

29. Verfahren nach Anspruch 27, worin die Säugetierzelle eine PER.C6^{®}-Zelllinie ist.

30. Verfahren nach Anspruch 27, worin die Säugetierzelle eine CHO-Zelllinie ist.

31. Verfahren nach Anspruch 28, worin die 293-Zelle eine 293-T-Zelle ist.

32. Verfahren nach Anspruch 28, worin die 293-Zelle eine 293-6E-Zelle ist.

## Revendications

1. Polypeptide hétérologue comprenant une tête de sécrétion et un deuxième polypeptide, où la tête de sécrétion est fonctionnellement liée au terminus-N du deuxième polypeptide, où la tête de sécrétion n'est pas liée ainsi au deuxième polypeptide dans la nature, et où la tête de sécrétion comprend une séquence de tête d'une protéine sécrétable, et la protéine sécrétable est une chaîne alpha-1 du collagène de type IX.

2. Polypeptide hétérologue selon la revendication 1, où la tête de sécrétion comprend une séquence des acides aminés de SEQ ID NO: 27.

3. Polypeptide hétérologue selon la revendication 1 ou 2, où le deuxième polypeptide est sélectionné parmi un deuxième polypeptide sécrétable, une portion extracellulaire d'une protéine de transmembrane et un récepteur soluble.

4. Polypeptide hétérologue selon la revendication 1, 2 ou 3, où le deuxième polypeptide sécrétable est sélectionné parmi un facteur de croissance, une cytokine, une lymphokine, un interféron, une hormone, un facteur de stimulation, un facteur d'inhibition et des variantes d'épissure de ceux-ci.

5. Polypeptide hétérologue selon la revendication 1, 2 ou 3, comprenant en outre un partenaire de fusion.

6. Polypeptide hétérologue selon la revendication 5, où le partenaire de fusion est un polymère.

7. Polypeptide hétérologue selon la revendication 6, où le polymère est une troisième molécule, et où la troisième molécule est sélectionnée parmi polyéthylène glycol et l'ensemble ou une partie de l'albumine du sérum humain, la fétuine A, la fétuine B et Fc.

8. Molécule d'acide nucléique isolée comprenant une séquence de polynucléotides, où la séquence de polynucléotides code pour une séquence d'acides aminés d'un polypeptide hétérologue selon l'une quelconque des revendications 1 à 7, où la séquence de tête est codée par un premier nucléotide et le deuxième polypeptide est codé par un deuxième polynucléotide, le premier polynucléotide et le deuxième polynucléotide sont fonctionnellement liés pour faciliter la sécrétion du polypeptide hétérologue d'une cellule, et où les premier et deuxième polynucléotides ne sont pas ainsi liés dans la nature.

9. Molécule d'acide nucléique codant pour un polypeptide hétérologue, comprenant un premier polynucléotide qui code la séquence de tête de la chaîne alpha 1 du collagène de type IX et un deuxième polynucléotide qui code pour un deuxième polypeptide, où le premier polynucléotide et le deuxième polynucléotide sont fonctionnellement liés pour faciliter la sécrétion du polypeptide hétérologue d'une cellule, et où le premier et le deuxième polynucléotide ne sont pas ainsi liés dans la nature.

10. Molécule d'acide nucléique selon la revendication 9, où la séquence de tête de la chaîne alpha 1 du collagène de type IX comprend une séquence d'acides aminés de la SEQ ID NO:27.

11. Molécule d'acide nucléique selon la revendication 9 ou 10, où le deuxième polypeptide est sélectionné parmi un polypeptide sécrétable, une portion extracellulaire d'une protéine de transmembrane et un récepteur soluble.

12. Molécule d'acide nucléique selon la revendication 8 ou 9, comprenant en outre un troisième polynucléotide, où le troisième polynucléotide est une séquence Kozak ou un fragment de celle-ci qui est située à son extrémité 5'.

13. Molécule d'acide nucléique selon la revendication 12, comprenant en outre un quatrième polynucléotide, où le quatrième polynucléotide comprend une séquence de restriction enzyme-clivable à son extrémité 3'.

14. Molécule d'acide nucléique selon la revendication 13, comprenant en outre un cinquième polynucléotide qui encode pour un indicateur.

15. Molécule d'acide nucléique selon la revendication 14, où l'indicateur est un indicateur de purification.

16. Molécule d'acide nucléique selon la revendication 14, où l'indicateur est sélectionné parmi V5, HisX6V(SEQ ID NO:257), HisX8 (SEQ ID NO: 258, une molécule d'avidine et une molécule de biotine.

17. Molécule d'acide nucléique selon la revendication 13, comprenant en outre un sixième polynucléotide qui code pour une deuxième séquence enzyme-clivable qui peut être clivée par une deuxième enzyme, où la deuxième séquence clivable se situe en amont de l'indicateur si l'indicateur est situé au terminus-C du polypeptide hétérologue ou en aval de l'indicateur si l'indicateur est situé au terminus-N du polypeptide hétérologue.

18. Molécule d'acide nucléique selon la revendication 17, où la deuxième enzyme est une thrombine ou un TEV d'un virus de tabac.

19. Vecteur comprenant la molécule d'acide nucléique selon l'une quelconque des revendications 8 à 18, comprenant en outre une origine de réplication et un marqueur sélectionnable.

20. Vecteur selon la revendication 19, où l'origine de la réplication est sélectionnée parmi SV40 ori, Pol ori, EBNA ori et pMBI ori.

21. Vecteur selon la revendication 19, où le marqueur sélectionnable est un gène de résistance aux antibiotiques.

22. Vecteur selon la revendication 21, où la résistance aux antibiotiques est sélectionnée parmi la résistance à la puromycine, la résistance à la kanamycine et la résistance à l'ampicilline.

23. Cellule hôte recombinante comprenant une cellule et le polypeptide hétérologue selon l'une quelconque des revendications 1 à 7, la molécule d'acide nucléique selon l'une quelconque des revendications 8 à 18 ou le vecteur selon l'une quelconque des revendications 19-22.

24. Cellule hôte recombinante selon la revendication 23, où la cellule est une cellule eucaryotique.

25. Cellule hôte recombinante selon la revendication 24, où la cellule est une cellule mammalienne.

26. Méthode de production d'un polypeptide sécrété, comprenant:
(a) réaliser la molécule d'acide nucléique selon l'une quelconque des revendications 8 à 18; et
(b) exprimer la molécule d'acide nucléique dans un système d'expression.

27. Méthode selon la revendication 26, où le système d'expression est un système d'expression cellulaire, et la cellule est une cellule mammalienne.

28. Méthode selon la revendication 27, où la cellule mammalienne est une lignée de cellules 293.

29. Méthode selon la revendication 27, où la cellule mammalienne est une lignée de cellules PER.C6^{®}.

30. Méthode selon la revendication 27, où la cellule mammalienne est une lignée de cellules CHO.

31. Méthode selon la revendication 28, où la cellule 293 est une cellule 293-T.

32. Méthode selon la revendication 28, où la cellule 293 est une cellule 293-6E.
